# EUROPEAN PATENT SPECIFICATION

(11) **EP 3 772 928 B1**
(45) Date of publication and mention of the grant of the patent: **26.02.2025**
(21) Application number: 19780971.8
(22) Date of filing: 05.04.2019
(51) Int. Cl.: A01K 67/027, C12N 9/02, C12N 15/113, C12N 15/85, C12N 15/873, C12N 15/877

(54) **COMPOSITIONS AND METHODS FOR SOMATIC CELL REPROGRAMMING AND MODULATING IMPRINTING**
ZUSAMMENSETZUNGEN UND VERFAHREN ZUR PRÄGUNG DER UMPROGRAMMIERUNG UND MODULATION VON SOMATISCHEN ZELLEN
COMPOSITIONS ET PROCÉDÉS DE REPROGRAMMATION DE CELLULES SOMATIQUES ET DE MODULATION D'IMPRESSION

(30) Priority: 06.04.2018 US 201862654199 P
(43) Date of publication of application: 17.02.2021
(73) Proprietor: CHILDREN'S MEDICAL CENTER CORPORATION, Boston, Massachusetts 02115 (US)
(72) Inventor: ZHANG, Yi, Boston, Massachusetts 02115 (US); MATOBA, Shogo, Boston, MA 02115 (US)
(74) Representative: Impact Intellectual Property LLP
(86) International application number: PCT/US2019/026074
(87) International publication number: WO 2019/195738

(56) References cited:
- WO-A1-2019/018635
- WO-A2-2016/044271
- US-A1- 2017 327 846
- LIU ZHEN ET AL: "Cloning of Macaque Monkeys by Somatic Cell Nuclear Transfer", CELL, ELSEVIER, AMSTERDAM NL, vol. 172, no. 4, 1 February 2018 (2018-02-01), pages 881, XP085347123, ISSN: 0092-8674, DOI: 10.1016/J.CELL.2018.01.020
- LOI PASQUALINO ET AL: "A New, Dynamic Era for Somatic Cell Nuclear Transfer?", TRENDS IN BIOTECHNOLOGY, ELSEVIER PUBLICATIONS, CAMBRIDGE, GB, vol. 34, no. 10, 22 April 2016 (2016-04-22), pages 791 - 797, XP029740898, ISSN: 0167-7799, DOI: 10.1016/J.TIBTECH.2016.03.008
- DEGONG RUAN ET AL: "XIST Derepression in Active X Chromosome Hinders Pig Somatic Cell Nuclear Transfer", STEM CELL REPORTS, vol. 10, no. 2, 1 February 2018 (2018-02-01), United States, pages 494 - 508, XP055642442, ISSN: 2213-6711, DOI: 10.1016/j.stemcr.2017.12.015
- MATOBA SHOGO ET AL: "Loss of H3K27me3 Imprinting in Somatic Cell Nuclear Transfer Embryos Disrupts PostImplantation Development", CELL STEM CELL, vol. 23, no. 3, 6 September 2018 (2018-09-06), pages 343 - 354, XP055862599, Retrieved from the Internet <URL:https://www.cell.com/cell-stem-cell/pdfExtended/S1934-5909(18)30290-X> DOI: 10.1016/j.stem.2018.06.008
- RUAN, D. ET AL.: "XIST Derepression in Active X Chromosome Hinders Pig Somatic Cell Nuclear Transfer", STEM CELL REPORTS, vol. 10, no. 2, 11 January 2018 (2018-01-11), pages 494 - 508, XP055642442
- WENQIANG LIU ET AL.: "Identification of Key Factors Conquering Developmental Arrest of Somatic Cell Cloned Embryos by Combining Embryo Biopsy and Single- Cell Sequencing", CELL DISCOVERY, vol. 2, no. 16010, 7 June 2016 (2016-06-07), pages 1 - 15, XP055642444
- MATOBA ET AL.: "RNAi-Mediated Knockdown of Xist can Rescue the Impaired Postimplantation Development of Cloned Mouse Embryos", PROC. NATL. ACAD. SCI. USA, vol. 108, no. 51, 20 December 2011 (2011-12-20), pages 20621 - 20626, XP055642455

## Description

### CROSS-REFERENCE TO RELATED APPLICATION

This application claims the benefit of the following U.S. Provisional Application No.: 62/654,199, filed April 6, 2018.

### STATEMENT OF RIGHTS TO INVENTIONS MADE UNDER FEDERALLY SPONSORED RESEARCH

This invention was made with government support under grant number HD092465 awarded by the National Institutes of Health. The government has certain rights in the invention.

### BACKGROUND

Mammalian oocytes are capable of reprogramming somatic cells into a totipotent state through somatic cell nuclear transfer (SCNT). SCNT is used in therapeutic cloning which involves the generation of tissues from a donor organism that is genetically identical to or similar to the intended host. SCNT also enables cloning of animals. This technique has great potential in agro-biotechnology, as well as in the conservation of endangered species. However, the extremely low success rate of cloning makes the actual use of this technique difficult. For example, in the case of mouse, only about 30% of SCNT embryos develop to blastocysts and only 1-2 % of embryos transferred to surrogate mothers can reach term. Furthermore, in the surviving embryos, abnormalities are frequently observed in extraembryonic tissues, such as placenta and umbilical cord in almost all cloned mammalian species. These observations suggest that SCNT reprogramming has some deficiencies that impede the embryo developmental process. Various epigenetic abnormalities in DNA methylation, histone modifications, and genomic imprinting have been implicated in the low success rate of SCNT. There is a significant need for improving the efficiency of cloning.

### SUMMARY OF THE INVENTION

The invention provides methods for improving cloning efficiency. In particular embodiments, the invention provides methods for improving non-human somatic cell nuclear transfer efficiency that involve *Kdm4d* overexpression is an *Xist* knockout donor cell.

In one aspect, the invention provides a method for obtaining a cloned non-human, mammalian blastocyst that includes (a) transferring a donor nucleus obtained from an isolated or cultured non-human, mammalian somatic cell lacking Xist activity into a non-human, mammalian enucleated oocyte; and (b) overexpressing *Kdm4d* in the oocyte, thereby obtaining a cloned non-human, mammalian blastocyst, wherein the somatic cell lacking Xist activity is produced by the use of: genome editing; a CRISPR system which introduces a deletion of inactivity mutation in a genomic Xist polynucleotide; siRNA; shRNA; or RNAi. In some embodiments of the method, the oocyte is injected with a *Kdm4d* mRNA. In some embodiments, the donor cell nucleus is obtained from a non-human embryoic fibroblast comprising a deletion in Xist or comprising an inactive form of Xist. In some embodiments, the donor nucleus is obtained from a cat, cow, dog, pig, or horse. In some embodiments, as described but not specifically claimed herein, the method also includes transferring the blastocyst into a host uterus for gestation. In some embodiments, as described but not specifically claimed herein, the method increases the rate of live births relative to conventional somatic cell nuclear transfer by at least about 10-20%. Some aspects of the invention include a non-human blastocyst produced by the method described above. Some aspects of the invention include a non-human cloned organism produced by implanting the blastocyst produced by the method described above.

In another aspect, the invention provides a method for obtaining a cell or tissue suitable for transplantation into a non-human, mammalian subject, the method comprising: (a) inactivating *Xist* or reducing Xist activity or expression in a cultured cell obtained from a non-human, mammalian subject; (b) transferring the nucleus from the cultured cell into anon-human, mammalian enucleated oocyte, thereby activating the oocyte; and (c) injecting the activated non-human, mammalian oocyte obtained in step (b) with a *Kdm4d* mRNA; overexpressing Kdm4d in the oocyte; and culturing the resulting oocyte, thereby obtaining a non-human, mammalian cell or tissue suitable for transplantation into the non-human, mammalian subject.. In some embodiments, Xist is inactivated by genome editing of the cultured cell of step (a). For example, in some embodiments, *Xist* is inactivated by using a CRISPR system to introduce a deletion or inactivating mutation in a genomic *Xist* polynucleotide in the cultured cell of step (a). In other embodiments of the method, Xist polynucleotide expression or activity is reduced by using siRNA or shRNA in the cultured cell of step (a).

In other aspects of the invention, there is provided a non-human cell comprising a deletion in Xist or a level of of Xist expression reduced by the use of genome editing, a CRISPR system which introduces a deletion or inactivity mutation in a genomic Xist polynucleotide, siRNA, shRNA, or RNAi and comprisong a heterologous polynucleotide encoding *Kdm4d,* which is expressed at an increased level in the non-human cell.

As described but not specifically claimed herein, additional aspects include a non-human oocyte comprising a donor nucleus obtained from a somatic cell lacking Xist activity and expressing an increased level of Kdm4d relative to a conventional oocyte.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

By "KDM4D polypeptide" is meant a polypeptide or fragment thereof having at least about 85% amino acid sequence identity to NCBI Reference No. Q6B0I6 and having demethylase activity. An exemplary KDM4D amino acid sequence is provided below:
>sp | Q6B0I6 | KDM4D_HUMAN Lysine-specific demethylase 4D OS=Homo sapiens OX=9606 GN=KDM4D PE=1 SV=3

METMKSKANCAQNPNCNIMIFHPTKEEFNDFDKYIAYMESQGAHRAGLAKIIPPKEWKAR
ETYDNISEILIATPLQQVASGRAGVFTQYHKKKKAMTVGEYRHLANSKKYQTPPHQNFED
LERKYWKNRIYNSPIYGADISGSLFDENTKQWNLGHLGTIQDLLEKECGVVIEGVNTPYL
YFGMWKTTFAWHTEDMDLYSINYLHLGEPKTWYVVPPEHGQRLERLARELFPGSSRGCGA
FLRHKVALISPTVLKENGIPFNRITQEAGEFMVTFPYGYHAGFNHGFNCAEAINFATPRW
IDYGKMASQCSCGEARVTFSMDAFVRILQPERYDLWKRGQDRAVVDHMEPRVPASQELST
QKEVQLPRRAALGLRQLPSHWARHSPWPMAARSGTRCHTLVCSSLPRRSAVSGTATQPRA
AAVHSSKKPSSTPSSTPGPSAQIIHPSNGRRGRGRPPQKLRAQELTLQTPAKRPLLAGTT
CTASGPEPEPLPEDGALMDKPVPLSPGLQHPVKASGCSWAPVP

By "KDM4D polynucleotide" is meant a nucleic acid molecule encoding a KDM4D polypeptide. An exemplary KDM4D nucleic acid is provided below:

1 aaggggcggg gccgaagcgg cccagggggc gggcgtttga aatcagtgcc ttagagtaga
61 ccctaaacct cattttatac cttcaagaac caattactta atgtctcttc cgtcttttcc
121 gtccccgacc ccctcccaga ctccttcatt ccggtactgc gtggacggaa agccccgggt
181 agccgacacc acgtccccgg ctagcgggag agagcgtgga aaaggattac accaaactgt

241 ttaaatccaa cgactcctgc ttccatcctt tctcctgagc tagaaccaac aaacctagag
301 agttgggctt cggaaaaact agtgttttca tttaattgga tatgaagaaa gaacaaatat
361 gtacggggca accacgatct ttacaaagaa cataagttcc aggaaagcag gaaccttgtc
421 tctcttgttc actgggtgta tcctctgcat atagaacagt gcctggcaca taataggtgc
481 tgaattttgt tctaaacact gaggacattc tctgctacat ttgggtcgta cccccaggtc
541 tgagtaattc aatagactta agaagacaga gcccagcagc aaccgaaaca taacagagtt
601 gcaggatcag ctaacgtcaa tgcctgggca aagctgctgc ccagagtgga atctcactag
661 tgaataaaca agcccaagaa agattatcat ctcatttgca aaaaaaaaag tacgctggta
721 gatcctgcta cctcatagat aacaccagtc aaattttttt ttaaagtagc attttcctac
781 attgtcaact atctagaaca tacctaaaaa ctaagagttt actgcttatt aaatggaaac
841 tatgaagtct aaggccaact gtgcccagaa tccaaattgt aacataatga tatttcatcc
901 aaccaaagaa gagtttaatg attttgataa atatattgct tacatggaat cccaaggtgc
961 acacagagct ggcttggcta agataattcc acccaaagaa tggaaagcca gagagaccta
1021 tgataatatc agtgaaatct taatagccac tcccctccag caggtggcct ctgggcgggc
1081 aggggtgttt actcaatacc ataaaaaaaa gaaagccatg actgtggggg agtatcgcca
1141 tttggcaaac agtaaaaaat atcagactcc accacaccag aatttcgaag atttggagcg
1201 aaaatactgg aagaaccgca tctataattc accgatttat ggtgctgaca tcagtggctc
1261 cttgtttgat gaaaacacta aacaatggaa tcttgggcac ctgggaacaa ttcaggacct
1321 gctggaaaag gaatgtgggg ttgtcataga aggcgtcaat acaccctact tgtactttgg
1381 catgtggaaa accacgtttg cttggcatac agaggacatg gacctttaca gcatcaacta
1441 cctgcacctt ggggagccca aaacttggta tgtggtgccc ccagaacatg gccagcgcct
1501 ggaacgcctg gccagggagc tcttcccagg cagttcccgg ggttgtgggg ccttcctgcg
1561 gcacaaggtg gccctcatct cgcctacagt tctcaaggaa aatgggattc ccttcaatcg
1621 cataactcag gaggctggag agttcatggt gacctttccc tatggctacc atgctggctt
1681 caaccatggt ttcaactgcg cagaggccat caattttgcc actccgcgat ggattgatta
1741 tggcaaaatg gcctcccagt gtagctgtgg ggaggcaagg gtgacctttt ccatggatgc
1801 cttcgtgcgc atcctgcaac ctgaacgcta tgacctgtgg aaacgtgggc aagaccgggc
1861 agttgtggac cacatggagc ccagggtacc agccagccaa gagctgagca cccagaagga
1921 agtccagtta cccaggagag cagcgctggg cctgagacaa ctcccttccc actgggcccg
1981 gcattcccct tggcctatgg ctgcccgcag tgggacacgg tgccacaccc ttgtgtgctc
2041 ttcactccca cgccgatctg cagttagtgg cactgctacg cagccccggg ctgctgctgt
2101 ccacagctct aagaagccca gctcaactcc atcatccacc cctggtccat ctgcacagat
2161 tatccacccg tcaaatggca gacgtggtcg tggtcgccct cctcagaaac tgagagctca
2221 ggagctgacc ctccagactc cagccaagag gcccctcttg gcgggcacaa catgcacagc
2281 ttcgggccca gaacctgagc ccctacctga ggatggggct ttgatggaca agcctgtacc
2341 actgagccca gggctccagc atcctgtcaa ggcttctggg tgcagctggg cccctgtgcc
2401 ctaagtccac gggctgtctt tatatcccac tgccctgctg tgtgacagtt tgatgaaact
2461 ggttacattt acatcccaaa actttggttg agtttgcagg actctaggca tgcatgaaag
2521 agcccccctg gtgatgccct tggatgctgc caagtccatg gtagttttca attttgccat
2581 acttttgttc ttcctaccgg accctggaat gtctttggat attgctaaaa tctatttctg
2641 cagctgaggt tttatccact ggacacattt gtgtgtgaga actaggtctt gttgaggtta
2701 gcgtaacctg gtatatgcaa ctaccatcct ctgggccaac tgtggaagct gctgcacttg
2761 tgaagaatcc tgagctttga ttcctcttca gtctacgcat ttctctcttc cctccctca
2821 cccccttttt cttataaaac taggttcttt atacagataa ggtcagtaga gttccagaat

2881 aaaagatatg acttttctga gttatttatg tacttaaaat atgttgtcac agtatttgtt
2941 cccaaatata ttaaaggtaa ccaaaatgtt aaaaaaaaaa aaaaaaaa

By "EZH1 polypeptide" (histone-lysine N-methyltransferase EZH1) is meant a protein having at least about 85% amino acid identity to the sequence provided at NCBI Reference Sequence: NP_001982, or a fragment thereof, and having methyltransferase activity. An exemplary H3K27 methyltransferase amino acid sequence is provided below:

  1 meipnpptsk citywkrkvk seymrlrqlk rlqanmgaka lyvanfakvq ektqilneew
 61 kklrvqpvqs mkpvsghpfl kkctiesifp gfasqhmlmr slntvalvpi myswsplqqn
121 fmvedetvlc nipymgdevk eedetfieel innydgkvhg eeemipgsvl isdavflelv
181 dalnqysdee eeghndtsdg kqddskedlp vtrkrkrhai egnkksskkq fpndmifsai
241 asmfpengvp ddmkeryrel temsdpnalp pqctpnidgp naksvqreqs lhsfhtlfcr
301 rcfkydcflh pfhatpnvyk rknkeikiep epcgtdcfll legakeyaml hnprskcsgr
361 rrrrhhivsa scsnasasav aetkegdsdr dtgndwasss seansrcqtp tkqkaspapp
421 qlcvveapse pvewtgaees lfrvfhgtyf nnfcsiarll gtktckqvfq favkeslilk
481 lptdelmnps qkkkrkhrlw aahcrkiqlk kdnsstqvyn yqpcdhpdrp cdstcpcimt
541 qnfcekfcqc npdcqnrfpg crcktqcntk qcpcylavre cdpdlcltcg asehwdckvv
601 sckncsiqrg lkkhlllaps dvagwgtfik esvqknefis eycgelisqd eadrrgkvyd
661 kymssflfnl nndfvvdatr kgnkirfanh svnpncyakv vmvngdhrig ifakraiqag
721 eelffdyrys qadalkyvgi eretdvl

By "EZH1 polynucleotide" is meant a nucleic acid molecule encoding the EZH1 polypeptide. An exemplary EZH1 polynucleotide sequence is provided at NM_001991.4 and reproduced below:

1 aggaggcgcg gggcggggca cggcgcaggg gtggggccgc ggcgcgcatg cgtcctagca
61 gcgggacccg cggctcggga tggaggctgg acacctgttc tgctgttgtg tcctgccatt
121 ctcctgaaga acagaggcac actgtaaaac ccaacacttc cccttgcatt ctataagatt
181 acagcaagat ggaaatacca aatcccccta cctccaaatg tatcacttac tggaaaagaa
241 aagtgaaatc tgaatacatg cgacttcgac aacttaaacg gcttcaggca aatatgggtg
301 caaaggcttt gtatgtggca aattttgcaa aggttcaaga aaaaacccag atcctcaatg
361 aagaatggaa gaagcttcgt gtccaacctg ttcagtcaat gaagcctgtg agtggacacc
421 cttttctcaa aaagtgtacc atagagagca ttttcccggg atttgcaagc caacatatgt
481 taatgaggtc actgaacaca gttgcattgg ttcccatcat gtattcctgg tcccctctcc
541 aacagaactt tatggtagaa gatgagacgg ttttgtgcaa tattccctac atgggagatg
601 aagtgaaaga agaagatgag acttttattg aggagctgat caataactat gatgggaaag
661 tccatggtga agaagagatg atccctggat ccgttctgat tagtgatgct gtttttctgg
721 agttggtcga tgccctgaat cagtactcag atgaggagga ggaagggcac aatgacacct
781 cagatggaaa gcaggatgac agcaaagaag atctgccagt aacaagaaag agaaagcgac
841 atgctattga aggcaacaaa aagagttcca agaaacagtt cccaaatgac atgatcttca
901 gtgcaattgc ctcaatgttc cctgagaatg gtgtcccaga tgacatgaag gagaggtatc
961 gagaactaac agagatgtca gaccccaatg cacttccccc tcagtgcaca cccaacatcg
1021 atggccccaa tgccaagtct gtgcagcggg agcaatctct gcactccttc cacacacttt
1081 tttgccggcg ctgctttaaa tacgactgct tccttcaccc ttttcatgcc acccctaatg
1141 tatataaacg caagaataaa gaaatcaaga ttgaaccaga accatgtggc acagactgct
1201 tccttttgct ggaaggagca aaggagtatg ccatgctcca caacccccgc tccaagtgct

1261 ctggtcgtcg ccggagaagg caccacatag tcagtgcttc ctgctccaat gcctcagcct
1321 ctgctgtggc tgagactaaa gaaggagaca gtgacaggga cacaggcaat gactgggcct
1381 ccagttcttc agaggctaac tctcgctgtc agactcccac aaaacagaag gctagtccag
1441 ccccacctca actctgcgta gtggaagcac cctcggagcc tgtggaatgg actggggctg
1501 aagaatctct ttttcgagtc ttccatggca cctacttcaa caacttctgt tcaatagcca
1561 ggcttctggg gaccaagacg tgcaagcagg tctttcagtt tgcagtcaaa gaatcactta
1621 tcctgaagct gccaacagat gagctcatga acccctcaca gaagaagaaa agaaagcaca
1681 gattgtgggc tgcacactgc aggaagattc agctgaagaa agataactct tccacacaag
1741 tgtacaacta ccaaccctgc gaccacccag accgcccctg tgacagcacc tgcccctgca
1801 tcatgactca gaatttctgt gagaagttct gccagtgcaa cccagactgt cagaatcgtt
1861 tccctggctg tcgctgtaag acccagtgca ataccaagca atgtccttgc tatctggcag
1921 tgcgagaatg tgaccctgac ctgtgtctca cctgtggggc ctcagagcac tgggactgca
1981 aggtggtttc ctgtaaaaac tgcagcatcc agcgtggact taagaagcac ctgctgctgg
2041 ccccctctga tgtggccgga tggggcacct tcataaagga gtctgtgcag aagaacgaat
2101 tcatttctga atactgtggt gagctcatct ctcaggatga ggctgatcga cgcggaaagg
2161 tctatgacaa atacatgtcc agcttcctct tcaacctcaa taatgatttt gtagtggatg
2221 ctactcggaa aggaaacaaa attcgatttg caaatcattc agtgaatccc aactgttatg
2281 ccaaagtggt catggtgaat ggagaccatc ggattgggat ctttgccaag agggcaattc
2341 aagctggcga agagctcttc tttgattaca ggtacagcca agctgatgct ctcaagtacg
2401 tggggatcga gagggagacc gacgtccttt agccctccca ggccccacgg cagcacttat
2461 ggtagcggca ctgtcttggc tttcgtgctc acaccactgc tgctcgagtc tcctgcactg
2521 tgtctcccac actgagaaac cccccaaccc actccctctg tagtgaggcc tctgccatgt
2581 ccagagggca caaaactgtc tcaatgagag gggagacaga ggcagctagg gcttggtctc
2641 ccaggacaga gagttacaga aatgggagac tgtttctctg gcctcagaag aagcgagcac
2701 aggctggggt ggatgactta tgcgtgattt cgtgtcggct ccccaggctg tggcctcagg
2761 aatcaactta ggcagttccc aacaagcgct agcctgtaat tgtagctttc cacatcaaga
2821 gtccttatgt tattgggatg caggcaaacc tctgtggtcc taagacctgg agaggacagg
2881 ctaagtgaag tgtggtccct ggagcctaca agtggtctgg gttagaggcg agcctggcag
2941 gcagcacaga ctgaactcag aggtagacag gtcaccttac tacctcctcc ctcgtggcag
3001 ggctcaaact gaaagagtgt gggttctaag tacaggcatt caaggctggg ggaaggaaag
3061 ctacgccatc cttccttagc cagagaggga gaaccagcca gatgatagta gttaaactgc
3121 taagcttggg cccaggaggc tttgagaaag ccttctctgt gtactctgga gatagatgga
3181 gaagtgtttt cagattcctg ggaacagaca ccagtgctcc agctcctcca aagttctggc
3241 ttagcagctg caggcaagca ttatgctgct attgaagaag cattaggggt atgcctggca
3301 ggtgtgagca tcctggctcg ctggatttgt gggtgttttc aggccttcca ttccccatag
3361 aggcaaggcc caatggccag tgttgcttat cgcttcaggg taggtgggca caggcttgga
3421 ctagagagga gaaagattgg tgtaatctgc tttcctgtct gtagtgcctg ctgtttggaa
3481 agggtgagtt agaatatgtt ccaaggttgg tgaggggcta aattgcacgc gtttaggctg
3541 gcaccccgtg tgcagggcac actggcagag ggtatctgaa gtgggagaag aagcaggtag
3601 accacctgtc ccaggctgtg gtgccaccct ctctggcatt catgcagagc aaagcacttt
3661 aaccatttct tttaaaaggt ctatagattg gggtagagtt tggcctaagg tctctagggt
3721 ccctgcctaa atcccactcc tgagggaggg ggaagaagag agggtgggag attctcctcc
3781 agtcctgtct catctcctgg gagaggcaga cgagtgagtt tcacacagaa gaatttcatg
3841 tgaatggggc cagcaagagc tgccctgtgt ccatggtggg tgtgccgggc tggctgggaa

3901 caaggagcag tatgttgagt agaaagggtg tgggcgggta tagattggcc tgggagtgtt
3961 acagtaggga gcaggcttct cccttctttc tgggactcag agccccgctt cttcccactc
4021 cacttgttgt cccatgaagg aagaagtggg gttcctcctg acccagctgc ctcttacggt
4081 ttggtatggg acatgcacac acactcacat gctctcactc accacactgg agggcacaca
4141 cgtaccccgc acccagcaac tcctgacaga aagctcctcc cacccaaatg ggccaggccc
4201 cagcatgatc ctgaaatctg catccgccgt ggtttgtatt cattgtgcat atcagggata
4261 ccctcaagct ggactgtggg ttccaaatta ctcatagagg agaaaaccag agaaagatga
4321 agaggaggag ttaggtctat ttgaaatgcc aggggctcgc tgtgaggaat aggtgaaaaa
4381 aaacttttca ccagcctttg agagactaga ctgaccccac ccttccttca gtgagcagaa
4441 tcactgtggt cagtctcctg tcccagcttc agttcatgaa tactcctgtt cctccagttt
4501 cccatccttt gtccctgctg tcccccactt ttaaagatgg gtctcaaccc ctccccacca
4561 cgtcatgatg gatggggcaa ggtggtgggg actaggggag cctggtatac atgcggcttc
4621 attgccaata aatttcatgc actttaaagt cctgtggctt gtgacctctt aataaagtgt
4681 tagaatccaa aaaaaaa

By "EZH2 polypeptide" (histone-lysine N-methyltransferase EZH2) is meant a protein having at least about 85% amino acid identity to the sequence provided at UniProtKB/Swiss-Prot: Q15910.2, or a fragment thereof, and having methyltransferase activity. An exemplary H3K27 methyltransferase amino acid sequence is provided below:

1 mgqtgkksek gpvcwrkrvk seymrlrqlk rfrradevks mfssnrqkil erteilnqew
61 kqrriqpvhi ltsvsslrgt recsvtsdld fptqviplkt lnavasvpim yswsplqqnf
121 mvedetvlhn ipymgdevld qdgtfieeli knydgkvhgd recgfindei fvelvnalgq
181 yndddddddg ddpeereekq kdledhrddk esrpprkfps dkifeaissm fpdkgtaeel
241 kekykelteq qlpgalppec tpnidgpnak svqreqslhs fhtlfcrrcf kydcflhpfh
301 atpntykrkn tetaldnkpc gpqcyqhleg akefaaalta eriktppkrp ggrrrgrlpn
361 nssrpstpti nvleskdtds dreagtetgg enndkeeeek kdetssssea nsrcqtpikm
421 kpnieppenv ewsgaeasmf rvligtyydn fcaiarligt ktcrqvyefr vkessiiapa
481 paedvdtppr kkkrkhrlwa ahcrkiqlkk dgssnhvyny qpcdhprqpc dsscpcviaq
541 nfcekfcqcs secqnrfpgc rckaqcntkq cpcylavrec dpdlcltcga adhwdsknvs
601 ckncsiqrgs kkhlllapsd vagwgifikd pvqknefise ycgeiisqde adrrgkvydk
661 ymcsflfnln ndfvvdatrk gnkirfanhs vnpncyakvm mvngdhrigi fakraiqtge
721 elffdyrysq adalkyvgie remeip

By "EZH2 polynucleotide" is meant a nucleic acid molecule encoding an EZH2 polypeptide. An exemplary EZH2 polynucleotide sequence is provided at NM_001203248.1 and is provided below:

1 ggcggcgctt gattgggctg ggggggccaa ataaaagcga tggcgattgg gctgccgcgt
61 ttggcgctcg gtccggtcgc gtccgacacc cggtgggact cagaaggcag tggagccccg
121 gcggcggcgg cggcggcgcg cgggggcgac gcgcgggaac aacgcgagtc ggcgcgcggg
181 acgaagaata atcatgggcc agactgggaa gaaatctgag aagggaccag tttgttggcg
241 gaagcgtgta aaatcagagt acatgcgact gagacagctc aagaggttca gacgagctga
301 tgaagtaaag agtatgttta gttccaatcg tcagaaaatt ttggaaagaa cggaaatctt
361 aaaccaagaa tggaaacagc gaaggataca gcctgtgcac atcctgactt cttgttcggt
421 gaccagtgac ttggattttc caacacaagt catcccatta aagactctga atgcagttgc
481 ttcagtaccc ataatgtatt cttggtctcc cctacagcag aattttatgg tggaagatga

541 aactgtttta cataacattc cttatatggg agatgaagtt ttagatcagg atggtacttt
601 cattgaagaa ctaataaaaa attatgatgg gaaagtacac ggggatagag aatgtgggtt
661 tataaatgat gaaatttttg tggagttggt gaatgccctt ggtcaatata atgatgatga
721 cgatgatgat gatggagacg atcctgaaga aagagaagaa aagcagaaag atctggagga
781 tcaccgagat gataaagaaa gccgcccacc tcggaaattt ccttctgata aaatttttga
841 agccatttcc tcaatgtttc cagataaggg cacagcagaa gaactaaagg aaaaatataa
901 agaactcacc gaacagcagc tcccaggcgc acttcctcct gaatgtaccc ccaacataga
961 tggaccaaat gctaaatctg ttcagagaga gcaaagctta cactcctttc atacgctttt
1021 ctgtaggcga tgttttaaat atgactgctt cctacatcct tttcatgcaa cacccaacac
1081 ttataagcgg aagaacacag aaacagctct agacaacaaa ccttgtggac cacagtgtta
1141 ccagcatttg gagggagcaa aggagtttgc tgctgctctc accgctgagc ggataaagac
1201 cccaccaaaa cgtccaggag gccgcagaag aggacggctt cccaataaca gtagcaggcc
1261 cagcaccccc accattaatg tgctggaatc aaaggataca gacagtgata gggaagcagg
1321 gactgaaacg gggggagaga acaatgataa agaagaagaa gagaagaaag atgaaacttc
1381 gagctcctct gaagcaaatt ctcggtgtca aacaccaata aagatgaagc caaatattga
1441 acctcctgag aatgtggagt ggagtggtgc tgaagcctca atgtttagag tcctcattgg
1501 cacttactat gacaatttct gtgccattgc taggttaatt gggaccaaaa catgtagaca
1561 ggtgtatgag tttagagtca aagaatctag catcatagct ccagctcccg ctgaggatgt
1621 ggatactcct ccaaggaaaa agaagaggaa acaccggttg tgggctgcac actgcagaaa
1681 gatacagctg aaaaaggacg gctcctctaa ccatgtttac aactatcaac cctgtgatca
1741 tccacggcag ccttgtgaca gttcgtgccc ttgtgtgata gcacaaaatt tttgtgaaaa
1801 gttttgtcaa tgtagttcag agtgtcaaaa ccgctttccg ggatgccgct gcaaagcaca
1861 gtgcaacacc aagcagtgcc cgtgctacct ggctgtccga gagtgtgacc ctgacctctg
1921 tcttacttgt ggagccgctg accattggga cagtaaaaat gtgtcctgca agaactgcag
1981 tattcagcgg ggctccaaaa agcatctatt gctggcacca tctgacgtgg caggctgggg
2041 gatttttatc aaagatcctg tgcagaaaaa tgaattcatc tcagaatact gtggagagat
2101 tatttctcaa gatgaagctg acagaagagg gaaagtgtat gataaataca tgtgcagctt
2161 tctgttcaac ttgaacaatg attttgtggt ggatgcaacc cgcaagggta acaaaattcg
2221 ttttgcaaat cattcggtaa atccaaactg ctatgcaaaa gttatgatgg ttaacggtga
2281 tcacaggata ggtatttttg ccaagagagc catccagact ggcgaagagc tgttttttga
2341 ttacagatac agccaggctg atgccctgaa gtatgtcggc atcgaaagag aaatggaaat
2401 cccttgacat ctgctacctc ctcccccctc ctctgaaaca gctgccttag cttcaggaac
2461 ctcgagtact gtgggcaatt tagaaaaaga acatgcagtt tgaaattctg aatttgcaaa
2521 gtactgtaag aataatttat agtaatgagt ttaaaaatca actttttatt gccttctcac
2581 cagctgcaaa gtgttttgta ccagtgaatt tttgcaataa tgcagtatgg tacatttttc
2641 aactttgaat aaagaatact tgaacttgtc cttgttgaat c

By "KDM6A polypeptide" (lysine-specific demethylase 6A, also referred to as histone demethylase UTX) is meant a protein having at least about 85% amino acid identity to the sequence provided at NCBI Reference Sequence: 015550.2, or a fragment thereof, and having demethylase activity. An exemplary KDM6A amino acid sequence is provided below:

1 

61 
121 
181 
241 
301 
361 
421 
481 
541 
601 
661 
721 
781 
841 
901 
961 
1021 
1081 
1141 
1201 
1261 
1321 
1381 

By "KDM6A polynucleotide" is meant a nucleic acid molecule encoding a KDM6A polypeptide. An exemplary KDM6A polynucleotide sequence is provided at NM_001291415.1.

By "KDM6B polypeptide" (lysine-specific demethylase 6, also referred to as JmjC domain-containing protein 3) is meant a protein having at least about 85% amino acid identity to the sequence provided at NCBI Reference Sequence: 015054.4, or a fragment thereof, and having demethylase activity. An exemplary KDM6B amino acid sequence is provided below:

1 mhravdppga raareafalg glscagawss cpphppprsa wlpggrcsas igqpplpapl
61 ppshgsssgh pskpyyapga ptprplhgkl eslhgcvqal lrepaqpglw eqlgqlyese
121 hdseeatrcy hsalryggsf aelgprigrl qqaqlwnfht gscqhrakvl ppleqvwnll
181 hlehkrnyga krggppvkra aeppvvqpvp paalsgpsge eglspggkrr rgcnseqtgl
241 ppglplpppp lppppppppp pppplpglat sppfqltkpg lwstlhgdaw gperkgsapp
301 erqeqrhslp hpypypapay tahppghrlv paappgpgpr ppgaeshgcl patrppgsdl
361 resrvqrsrm dssvspaatt acvpyapsrp pglpgtttss ssssssntgl rgvepnpgip

421 gadhyqtpal evshhgrlgp sahssrkpfl gapaatphls lppgpssppp ppcprllrpp
481 pppawlkgpa craaredgei leelffgteg pprpappplp hregflgppa srfsvgtqds
541 htpptpptpt tsssnsnsgs hssspagpvs fppppylars idplprppsp aqnpqdpplv
601 pltlalppap psschqntsg sfrrpesprp rvsfpktpev gpgpppgpls kapqpvppgv
661 gelpargprl fdfpptpled qfeepaefki lpdglanimk mldesirkee eqqheagva
721 pqpplkepfa slqspfptdt aptttapava vttttttttt ttatqeeekk pppalppppp
781 lakfpppsqp qpppppppsp asllkslasv legqkycyrg tgaavstrpg plpttqyspg
841 ppsgatalpp tsaapsaqgs pqpsassssq fstsggpwar errageepvp gpmtptqppp
901 plslpparse sevleeisra cetlvervgr satdpadpvd taepadsgte rllppaqake
961 eaggvaavsg sckrrqkehq kehrrhrrac kdsvgrrpre grakakakvp keksrrvlgn
1021 ldlqseeiqg reksrpdlgg askakpptap appsapapsa qptppsasvp gkkareeapg
1081 ppgvsradml klrslsegpp kelkirlikv esgdketfia seveerrlrm adltishcaa
1141 dvvrasrnak vkgkfresyl spaqsvkpki nteeklprek lnpptpsiyl eskrdafspv
1201 llqfctdprn pitvirglag slrlnlglfs tktlveasge htvevrtqvq qpsdenwdlt
1261 gtrqiwpces srshttiaky aqyqassfqe slqeekesed eeseepdstt gtppssapdp
1321 knhhiikfgt nidlsdakrw kpqlqellkl pafmrvtstg nmlshvghti lgmntvqlym
1381 kvpgsrtpgh qennnfcsvn inigpgdcew favhehywet isafcdrhgv dyltgswwpi
1441 lddlyasnip vyrfvqrpgd lvwinagtvh wvqatgwcnn iawnvgplta yqyqlalery
1501 ewnevknvks ivpmihvswn vartvkisdp dlfkmikfcl lqsmkhcqvq reslvragkk
1561 iayqgrvkde payycnecdv evfnilfvts engsrntylv hcegcarrrs aglqgvvvle
1621 qyrteelaqa ydaftlapas tsr

By "KDM6B polynucleotide" is meant a nucleic acid molecule encoding a KDM6B polypeptide. An exemplary KDM6B polynucleotide sequence is provided at NM_001080424.2 and reproduced below:

1 ggcaacatgc cagccccgta gcactgccca ccccacccac tgtggtctgt tgtaccccac
61 tgctggggtg gtggttccaa tgagacaggg cacaccaaac tccatctggc tgttactgag
121 gcggagacac gggtgatgat tggctttctg gggagagagg aagtcctgtg attggccaga
181 tctctggagc ttgccgacgc ggtgtgagga cgctcccacg gaggccggaa ttggctgtga
241 aaggactgag gcagccatct gggggtagcg ggcactctta tcagagcggc tggagccgga
301 ccatcgtccc agagagctgg ggcagggggc cgtgcccaat ctccagggct cctgggccca
361 ctgctgacct ggctggatgc atcgggcagt ggaccctcca ggggcccgcg ctgcacggga
421 agcctttgcc cttgggggcc tgagctgtgc tggggcctgg agctcctgcc cgcctcatcc
481 ccctcctcgt agcgcatggc tgcctggagg cagatgctca gccagcattg ggcagccccc
541 gcttcctgct cccctacccc cttcacatgg cagtagttct gggcacccca gcaaaccata
601 ttatgctcca ggggcgccca ctccaagacc cctccatggg aagctggaat ccctgcatgg
661 ctgtgtgcag gcattgctcc gggagccagc ccagccaggg ctttgggaac agcttgggca
721 actgtacgag tcagagcacg atagtgagga ggccacacgc tgctaccaca gcgcccttcg
781 atacggagga agcttcgctg agctggggcc ccgcattggc cgactgcagc aggcccagct
841 ctggaacttt catactggct cctgccagca ccgagccaag gtcctgcccc cactggagca
901 agtgtggaac ttgctacacc ttgagcacaa acggaactat ggagccaagc ggggaggtcc

961 cccggtgaag cgagctgctg aacccccagt ggtgcagcct gtgcctcctg cagcactctc
1021 aggcccctca ggggaggagg gcctcagccc tggaggcaag cgaaggagag gctgcaactc
1081 tgaacagact ggccttcccc cagggctgcc actgcctcca ccaccattac caccaccacc
1141 accaccacca ccaccaccac caccacccct gcctggcctg gctaccagcc ccccatttca
1201 gctaaccaag ccagggctgt ggagtaccct gcatggagat gcctggggcc cagagcgcaa
1261 gggttcagca cccccagagc gccaggagca gcggcactcg ctgcctcacc catatccata
1321 cccagctcca gcgtacaccg cgcacccccc tggccaccgg ctggtcccgg ctgctccccc
1381 aggcccaggc cccgcccccc caggagcaga gagccatggc tgcctgcctg ccacccgtcc
1441 ccccggaagt gaccttagag agagcagagt tcagaggtcg cggatggact ccagcgtttc
1501 accagcagca accaccgcct gcgtgcctta cgccccttcc cggccccctg gcctccccgg
1561 caccaccacc agcagcagca gtagcagcag cagcaacact ggtctccggg gcgtggagcc
1621 gaacccaggc attcccggcg ctgaccatta ccaaactccc gcgctggagg tctctcacca
1681 tggccgcctg gggccctcgg cacacagcag tcggaaaccg ttcttggggg ctcccgctgc
1741 cactccccac ctatccctgc cacctggacc ttcctcaccc cctccacccc cctgtccccg
1801 cctcttacgc cccccaccac cccctgcctg gttgaagggt ccggcctgcc gggcagcccg
1861 agaggatgga gagatcttag aagagctctt ctttgggact gagggacccc cccgccctgc
1921 cccaccaccc ctcccccatc gcgagggctt cttggggcct ccggcctccc gcttttctgt
1981 gggcactcag gattctcaca cccctcccac tcccccaacc ccaaccacca gcagtagcaa
2041 cagcaacagt ggcagccaca gcagcagccc tgctgggcct gtgtcctttc ccccaccacc
2101 ctatctggcc agaagtatag acccccttcc ccggcctccc agcccagcac agaaccccca
2161 ggacccacct cttgtacccc tgactcttgc cctgcctcca gcccctcctt cctcctgcca
2221 ccaaaatacc tcaggaagct tcaggcgccc ggagagcccc cggcccaggg tctccttccc
2281 aaagaccccc gaggtggggc cggggccacc cccaggcccc ctgagtaaag ccccccagcc
2341 tgtgccgccc ggggttgggg agctgcctgc ccgaggccct cgactctttg attttccccc
2401 cactccgctg gaggaccagt ttgaggagcc agccgaattc aagatcctac ctgatgggct
2461 ggccaacatc atgaagatgc tggacgaatc cattcgcaag gaagaggaac agcaacaaca
2521 cgaagcaggc gtggcccccc aacccccgct gaaggagccc tttgcatctc tgcagtctcc
2581 tttccccacc gacacagccc ccaccactac tgctcctgct gtcgccgtca ccaccaccac
2641 caccaccacc accaccacca cggccaccca ggaagaggag aagaagccac caccagccct
2701 accaccacca ccgcctctag ccaagttccc tccaccctct cagccacagc caccaccacc
2761 cccacccccc agcccggcca gcctgctcaa atccttggcc tccgtgctgg agggacaaaa
2821 gtactgttat cgggggactg gagcagctgt ttccacccgg cctgggccct tgcccaccac
2881 tcagtattcc cctggccccc catcaggtgc taccgccctg ccgcccacct cagcggcccc
2941 tagcgcccag ggctccccac agccctctgc ttcctcgtca tctcagttct ctacctcagg
3001 cgggccctgg gcccgggagc gcagggcggg cgaagagcca gtcccgggcc ccatgacccc
3061 cacccaaccg cccccacccc tatctctgcc ccctgctcgc tctgagtctg aggtgctaga
3121 agagatcagc cgggcttgcg agacccttgt ggagcgggtg ggccggagtg ccactgaccc
3181 agccgaccca gtggacacag cagagccagc ggacagtggg actgagcgac tgctgccccc
3241 cgcacaggcc aaggaggagg ctggcggggt ggcggcagtg tcaggcagct gtaagcggcg
3301 acagaaggag catcagaagg agcatcggcg gcacaggcgg gcctgtaagg acagtgtggg
3361 tcgtcggccc cgtgagggca gggcaaaggc caaggccaag gtccccaaag aaaagagccg
3421 ccgggtgctg gggaacctgg acctgcagag cgaggagatc cagggtcgtg agaagtcccg
3481 gcccgatctt ggcggggcct ccaaggccaa gccacccaca gctccagccc ctccatcagc
3541 tcctgcacct tctgcccagc ccacaccccc gtcagcctct gtccctggaa agaaggctcg

3601 ggaggaagcc ccagggccac cgggtgtcag ccgggccgac atgctgaagc tgcgctcact
3661 tagtgagggg ccccccaagg agctgaagat ccggctcatc aaggtagaga gtggtgacaa
3721 ggagaccttt atcgcctctg aggtggaaga gcggcggctg cgcatggcag acctcaccat
3781 cagccactgt gctgctgacg tcgtgcgcgc cagcaggaat gccaaggtga aagggaagtt
3841 tcgagagtcc tacctttccc ctgcccagtc tgtgaaaccg aagatcaaca ctgaggagaa
3901 gctgccccgg gaaaaactca acccccctac acccagcatc tatctggaga gcaaacggga
3961 tgccttctca cctgtcctgc tgcagttctg tacagaccct cgaaatccca tcacagtgat
4021 ccggggcctg gcgggctccc tgcggctcaa cttgggcctc ttctccacca agaccctggt
4081 ggaagcgagt ggcgaacaca ccgtggaagt tcgcacccag gtgcagcagc cctcagatga
4141 gaactgggat ctgacaggca ctcggcagat ctggccttgt gagagctccc gttcccacac
4201 caccattgcc aagtacgcac agtaccaggc ctcatccttc caggagtctc tgcaggagga
4261 gaaggagagt gaggatgagg agtcagagga gccagacagc accactggaa cccctcctag
4321 cagcgcacca gacccgaaga accatcacat catcaagttt ggcaccaaca tcgacttgtc
4381 tgatgctaag cggtggaagc cccagctgca ggagctgctg aagctgcccg ccttcatgcg
4441 ggtaacatcc acgggcaaca tgctgagcca cgtgggccac accatcctgg gcatgaacac
4501 ggtgcagctg tacatgaagg tgcccggcag ccgaacgcca ggccaccagg agaataacaa
4561 cttctgctcc gtcaacatca acattggccc aggcgactgc gagtggttcg cggtgcacga
4621 gcactactgg gagaccatca gcgctttctg tgatcggcac ggcgtggact acttgacggg
4681 ttcctggtgg ccaatcctgg atgatctcta tgcatccaat attcctgtgt accgcttcgt
4741 gcagcgaccc ggagacctcg tgtggattaa tgcggggact gtgcactggg tgcaggccac
4801 cggctggtgc aacaacattg cctggaacgt ggggcccctc accgcctatc agtaccagct
4861 ggccctggaa cgatacgagt ggaatgaggt gaagaacgtc aaatccatcg tgcccatgat
4921 tcacgtgtca tggaacgtgg ctcgcacggt caaaatcagc gaccccgact tgttcaagat
4981 gatcaagttc tgcctgctgc agtccatgaa gcactgccag gtgcaacgcg agagcctggt
5041 gcgggcaggg aagaaaatcg cttaccaggg ccgtgtcaag gacgagccag cctactactg
5101 caacgagtgc gatgtggagg tgtttaacat cctgttcgtg acaagtgaga atggcagccg
5161 caacacgtac ctggtacact gcgagggctg tgcccggcgc cgcagcgcag gcctgcaggg
5221 cgtggtggtg ctggagcagt accgcactga ggagctggct caggcctacg acgccttcac
5281 gctggtgagg gcccggcggg cgcgcgggca gcggaggagg gcactggggc aggctgcagg
5341 gacgggcttc gggagcccgg ccgcgccttt ccctgagccc ccgccggctt tctcccccca
5401 ggccccagcc agcacgtcgc gatgaggccg gacgccccgc ccgcctgcct gcccgcgcaa
5461 ggcgccgcgg ggccaccagc acatgcctgg gctggaccta ggtcccgcct gtggccgaga
5521 agggggtcgg gcccagccct tccaccccat tggcagctcc cctcacttaa tttattaaga
5581 aaaacttttt tttttttttt agcaaatatg aggaaaaaag gaaaaaaaat gggagacggg
5641 ggagggggct ggcagcccct cgcccaccag cgcctcccct caccgacttt ggccttttta
5701 gcaacagaca caaggaccag gctccggcgg cggcgggggt cacatacggg ttccctcacc
5761 ctgccagccg cccgcccgcc cggcgcagat gcacgcggct cgtgtatgta catagacgtt
5821 acggcagccg aggtttttaa tgagattctt tctatgggct ttacccctcc cccggaacct
5881 ccttttttac ttccaatgct agctgtgacc cctgtacatg tctctttatt cacttggtta
5941 tgatttgtat tttttgttct tttcttgttt ttttgttttt aatttataac agtcccactc
6001 acctctattt attcattttt gggaaaaccc gacctcccac acccccaagc catcctgccc
6061 gcccctccag ggaccgcccg tcgccgggct ctccccgcgc cccagtgtgt gtccgggccc
6121 ggcccgaccg tctccacccg tccgcccgcg gctccagccg ggttctcatg gtgctcaaac
6181 ccgctcccct cccctacgtc ctgcactttc tcggaccagt ccccccactc ccgacccgac

6241 cccagcccca cctgagggtg agcaactcct gtactgtagg ggaagaagtg ggaactgaaa
6301 tggtattttg taaaaaaaat aaataaaata aaaaaattaa aggttttaaa gaaagaacta
6361 tgaggaaaag gaaccccgtc cttcccagcc ccggccaact ttaaaaaaca cagaccttca
6421 cccccacccc cttttctttt taagtgtgaa acaacccagg gccagggcct cactggggca
6481 gggacacccc ggggtgagtt tctctggggc tttattttcg ttttgttggt tgttttttct
6541 ccacgctggg gctgcggagg ggtggggggt ttacagtccc gcaccctcgc actgcactgt
6601 ctctctgccc caggggcaga ggggtcttcc caaccctacc cctattttcg gtgatttttg
6661 tgtgagaata ttaatattaa aaataaacgg agaaaaaaaa aaaaaaaaaa aaaaaaaaaa
6721 aaaaaaaaaa a

By "KDM6C polypeptide" (histone demethylase UTY, also referred to as ubiquitously-transcribed TPR protein on the Y chromosome) is meant a protein having at least about 85% amino acid identity to the sequence provided at NCBI Reference Sequence: 014607.2, or a fragment thereof, and having demethylase activity. An exemplary KDM6C amino acid sequence is provided below:

1 mkscavsltt aavafgdeak kmaegkasre seeesvsltv eerealggmd srlfgfvrlh
61 edgartktll gkavrcyesl ilkaegkves dffcqlghfn llledyskal sayqryyslq
121 adywknaafl yglglvyfyy nafhwaikaf qdvlyvdpsf crakeihlrl glmfkvntdy
181 ksslkhfqla lidcnpctls naeiqfhiah lyetqrkyhs akeayeqllq tenlpaqvka
241 tvlqqlgwmh hnmdlvgdka tkesyaiqyl qksleadpns gqswyflgrc yssigkvqda
301 fisyrqsidk seasadtwcs igvlyqqqnq pmdalqayic avqldhghaa awmdlgtlye
361 scnqpqdaik cylnaarskr csntstlaar ikflqngsdn wnggqslshh pvqqvyslcl
421 tpqklqhleq lranrdnlnp aqkhqleqle sqfvlmqqmr hkevaqvrtt gihngaitds
481 slptnsvsnr qphgaltrvs svsqpgvrpa cvekllssga fsagcipcgt skilgstdti
541 llgsnciags esngnvpylq qnthtlphnh tdlnssteep wrkqlsnsaq glhksqsscl
601 sgpneeqplf stgsaqyhqa tstgikkane hltlpsnsvp qgdadshlsc htatsggqqg
661 imftkeskps knrslvpets rhtgdtsngc advkglsnhv hqliadavss pnhgdspnll
721 iadnpqlsal ligkangnvg tgtcdkvnni hpavhtktdh svasspssai statpspkst
781 eqrsinsvts lnsphsglht vngeglgksq sstkvdlpla shrstsqilp smsvsicpss
841 tevlkacrnp gknglsnsci lldkcppprp ptspypplpk dklnpptpsi ylenkrdaff
901 pplhqfctnp knpvtvirgl agalkldlgl fstktlvean nehmvevrtq llqpadenwd
961 ptgtkkiwrc esnrshttia kyaqyqassf qeslreenek rtqhkdhsdn estssensgr
1021 rrkgpfktik fgtnidlsdn kkwklqlhel tklpafarvv sagnllthvg htilgmntvq
1081 lymkvpgsrt pghqennnfc svninigpgd cewfvvpedy wgvlndfcek nnlnflmssw
1141 wpnledlyea nvpvyrfiqr pgdlvwinag tvhwvqavgw cnniawnvgp ltacqyklav
1201 eryewnklks vkspvpmvhl swnmarnikv sdpklfemik ycllkilkqy qtlrealvaa
1261 gkeviwhgrt ndepahycsi cevevfnllf vtnesntqkt yivhchdcar ktskslenfv
1321 vleqykmedl iqvydqftla lslssss

By "KDM6C polynucleotide is meant a nucleic acid molecule encoding a KDM6C polypeptide. An exemplary KDM6A polynucleotide sequence is provided at NM_001258249.1, which sequence is reproduced below:

1 gctcatcgtt tgttgtttag ataatatcat gaactgataa atgcagttgc cacgttgatt
61 ccctagggcc tggcttaccg actgaggtca taagatatta tgccttctct ttagacttgg
121 tcagtggaga ggaaatgggc aaagaaccag cctatggagg tgacaaggcc ttagggccaa
181 aagtcttgag ggtgaaggtt tagggcctgc gcagcttccc tgccatgccc cgcaaggtct
241 cgcattcgca aggcttgtga cagtgggagc ctcattacgg actctcctaa agtccatggt
301 gtcctctttt cgcatttgcg ccccgtgggt gatgcccgat gccgcccttc ccatcgctct
361 cttccccttc aagcgtatcg caactgcaaa aacacccagc acagacactc cattttctat
421 cttaatgcat ttaactagca caacctacag gttgttccat cccagagact acccttttct
481 ccatagacgt gaccatcaac caaccagcgg tcagaatcag tcagcctctg tcatgttcct
541 aggtccttgg cgaactggct gggcggggtc ccagcagcct aggagtacag tggagcaatg
601 cctgacgtaa gtcaacaaag atcacgtgag acgaatcagt cgcctagatt ggctacaact
661 aagtggttgg gagcggggag gtcgcggcgg ctgcgtgggg ttcgcccgtg acacaattac
721 aactttgtgc tggtgctggc aaagtttgtg attttaagaa attctgctgt gctctccagc
781 actgcgagct tctgccttcc ctgtagtttc ccagatgtga tccaggtagc cgagttccgc
841 tgcccgtgct tcggtagctt aagtctttgc ctcagctttt ttccttgcag ccgctgagga
901 ggcgataaaa ttggcgtcac agtctcaagc agcgattgaa ggcgtctttt caactactcg
961 attaaggttg ggtatcgtcg tgggacttgg aaatttgttg tttccatgaa atcctgcgca
1021 gtgtcgctca ctaccgccgc tgttgccttc ggtgatgagg caaagaaaat ggcggaagga
1081 aaagcgagcc gcgagagtga agaggagtct gttagcctga cagtcgagga aagggaggcg
1141 cttggtggca tggacagccg tctcttcggg ttcgtgaggc ttcatgaaga tggcgccaga
1201 acgaagaccc tactaggcaa ggctgttcgc tgctacgaat ctttaatctt aaaagctgaa
1261 ggaaaagtgg agtctgactt cttttgccaa ttaggtcact tcaacctctt gttggaagat
1321 tattcaaaag cattatctgc atatcagaga tattacagtt tacaggctga ctactggaag
1381 aatgctgcgt ttttatatgg ccttggtttg gtctacttct actacaatgc atttcattgg
1441 gcaattaaag catttcaaga tgtcctttat gttgacccca gcttttgtcg agccaaggaa
1501 attcatttac gacttgggct catgttcaaa gtgaacacag actacaagtc tagtttaaag
1561 cattttcagt tagccttgat tgactgtaat ccatgtactt tgtccaatgc tgaaattcaa
1621 tttcatattg cccatttgta tgaaacccag aggaagtatc attctgcaaa ggaggcatat
1681 gaacaacttt tgcagacaga aaaccttcct gcacaagtaa aagcaactgt attgcaacag
1741 ttaggttgga tgcatcataa tatggatcta gtaggagaca aagccacaaa ggaaagctat
1801 gctattcagt atctccaaaa gtctttggag gcagatccta attctggcca atcgtggtat
1861 tttcttggaa ggtgttattc aagtattggg aaagttcagg atgcctttat atcttacagg
1921 caatctattg ataaatcaga agcaagtgca gatacatggt gttcaatagg tgtgttgtat
1981 cagcagcaaa atcagcctat ggatgcttta caggcatata tttgtgctgt acaattggac
2041 catgggcatg ccgcagcctg gatggaccta ggtactctct atgaatcctg caatcaacct
2101 caagatgcca ttaaatgcta cctaaatgca gctagaagca aacgttgtag taatacctct
2161 acgcttgctg caagaattaa atttctacag gctcagttgt gtaaccttcc acaaagtagt
2221 ctacagaata aaactaaatt acttcctagt attgaggagg catggagcct accaatcccc
2281 gcagagctta cctccaggca gggtgccatg aacacagcac agcaggctta tagagctcat
2341 gatccaaata ctgaacatgt attaaaccac agtcaaacac caattttaca gcaatccttg

2401 tcactacaca tgattacttc tagccaagta gaaggcctgt ccagtcctgc caagaagaaa
2461 agaacatcta gtccaacaaa gaatggttct gataactgga atggtggcca gagtctttca
2521 catcatccag tacagcaagt ttattcgttg tgtttgacac cacagaaatt acagcacttg
2581 gaacaactgc gagcaaatag agataattta aatccagcac agaagcatca gctggaacag
2641 ttagaaagtc agtttgtctt aatgcagcaa atgagacaca aagaagttgc tcaggtacga
2701 actactggaa ttcataacgg ggccataact gattcatcac tgcctacaaa ctctgtctct
2761 aatcgacaac cacatggtgc tctgaccaga gtatctagcg tctctcagcc tggagttcgc
2821 cctgcttgtg ttgaaaaact tttgtccagt ggagcttttt ctgcaggctg tattccttgt
2881 ggcacatcaa aaattctagg aagtacagac actatcttgc taggcagtaa ttgtatagca
2941 ggaagtgaaa gtaatggaaa tgtgccttac ctgcagcaaa atacacacac tctacctcat
3001 aatcatacag acctgaacag cagcacagaa gagccatgga gaaaacagct atctaactcc
3061 gctcaggggc ttcataaaag tcagagttca tgtttgtcag gacctaatga agaacaacct
3121 ctgttttcca ctgggtcagc ccagtatcac caggcaacta gcactggtat taagaaggcg
3181 aatgaacatc tcactctgcc tagtaattca gtaccacagg gggatgctga cagtcacctc
3241 tcctgtcata ctgctacctc aggtggacaa caaggcatta tgtttaccaa agagagcaag
3301 ccttcaaaaa atagatcctt ggtgcctgaa acaagcaggc atactggaga cacatctaat
3361 ggctgtgctg atgtcaaggg actttctaat catgttcatc agttgatagc agatgctgtt
3421 tccagtccta accatggaga ttcaccaaat ttattaattg cagacaatcc tcagctctct
3481 gctttgttga ttggaaaagc caatggcaat gtgggtactg gaacctgtga caaagtgaat
3541 aatattcacc cagctgttca tacaaagact gatcattctg ttgcctcttc accctcttca
3601 gccatttcca cagcaacacc ttctcctaaa tccactgagc agagaagcat aaacagtgtt
3661 accagcctta acagtcctca cagtggatta cacacagtca atggagaggg gctggggaag
3721 tcacagagct ctacaaaagt agacctgcct ttagctagcc acagatctac ttctcagatc
3781 ttaccatcaa tgtcagtgtc tatatgcccc agttcaacag aagttctgaa agcatgcagg
3841 aatccaggta aaaatggctt gtctaatagc tgcattttgt tagataaatg tccacctcca
3901 agaccaccaa cttcaccata cccacccttg ccaaaggaca agttgaatcc acccacacct
3961 agtatttact tggaaaataa acgtgatgct ttctttcctc cattacatca attttgtaca
4021 aatccaaaaa accctgttac agtaatacgt ggccttgctg gagctcttaa attagatctt
4081 ggacttttct ctaccaaaac tttggtagaa gctaacaatg aacatatggt agaagtgagg
4141 acacagttgc tgcaaccagc agatgaaaac tgggatccca ctggaacaaa gaaaatctgg
4201 cgttgtgaaa gcaatagatc tcatactaca attgccaaat acgcacaata ccaggcttcc
4261 tccttccagg aatcattgag agaagaaaat gagaaaagaa cacaacacaa agatcattca
4321 gataacgaat ccacatcttc agagaattct ggaaggagaa ggaaaggacc ttttaaaacc
4381 ataaaatttg ggaccaacat tgacctctct gataacaaaa agtggaagtt gcagttacat
4441 gaactgacta aacttcctgc ttttgcgcgt gtggtgtcag caggaaatct tctaacccat
4501 gttgggcata ccattctggg catgaataca gtacaactgt atatgaaagt tccagggagt
4561 cggacaccag gtcaccaaga aaataacaac ttctgctctg ttaacataaa tattggtcca
4621 ggagattgtg aatggtttgt tgtacctgaa gattattggg gtgttctgaa tgacttctgt
4681 gaaaaaaata atttgaattt tttaatgagt tcttggtggc ccaaccttga agatctttat
4741 gaagcaaatg tccctgtgta tagatttatt cagcgacctg gagatttggt ctggataaat
4801 gcaggcactg tgcattgggt tcaagctgtt ggctggtgca ataacattgc ctggaatgtt
4861 ggtccactta cagcctgcca gtataaattg gcagtggaac ggtatgaatg gaacaaattg
4921 aaaagtgtga agtcaccagt acccatggtg catctttcct ggaatatggc acgaaatatc
4981 aaagtctcag atccaaagct ttttgaaatg attaagtatt gtcttttgaa aattctgaag

5041 caatatcaga cattgagaga agctcttgtt gcagcaggaa aagaggttat atggcatggg
5101 cggacaaatg atgaaccagc tcattactgt agcatttgtg aggtggaggt ttttaatctg
5161 ctttttgtca ctaatgaaag caatactcaa aaaacctaca tagtacattg ccatgattgt
5221 gcacgaaaaa caagcaaaag tttggaaaat tttgtggtgc tcgaacagta caaaatggag
5281 gacctaatcc aagtttatga tcaatttaca ctagctcttt cattatcatc ctcatcttga
5341 tatagttcca tgaatattaa atgagattat ttctgctctt caggaaattt ctgcaccact
5401 ggttttgtag ctgtttcata aaactgttga ctaaaagcta tgtctatgca accttccaag
5461 aatagtatgt caagcaactg gacacagtgc tgcctctgct tcaggactta acatgctgat
5521 ccagctgtac ttcagaaaaa taatattaat catatgtttt gtgtacgtat gacaaactgt
5581 caaagtgaca cagaatactg atttgaagat agcctttttt atgtttctct atttctgggc
5641 tgatgaatta atattcattt gtattttaac cctgcagaat tttccttagt taaaaacact
5701 ttcctagctg gtcatttctt cataagatag caaatttaaa tctctcctcg atcagctttt
5761 aaaaaatgtg tactattatc tgaggaagtt ttttactgct ttatgttttt gtgtgttttg
5821 aggccatgat gattacattt gtggttccaa aataattttt ttaaatatta atagcccata
5881 tacaaagata atggattgca catagacaaa gaaataaact tcagatttgt gatttttgtt
5941 tctaaacttg atacagattt acactattta taaatacgta tttattgcct gaaaatattt
6001 gtgaatggaa tgttgttttt ttccagacgt aactgccatt aaatactaag gagttctgta
6061 gttttaaaca ctactcctat tacattttat atgtgtagat aaaactgctt agtattatac
6121 agaaattttt attaaaattg ttaaatgttt aaagggtttc ccaatgtttg agtttaaaaa
6181 agactttctg aaaaaatcca ctttttgttc attttcaaac ctaatgatta tatgtatttt
6241 atatgtgtgt gtatgtgtac acacatgtat aatatataca gaaacctcga tatataattg
6301 tatagatttt aaaagtttta ttttttacat ctatggtagt ttttgaggtg cctattataa
6361 agtattacgg aagtttgctg tttttaaagt aaatgtcttt tagtgtgatt tattaagttg
6421 tagtcaccat agtgatagcc cataaataat tgctggaaaa ttgtatttta taacagtaga
6481 aaacatatag tcagtgaagt aaatatttta aaggaaacat tatatagatt tgataaatgt
6541 tgtttataat taagagtttc ttatggaaaa gagattcaga atgataacct cttttagaga
6601 acaaataagt gacttatttt tttaaagcta gatgactttg aaatgctata ctgtcctgct
6661 tgtacaacat ggtttggggt gaaggggagg aaagtattaa aaaatctata tcgctagtaa
6721 attgtaataa gttctattaa aacttgtatt tcatatgaaa aatttgctaa tttaatatta
6781 actcatttga taataatact tgtcttttct acctctc

By "Gab1 polypeptide" (GRB2-associated-binding protein 1) is meant a protein having at least about 85% amino acid identity to the sequence provided at NCBI Reference Sequence: NP_997006.1, or a fragment thereof. An exemplary Gab1 amino acid sequence is provided below:

1 msggevvcsg wlrksppekk lkryawkrrw fvlrsgrltg dpdvleyykn dhakkpirii
61 dlnlcqqvda gltfnkkefe nsyifdinti drifylvads eeemnkwvrc icdicgfnpt
121 eedpvkppgs slqapadlpl aintappstq adsssatlpp pyqlinvpph letlgiqedp
181 qdylllincq skkpeptrth adsakstsse tdcndnvpsh knpassqskh gmngffqqqm
241 iydsppsrap sasvdsslyn lprsyshdvl pkvspsstea dgelyvfntp sgtssvetqm
301 rhvsisydip ptpgntyqip rtfpegtlgq tskldtipdi ppprppkphp ahdrspvetc
361 siprtasdtd ssyciptagm spsrsntist vdlnklrkda ssqdcydipr afpsdrsssl

421 egfhnhfkvk nvltvgsvss eeldenyvpm npnspprqhs ssftepiqea nyvpmtpgtf

481 dfssfgmqvp ppahmgfrss pktpprrpvp vadcepppvd rnlkpdrkgq spkilrlkph

541 glertdsqti gdfatrrkvk papleikplp eweelqapvr spitrsfard ssrfpmsprp

601 dsvhsttsss dshdseenyv pmnpnlssed pnlfgsnsld ggsspmikpk gdkqveyldl

661 dldsgkstpp rkqkssgsgs svadervdyv vvdqqktlal kstreawtdg rqstesetpa

721 ksvk

By "Gab1 polynucleotide" is meant a nucleic acid molecule encoding a Gab1 polypeptide. An exemplary Gab1 polynucleotide sequence is provided at NM_002039.3, which is reproduced below:

1 agggggcgga gcgcaaagga cagaagctcc ggcaccgagt cggggcagag tcccgctgag
61 tccgagcgct gctgaggcag ctggcgagac ggcacgtctg gaggcgaggc gggcgcactg
121 aaaggaggcc ggcgcgcccg cggccccggc tcgcgttctg ttcaggttcg tgggcctgca
181 gaggagagac tcgaactcgt ggaacccgcg caccgtggag tctgtccgcc cagtccgtcc
241 ggggtgcgcg accaggagag ctaggttctc gccactgcgc gctcggcagg cgtcggctgt
301 gtcgggagcg cgcccgccgc ccctcagctg cccggcccgg agcccgagac gcgcgcacca
361 tgagcggtgg tgaagtggtc tgctccggat ggctccgcaa gtcccccccg gagaaaaagt
421 tgaagcgtta tgcatggaag aggagatggt tcgtgttacg cagtggccgt ttaactggag
481 atccagatgt tttggaatat tacaaaaatg atcatgccaa gaagcctatt cgtattattg
541 atttaaattt atgtcaacaa gtagatgctg gattgacatt taacaaaaaa gagtttgaaa
601 acagctacat ttttgatatc aacactattg accggatttt ctacttggta gcagacagcg
661 aggaggagat gaataagtgg gttcgttgta tttgtgacat ctgtgggttt aatccaacag
721 aagaagatcc tgtgaagcca cctggcagct ctttacaagc accagctgat ttacctttag
781 ctataaatac agcaccacca tccacccagg cagattcatc ctctgctact ctacctcctc
841 catatcagct aatcaatgtt ccaccacacc tggaaactct tggcattcag gaggatcctc
901 aagactacct gttgctcatc aactgtcaaa gcaagaagcc cgaacccacc agaacgcatg
961 ctgattctgc aaaatccacc tcttctgaaa cagactgcaa tgataacgtc ccttctcata
1021 aaaatcctgc ttcctcccag agcaaacatg gaatgaatgg cttttttcag cagcaaatga
1081 tatacgactc tccaccttca cgtgccccat ctgcttcagt tgactccagc ctttataacc
1141 tgcccaggag ttattcccat gatgttttac caaaggtgtc tccatcaagt actgaagcag
1201 atggagaact ctatgttttt aataccccat ctgggacatc gagtgtagag actcaaatga
1261 ggcatgtatc tattagttat gacattcctc caacacctgg taatacttat cagattccac
1321 gaacatttcc agaaggaacc ttgggacaga catcaaagct agacactatt ccagatattc
1381 ctccacctcg gccaccgaaa ccacatccag ctcatgaccg atctcctgtg gaaacgtgta
1441 gtatcccacg caccgcctca gacactgaca gtagttactg tatccctaca gcagggatgt
1501 cgccttcacg tagtaatacc atttccactg tggatttaaa caaattgcga aaagatgcta
1561 gttctcaaga ctgctatgat attccacgag catttccaag tgatagatct agttcacttg
1621 aaggcttcca taaccacttt aaagtcaaaa atgtgttgac agtgggaagt gtttcaagtg
1681 aagaactgga tgaaaattac gtcccaatga atcccaattc accaccacga caacattcca
1741 gcagttttac agaaccaatt caggaagcaa attatgtgcc aatgactcca ggaacatttg
1801 atttttcctc atttggaatg caagttcctc ctcctgctca tatgggcttc aggtccagcc
1861 caaaaacccc tcccagaagg ccagttcctg ttgcagactg tgaaccaccc cccgtggata

1921 ggaacctcaa gccagacaga aaagtcaagc cagcgccttt agaaataaaa cctttgccag
1981 aatgggaaga attacaagcc ccagttagat ctcccatcac taggagtttt gctcgagact
2041 cttccaggtt tcccatgtcc ccccgaccag attcagtgca tagcacaact tcaagcagtg
2101 actcacacga cagtgaagag aattatgttc ccatgaaccc aaacctgtcc agtgaagacc
2161 caaatctctt tggcagtaac agtcttgatg gaggaagcag ccctatgatc aagcccaaag
2221 gagacaaaca ggtggaatac ttagatctcg acttagattc tgggaaatcc acaccaccac
2281 gtaagcaaaa gagcagtggc tcaggcagca gtgtagcaga tgagagagtg gattatgttg
2341 ttgttgacca acagaagacc ttggctctaa agagtacccg ggaagcctgg acagatggga
2401 gacagtccac agaatcagaa acgccagcga agagtgtgaa atgaaaatat tgccttgcca
2461 tttctgaaca aaagaaaact gaattgtaaa gataaatccc ttttgaagaa tgacttgaca
2521 cttccactct aggtagatcc tcaaatgagt agagttgaag tcaaaggacc tttctgacat
2581 aatcaagcaa tttagactta agtggtgctt tgtggtatct gaacaattca taacatgtaa
2641 ataatgtggg aaaatagtat tgtttagctc ccagagaaac atttgttcca cagttaacac
2701 actcgtagta ttactgtatt tatgcacttt ttcatctaaa acattgttct gggttttccc
2761 aatgtacctt accataattc ctttgggagt tcttgttttt tgtcacacta ctttatataa
2821 caatactaag tcaactaagc tacttttaga tttggaaatt gctgtttaca gtctaacaac
2881 attaaaatga gaggtagatt cacaagttag ctttctacct gaagcttcag gtgataacca
2941 ttagcttata cttggactca tcatttgttg ccttccaaaa tgctgaggat aatgtatgta
3001 ctggtgtcag gacctagttc tctggttaat gtacatttag tttttaatgg tggaactttg
3061 ttatattttg ttaattacag tgtttttggt tcattgagtg aagattctgc cgggtgggat
3121 cttgcacctt tgaaagactg aataattaca ctaccaagta agcctgcaaa tcattgatgg
3181 catgcagtga tgatgtgctc ttacacttgt taacatgtat taagtgttat ttgcaaaagg
3241 tagattatgt aaccaatcag gtacgtacca ggcagtgatg tgctaataca ctgatcaggt
3301 ttagacaatg agctttggtt gtgttcttgt tagtcctaat attggttttc agtttggaat
3361 taataaagca gttgacattc actgttagtt acagcaacat actgtgattt ttaattagat
3421 agtaattcag atttattact ctatgaaatt ctgtcttttg acaccatagt gccctttcta
3481 tgattttttt tacttaatat tcttcttggc cttatattta attccctatg caattaatat
3541 tttatatctg cattttttta aaaaaaatag atgttatata agtgattctc gtatgtagca
3601 cctgttgctt ttccactgaa agaattacgg attttgtact gtgatttata ttcactgccc
3661 caattcaaga aatattggag ccttgctaca atgtgaaatg ttatagtcat ggactccttc
3721 caaccagatt tctgaaaaca ccagagggat ggtataattc tgtctcacct ataacatggt
3781 cctgtgacat agatattaag accacaagtt gtagtgaggc tacaattata ttcgtctgtc
3841 ttggctttgc aacataattt agaaagcacg tatagttgtt ttttaaccaa gttacataca
3901 atctcatgta ctgatttgag acttataaca atttttggag gggcatagaa gaaaggagtg
3961 cccacagttg aggcatgacc ccctccattc agacctctaa ctgttgcctg agtacacaga
4021 tgtgccctga tttctggccc attggccata gtactgtgcc taatcaatgt aataggttta
4081 ttttcccaat cctcaaacta aaaatgttca taacaagatg aattgtagac tagtaacatt
4141 tgatgctttt aaatatttgc ttctttttaa acaaaaacta aaacccagaa gtgaattttt
4201 aggtggattt ttaaataaaa aagattgatt gagtttggtg tgcaagctgt tttataatga
4261 aacaacaaaa tgaaatctaa aatcctgaaa tgtgcctaaa ctatcaaaac acacgataca
4321 gctaatgtgt aaagatgcta aattctgtta cttggaggat gaatatattt aagatttaaa
4381 acacaataat aaatacatga ttaattcaaa aataaaaatc tttacagctg cctatcaagg
4441 gtctaaagca cttaatgaat gtttttagtc taacttatca ttaacttttt acaagtcacc
4501 atatttgaag atctgtagca ctctgatttt cagaaaattt ttcattctga ataatttaaa

4561 aatggtgatg tattagaaag gcagtttgct ttagaaaact aaatcacatt gaacattgta
4621 ttagagaatt aaattaaaag tttcttacag agcagtattt tccaaacatt tttagcacta
4681 gaatcttttt agatgaaatt ttatgtataa ccccaataca taaagcctga aaactcaatt
4741 ttatcaatat aaatgtattt tgggttcaca tttatgctta ttcattttgg ctcattacta
4801 agcataataa gattctgagt tatttctgaa taacacaaat gtggagttat acatagttga
4861 tgaaaccagc agccaattta tagctatgcc ctgttttatt tgtatactat caagaaaatt
4921 ttgattcaca caaatgtaag caaaaataat aggttttaaa catacatctc aggaaattct
4981 ttaattagag atagctaaag ttattcaagg tctatacaaa aataagttat cctggtagtg
5041 gaagttaata cataagcagt ctccagtgtg gtaaagtagg gtatgtaaca catcagaatg
5101 tgcgttttta ttaggtttta aaatatgcac gtataaaaac taaatttgaa tcaaaccctt
5161 ttaactcacc tccaagaagc tagactttgg ccaggaatgg gctaaaaacc actggttaac
5221 gatgtgacag ttatgatctt ggagattgga aatctttctt ccacattaga gttctttacc
5281 ttaattcctt attctgaaaa attgtaagat tttatgaagg tttgaatact gaagcacagt
5341 tctgctttca aaaattaaaa ttcaaacttg aaaaagctgt ttaacccatg gaagatatca
5401 tttagtaaga tgtaaaagat tttttaaatc tacacttcag tttatacatc tttatcatta
5461 tcaatactat ataagttact gtgagcattt tagagaattc cataaaggta ctatgagtgt
5521 gtctgtatgt gtgtgtatat atagcattgt atttaatcat agactaaatt taatttgata
5581 tagaaatact actttacttg tacattaagg tcataatttc tgctggactc ttttatattt
5641 aattaatggg gattatagtc ttccttcata aatgcattta aacctgaaat tgaacaccag
5701 tgtttttctt tttctactta tgggaagttg tctgcttccc cctttagaga aaacagtatt
5761 tttatatttt gttaaaatat taactacttt atgcctacac actatgctgt agatactgat
5821 cataattctt gggtgttcac aaacactcct agtgcctctt ttttggcccg ttgaaagtgt
5881 tggtattact actttcacta cagagccttt ggccctctaa taatgctgag gtgggctgat
5941 ccttcccatt tctgtcttcg ggtcattctg gtaggtcttc tcctccactg tcaagtaagc
6001 aatcaggtcc gtgacaggga ttggacatat gaacaaatta agtggataca cacagtgaga
6061 aagatacatg cattctatgg taacaactac tgtcaataac atctgatgtt acatgcacat
6121 ttatatatat ataattttaa aaactgaact atgagaagcc atggtataaa tgaatattgt
6181 ggacatcatg gacttgatat gatagaaatc aattgtcagc ttgagaaagt tgtttttaat
6241 ctgtctaaat agttcatgca ttactacagt taaaaatagt ttcatttgtc ttctatagac
6301 ttaattttat tccggttcag tataatctct gttaacagag tttcagcaaa ctgattggtc
6361 aaggtattaa catagcttct acttccttta cttaaaaaga tgtggtttta tgtaagttct
6421 tgattactga tgatcatccc aaattttgac aacaaaatca tatgtataaa tttatttctc
6481 ccctcttgtt catcatcttt tgtaaaggtc ccattgtaga tcttttctgc taccaaataa
6541 aacttttcaa acaatttggt ttcaagacct taaatagaca agttggatac taagattgtg
6601 aactgataag gacatataaa tttatatttc cagcccttcc ttagagtctt tatctgcatc
6661 aaaaacccaa ttctgccatt aactgtgctt cccagtccca cctctatatg tcactcattt
6721 tctgcaacaa agatctcact aaatcatgtt gaaacacaag tcatgatcct ctctaagtaa
6781 atagaaaaag ctccctggaa aaactctgtt gccacatgca cgtgccctgt tactcctcca
6841 gccagccagt gctgccagca ttttattgtg taaaagtcca aataaataag ggcctgcatg
6901 caacctttat cttcagaaac taggttttat atgtaaaatg tgacttggga aatgattctg
6961 tttattaact ggctgggatt tttcatttct atgaaagttt caaacatctc cagtacttta
7021 taaaatccca acaattgctg taagtcagca ctttggtcca ctcagcccac ccagcccact
7081 tgcaactctg actcttcact gaatcatatt tgggaagttt gggtagggtg aggctatctt
7141 cttcaagatt attttctcat atgtctgtct gtcaccttgt aaaccatgag actcctgggt

7201 atttgcatgt aacttctttg aggaagttac caccatctct gatatagaca cactttttga
7261 gttgcagttt ctgttagaat tttttggaga ctaacttgcc aattctgtga atgttattga
7321 atatttaaaa agctgggtct gtaatgggag gcattttatt agctgttgtg attgggtaac
7381 atgtcccctt agatttcctg atttaaaatt atacaaaatt actatttttg ataaaataaa
7441 ggaacaccta cagaaaatta agtttctaag atgtttctat acttcattag aaaagatttt
7501 attactatta cttatggtta ttggtgatta acacttaatg cgtctcctct gattttgtgt
7561 tccatgaggt gcttggaaca tttggagtgc tctgtgcgag ggacatacag tgatatagga
7621 aatttaaaaa ttaaaataat acccaaaacc cactttatca gatatggtat tgtgatggtt
7681 aatattatgt gtcaacttgg tgaggctatg gcgcccatgt gtttggtcaa acactagcct
7741 agatgttgct gtgaatatat tttgtagatg tgattaacat ttacaatcag ttgattttaa
7801 gtaaagcaga ttctcatcca aaaaaaaaaa aaaaaa

By "Sfmbt2 polypeptide" (scm-like with four MBT domains protein 2) is meant a protein having at least about 85% amino acid identity to the sequence provided at NCBI Reference Sequence: NP_001018049.1, or a fragment thereof. An exemplary Sfmbt2 amino acid sequence is provided below:

1 mestlsasnm qdpsssplek clgsangngd ldseegssle etgfnwgeyl eetgasaaph
61 tsfkhveisi qsnfqpgmkl evanknnpdt ywvatiittc gqllllrycg ygedrradfw
121 cdvviadlhp vgwctqnnkv lmppdaikek ytdwteflir dltgsrtapa nllegplrgk
181 gpidlitvgs lielqdsqnp fqywivsvie nvggrlrlry vgledtesyd qwlfyldyrl
241 rpvgwcqenk yrmdppseiy plkmasewkc tlekslidaa kfplpmevfk dhadlrshff
301 tvgmkletvn mcepfyispa svtkvfnnhf fqvtiddlrp epsklsmlch adslgilpvq
361 wclkngvslt ppkgysgqdf dwadyhkqhg aqeappfcfr ntsfsrgftk nmkleavnpr
421 npgelcvasv vsvkgrlmwl hleglqtpvp evivdvesmd ifpvgwcean sypltaphkt
481 vsqkkrkiav vqpekqlppt vpvkkiphdl clfphldttg tvngkyccpq lfinhrcfsg
541 pylnkgriae lpqsvgpgkc vlvlkevlsm iinaaykpgr vlrelqlved phwnfqeetl
601 kakyrgktyr avvkivrtsd qvanfcrrvc akleccpnlf spvlisencp encsihtktk
661 ytyyygkrkk iskppigesn pdsghpkpar rrkrrksifv qkkrrssavd ftagsgeese
721 eedadamddd taseetgsel rddqtdtssa evpsarprra vtlrsgsepv rrpppertrr
781 grgapaassa eegekcpptk pegtedtkqe eeerlvlesn plewtvtdvv rfikltdcap
841 lakifqeqdi dgqalllltl ptvqecmelk lgpaiklchq iervkvafya qyan

By "Sfmbt2 polynucleotide" is meant a polypeptide encoding an Sfmbt2 polypeptide. An exemplary Sfmbt2 polynucleotide sequence is provided at NM_001018039.1, which is reproduced below:

1 cgccttgtgt gtgctggatc ctgcgcgggt agatccccga gtaatttttt ctgcaggatg
61 aattaagaga agagacactt gctcatcagg catggagagc actttgtcag cttccaatat
121 gcaagaccct tcatcttcac ccttggaaaa gtgtctcggc tcagctaatg gaaatggaga
181 ccttgattct gaagaaggct caagcttgga ggaaactggc tttaactggg gagaatattt
241 ggaagagaca ggagcaagtg ctgctcccca cacatcattc aaacacgttg aaatcagcat

301 tcagagcaac ttccagccag gaatgaaatt ggaagtggct aataagaaca acccggacac
361 gtactgggtg gccacgatca ttaccacgtg cgggcagctg ctgcttctgc gctactgcgg
421 ttacggggag gaccgcaggg ccgacttctg gtgtgacgta gtcatcgcgg atttgcaccc
481 cgtggggtgg tgcacacaga acaacaaggt gttgatgccg ccggacgcaa tcaaagagaa
541 gtacacagac tggacagaat ttctcatacg tgacttgact ggttcgagga cagcacccgc
601 caacctcctg gaaggtcctc tgcgagggaa aggccctata gacctcatta cagttggttc
661 cttaatagaa cttcaggatt cccagaaccc ttttcagtac tggatagtta gtgtgattga
721 aaatgttgga ggaagattac gccttcgcta tgtgggattg gaggacactg aatcctatga
781 ccagtggttg ttttacttgg attacagact tcgaccagtt ggttggtgtc aagagaataa
841 atacagaatg gacccacctt cagaaatcta tcctttgaag atggcctctg aatggaaatg
901 tactctggaa aaatccctta ttgatgctgc caaatttcct cttccaatgg aagtgtttaa
961 ggatcacgca gatttgcgaa gccatttctt cacagttggg atgaagcttg agacagtgaa
1021 tatgtgcgag cccttttaca tctctcctgc gtcggtgact aaggttttta acaatcactt
1081 ttttcaagtg actattgatg acctaagacc tgaaccaagt aaactgtcaa tgctgtgcca
1141 tgcagattct ttggggattt tgccagtaca gtggtgcctt aaaaatggag tcagcctcac
1201 tcctcccaaa ggttactctg gccaggactt cgactgggca gattatcaca agcagcatgg
1261 ggcgcaggaa gcccctccct tctgcttccg aaatacatca ttcagtcgag gtttcacaaa
1321 gaacatgaaa cttgaagctg tgaaccccag gaatccagga gaactgtgtg tggcctccgt
1381 tgtgagtgtg aaggggcggc taatgtggct tcacctggaa gggctgcaga ctcctgttcc
1441 agaggtcatt gttgatgtgg aatccatgga catcttccca gtgggctggt gtgaagccaa
1501 ttcttatcct ttgactgcac cacacaaaac agtctcacaa aagaagagaa agattgcagt
1561 cgtgcaacca gagaaacaat tgccgcccac agtgcctgtt aagaaaatac ctcatgacct
1621 ttgtttattc cctcacctgg acaccacagg aaccgtcaac gggaaatact gctgtcctca
1681 gctcttcatc aaccacaggt gtttctcagg cccttacctg aacaaaggaa ggattgcaga
1741 gctacctcag tcggtgggac cgggcaaatg cgtgctggtt cttaaagagg ttcttagcat
1801 gataatcaac gcagcctaca agcctggaag ggtattaaga gaattacagc tggtagaaga
1861 tccccactgg aatttccagg aagagacgct gaaggccaaa tacagaggca aaacatacag
1921 ggctgtggtc aaaatcgtac ggacatctga ccaagtcgca aatttctgcc gccgagtctg
1981 tgccaagcta gagtgctgtc caaatttgtt tagtcctgtg ctgatatctg aaaactgccc
2041 agagaactgc tccattcata ccaaaaccaa atacacctat tactatggaa agagaaagaa
2101 gatctccaag ccccccatcg gggaaagcaa ccccgacagc ggacacccca aacccgccag
2161 gcggaggaag cgacggaaat ccattttcgt gcagaagaaa cggaggtctt ctgccgtgga
2221 cttcaccgcg ggctcggggg aggaaagtga agaggaggac gctgacgcca tggacgatga
2281 caccgccagt gaggagaccg gctccgagct ccgggatgac cagacggaca cctcgtcggc
2341 ggaggtgccc tcggcccggc cccggagggc cgtcaccctg cggagcggct cagagcccgt
2401 gcgccggcca cccccagaga ggacacgaag ggccgcgggg gcgccggctg cctcctcagc
2461 agaggaaggg gagaagtgcc cgccgaccaa gcccgagggg acagaggaca cgaaacagga
2521 ggaggaggag agactggttc tggagagcaa cccgttggag tggacggtca ccgacgtggt
2581 gaggttcatt aagctgacag actgtgcccc cttggccaag atatttcagg agcaggatat
2641 tgacggccaa gcactcctgc ttctgaccct tccgacggtg caggagtgca tggagctgaa
2701 gctggggcct gccatcaagt tatgccacca gatcgagaga gtcaaagtgg ctttctacgc
2761 ccagtacgcc aactgagtct gccctcggga ggtggcccat tattgctggg atgcggtgtt
2821 ggtaaaggtt tccaggactg aaactttgat tttccgggat atgttaaatg gtacagccac
2881 taagtatcac cagaaaacca gaagcccagg atcttctgcc tccgccagcc tgtgagctgt

2941 ttccatgttt tcaaagcaca gcagcagtcg cttctgggga gtgccagtta aagtcatgca
3001 tcagaccctg ccagacgtgg gcctgcttct tggctcaccc acgttttgcc tttctcctgc
3061 cccaaatcag gcagctccct tggagcaggg tttcctcaga tgaggactgc attctttgaa
3121 aacaaagaat gtcgccaagg aagaaacctc acgccatgct gtagtgtttc ctgtaatcac
3181 acgagcacat ttatatatgc agtttcccat ggataggcgt gtgaccctgg ttgagtggca
3241 cttgcggttt catcttggtg gcaactcctt tgcaatgcag ctggcagcga catccttata
3301 aaaacatgtg ctaaagctct gtcctctgtt agaggtgcct tttaggaata cggggagtga
3361 aggaaggccg gcaggcatct ccatgcaact agatggtttg tttgtttgtt tgtttgtttg
3421 ttgttcattt tgttgtgttt tttgagacag ggtcttgctc tgtcgcccag gttgtaatgc
3481 agtggcgcaa tctcagctca ctgcaacctc tctctcccgg gttcaagtga ttctcctgcc
3541 tcagcctccc aagtagctgg gattacaggc acccaccacc atgcctggct aatttttgta
3601 tttttggtag agacagggtt tcaccatgtt ggtcaggcta gtcttgaact cccaacctca
3661 agtgatctgc ccgcctcggc ctcccaacgt gctgggatta caggtgtgag ccactacgcc
3721 ccggcccaac tggatggttt ttgattgaag cctagaacat ctgtagagac aaactctacc
3781 cagtcttttc tagaccctca actatctcca gtgttgttgt ttaatcgtag ccggatcagg
3841 gagtgagtct tttaggcaaa tgttggatta tatatcaaag gaaaagctta gtttcagaga
3901 ggaggaaggg aaagagatgt gagggaagca tttcatcaac cagctacgtc ccccttagaa
3961 ggatcactgc agcaggtcac cgagcaggag tccctctgag cgtcccttct gtctcgttct
4021 gccctagctg gcagcatatg aaccaggcat gatgcagcag gagcagtgaa tctggagtca
4081 gccacttggc accctggttt cgctgagaac aaactctgag atcttgggtg acttctcatc
4141 actctggacc tccattcctg tgaagtgaca ggtgtggacc ctgagggtgc ggtggtgagc
4201 acactgtctc ctgctggcat tcaccccact catgctggaa aggaagatcc agatcgtaca
4261 aaaattagaa aaagaaagaa taagaagggt ctggtcccag ttctgactcg gccattctta
4321 cagctctttc tggctttgag tttgcttgtg gaatttcctg ggcagttgtg ttaaatccgc
4381 caggtcacgt gcagacaaag ctgtggctgc gagagttggc tggcctcttg gaccagaagc
4441 catctccata tcctcatgag cgattccata tctccactca gaccctgtgg actacagtgt
4501 tccgctgtgg tggctgccaa gatgccttct taaacttatg caaggaaacc aaaccctccc
4561 acagttccca agcagacact ggaagcagag gcttctcacc cttcctgctt tttcaccaca
4621 atcaccttga gctcgtccct tggactagag tctccacagt tccagtaaaa ttctgcggtg
4681 ggctgatgag ctgcttgcat ttctgtgaca tttccagata tgattctcag tgggattttg
4741 gaaactttga ttgctcaagc tcacccttct taacattctg taatggttac agatgagaat
4801 ggaaaacaca tattttatgg atgaggcgtt ttggtctccc ctgcagtcga tttctagaat
4861 caagttttag agttcggctg atgcatctgc ctggggacct cagatgggag gagtgtgtca
4921 gttgtacccc gacagaaatg tctctgggat ctgtggctgg cttgccccgg gcatctctcc
4981 tttaagctca agttttgaac tctctgcggt tttccacccc tgccttctca gccacatgct
5041 tttggcctta aacgctcagt cttgtggagt tcaactctgt caaacgattg gaaagggcat
5101 ccatttccag atctttggca ttttccccgc gctgactctt tgatgatcct tcactgtggc
5161 cttttcaagc tcagctgttc ctgttgtatt tgagacgagg gtgagggaat gtggtggcca
5221 caaaagaaca gggacttgca gcacaaatgt cacttctgtc tcccttttca gtggtagcac
5281 ggaggaggag gtgctgcgtt ggagggaggg gatcctccag gagctctctg gagcccatct
5341 aggaagctag agtgtgtggc ccgccaggag ctcaggaagg atacagccac tgtcgcaggg
5401 gaaagtgttt gcttcccgtg gagccaagcg cccaagactc tccgtatcct tcaccctgac
5461 agtttaactt cagcgtttct ctgtgcagtt gcggtcacca tgggtgagca ctgtctgtgc
5521 acgtgccagg gaggagatgg ctgggaccac tgcacaggag ggcgcagcct ggcgtcgcca

5581 tgaaagttgt ctctgtgcca tctctccggt ccttgaggag agcccagaaa gattttagga
5641 cccaggaggt gcttttcctc cagctgttgc cagtgtcctt ctgagcctgg attctccggg
5701 gatttccgtc gtggtggatg gacttcacat cagcagcagt tctggtacag aattgtaatg
5761 tgttttcatt tctctgtagg attcacctct caccagcgtc tgtcttaaag gtagggccaa
5821 tttcatggag catttttctg tgtgtgtcct tgttgctttt gccagaaaaa gtggatttga
5881 catgcgtgcc ccgatgccac catagcccct aggccaacaa tgtcatggtc taaacaccaa
5941 aaagtgatgc cccgcattcc ttccctggat ggtaccgttt cttctccgtc tctctttgat
6001 gattctttgg gaccaaagtc ctctccttag tgcgcctact tcctgtgggc atcatgccac
6061 ttggaactta ttggaactgg cccgggagac tctgcagtct gcgccgtttg aaaaccctga
6121 gaaagagatg ccacctcaac ttgaatcatg acagcccatc gctcagtctc accctaaact
6181 catggagctt gtttcagctc ctcacttctt gactgtattt gtactatgtt gaaaaaatat
6241 cctgtccaca aagacataag cctaacaacc tagaaaaaca acagggtact actggcatta
6301 cagaacttct ttgcctttca aaacaaaagc aaaacacagt gaacttcacc acggagctgc
6361 acagcgtggg gaactcatcc atcactttca aaattagagt catttgatcc aagttggagt
6421 cagacacagt atttgagctg cacggcttct gggttctccc accttatttg atcatattcg
6481 aaagattatt tcctgtgttt gctttgattt gttcctcagt acattaaaat gatccacacc
6541 ttgaacactg ccctctctag aaggttgatt ttgatcagcc ttttgaagat gggtgtcgtt
6601 tccctaactt atctcacaga attttgagtg ttgtatttgg caagttctga gatttgcctt
6661 ctgtcttatg ccaaacaccc ctttctaaga gctgtccccg cttagtttta gaagtactag
6721 gggttttcat acttatttta tagaacaccc atttatattt atttctgtat atagaactaa
6781 aaaaaacagt agtgttaaaa atctttgttg tggtttgagc atctttgctg cttttggatt
6841 gagatggcga atcaaggctt cacttcctct ctcttctgtc tttagaaagc tgtgatcgtg
6901 cgtgcaatta tttgaaaggc aacatagtca attaagaaac ctgtagttgt taaggaagaa
6961 attgttggca agatatccat actgcccata tctcgttggt gcaataatta aatagcaaag
7021 gaaatctgta ttggcaacta ttataattca ataattcttt tgtttactgc ccttttctgt
7081 tcaagaattt tctggaaatt actccctttc acatggttga actcttaagt tgaccagttc
7141 tcatagctct atcactagaa tggtttgcag ataccccaaa catactatga taaaatcaaa
7201 ttgtgctact tttgacccat gtaatttacc taaaagttgt aattgctgac agagtactgc
7261 cttgaatttt ggtttaaaac ctctctagtt tcaatgacaa gtaacaactc aaataattcc
7321 atattgtttg aggaagaggc cataatcctt ctgaattgtt ggcactaagt aatgggattt
7381 ggcccagtaa gtatgacggt cgtgtcgcct aaccaacgca gagcagtgct tttgtgtgg
7441 ctgaagcgat gtgctgacga aaaaaggaaa attctaggac aatcgttggc taaaaatcac
7501 cttaggatga aaaatttgag gcaaattttt ttaaatgaca gaaaaagata atcatctcac
7561 ttgcttgaaa caggagccag catgatctct ggaagcatca actatccctc gtcgtgattg
7621 ttgaaagctc tttcactgtt ttgcattcta gtttgaatag tttgtattga aattggattc
7681 ctatcttgtg tatgtttttg gtgcgtaaaa gggaaaaatt ggtgtcatta cttttgaaat
7741 ttgcaggacg aagggcatgc ttttggtttg ctgtaagatt gtattctgta tatatgtttt
7801 catgtaaata aatgaaaatc tatatcagag ttatatttta atttttattc taaatgaaaa
7861 aaaccctttt tacttcaaaa aaattgtaag ccacattgtt aataaagtaa aaataaattc
7921 ta

By "Smoc1 polypeptide" (SPARC related modular calcium binding 1) is meant a protein having at least about 85% amino acid identity to the sequence provided at NCBI Reference Sequence: NP_001030024, or a fragment thereof.. An exemplary Smoc1 amino acid sequence is provided below:

1 mlparcarll tphlllvlvq lsparghrtt gprflisdrd pqcnlhcsrt qpkpicasdg
61 rsyesmceyq rakcrdptlg vvhrgrckda gqskcrlera qaleqakkpq eavfvpecge
121 dgsftqvqch tytgycwcvt pdgkpisgss vqnktpvcsg svtdkplsqg nsgrkddgsk
181 ptptmetqpv fdgdeitapt lwikhlvikd sklnntnirn sekvyscdqe rqsaleeaqq
241 npregivipe capgglykpv qchqstgycw cvlvdtgrpl pgtstryvmp scesdarakt
301 teaddpfkdr elpgcpegkk mefitsllda lttdmvqain saaptgggrf sepdpshtle
361 ervvhwyfsq ldsnssndin kremkpfkry vkkkakpkkc arrftdycdl nkdkvislpe
421 lkgclgvske vgrlv

By "Smoc1 polynucleotide" is meant a nucleic acid molecule encoding a Smoc1 polypeptide. An exemplary Smoc1 polynucleotide sequence is provided at XM_005267995.1, which is reproduced below:

1 ataacgggaa ttcccatggc ccgggctcag gcgtccaacc tgctgccgcc tgggccccgc
61 cgagcggagc tagcgccgcg cgcagagcac acgctcgcgc tccagctccc ctcctgcgcg
121 gttcatgact gtgtcccctg accgcagcct ctgcgagccc ccgccgcagg accacggccc
181 gctccccgcc gccgcgaggg ccccgagcga aggaaggaag ggaggcgcgc tgtgcgcccc
241 gcggagcccg cgaaccccgc tcgctgccgg ctgcccagcc tggctggcac catgctgccc
301 gcgcgctgcg cccgcctgct cacgccccac ttgctgctgg tgttggtgca gctgtcccct
361 gctcgcggcc accgcaccac aggccccagg tttctaataa gtgaccgtga cccacagtgc
421 aacctccact gctccaggac tcaacccaaa cccatctgtg cctctgatgg caggtcctac
481 gagtccatgt gtgagtacca gcgagccaag tgccgagacc cgaccctggg cgtggtgcat
541 cgaggtagat gcaaagatgc tggccagagc aagtgtcgcc tggagcgggc tcaagccctg
601 gagcaagcca agaagcctca ggaagctgtg tttgtcccag agtgtggcga ggatggctcc
661 tttacccagg tgcagtgcca tacttacact gggtactgct ggtgtgtcac cccggatggg
721 aagcccatca gtggctcttc tgtgcagaat aaaactcctg tatgttcagg ttcagtcacc
781 gacaagccct tgagccaggg taactcagga aggaaagtct cctttcgatt ctttttaacc
841 ctcaattcag atgacgggtc taagccgaca cccacgatgg agacccagcc ggtgttcgat
901 ggagatgaaa tcacagcccc aactctatgg attaaacact tggtgatcaa ggactccaaa
961 ctgaacaaca ccaacataag aaattcagag aaagtctatt cgtgtgacca ggagaggcag
1021 agtgccctgg aagaggccca gcagaatccc cgtgagggta ttgtcatccc tgaatgtgcc
1081 cctgggggac tctataagcc agtgcaatgc caccagtcca ctggctactg ctggtgtgtg
1141 ctggtggaca cagggcgccc gctgcctggg acctccacac gctacgtgat gcccagttgt
1201 gagagcgacg ccagggccaa gactacagag gcggatgacc ccttcaagga cagggagcta
1261 ccaggctgtc cagaagggaa gaaaatggag tttatcacca gcctactgga tgctctcacc
1321 actgacatgg ttcaggccat taactcagca gcgcccactg gaggtgggag gttctcagag
1381 ccagacccca gccacaccct ggaggagcgg gtagtgcact ggtatttcag ccagctggac
1441 agcaatagca gcaacgacat taacaagcgg gagatgaagc ccttcaagcg ctacgtgaag
1501 aagaaagcca agcccaagaa atgtgcccgg cgtttcaccg actactgtga cctgaacaaa
1561 gacaaggtca tttcactgcc tgagctgaag ggctgcctgg gtgttagcaa agaagtagga
1621 cgcctcgtct aaggagcaga aaacccaagg gcaggtggag agtccaggga ggcaggatgg

1681 atcaccagac acctaacctt cagcgttgcc catggccctg ccacatcccg tgtaacataa
1741 gtggtgccca ccatgtttgc acttttaata actcttactt gcgtgttttg tttttggttt
1801 cattttaaaa caccaatatc taataccaca gtgggaaaag gaaagggaag aaagacttta
1861 ttctctctct tattgtaagt ttttggatct gctactgaca acttttagag ggttttgggg
1921 gggtggggga gggtgttgtt ggggctgaga agaaagagat ttatatgctg tatataaata
1981 tatatgtaaa ttgtatagtt cttttgtaca ggcattggca ttgctgtttg tttatttctc
2041 tccctctgcc tgctgtgggt ggtgggcact ctggacacat agtccagctt tctaaaatcc
2101 aggactctat cctgggccta ctaaacttct gtttggagac tgacccttgt gtataaagac
2161 gggagtcctg caattgtact gcggactcca cgagttcttt tctggtggga ggactatatt
2221 gccccatgcc attagttgtc aaaattgata agtcacttgg ctctcggcct tgtccaggga
2281 ggttgggcta aggagagatg gaaactgccc tgggagagga agggagtcca gatcccatga
2341 atagcccaca caggtaccgg ctctcagagg gtccgtgcat tcctgctctc cggaccccca
2401 aagggcccag cattggtggg tgcaccagta tcttagtgac cctcggagca aattatccac
2461 aaaggatttg cattacgtca ctcgaaacgt tttcatccat gcttagcatc tactctgtat
2521 aacgcatgag aggggaggca aagaagaaaa agacacacag aagggccttt aaaaaagtag
2581 atatttaata tctaagcagg ggaggggaca ggacagaaag cctgcactga ggggtgcggt
2641 gccaacaggg aaactcttca cctccctgca aacctaccag tgaggctccc agagacgcag
2701 ctgtctcagt gccaggggca gattgggtgt gacctctcca ctcctccatc tcctgctgtt
2761 gtcctagtgg ctatcacagg cctgggtggg tgggttgggg gaggtgtcag tcaccttgtt
2821 ggtaacacta aagttgtttt gttggttttt taaaaaccca atactgaggt tcttcctgtt
2881 ccctcaagtt ttcttatggg cttccaggct ttaagctaat tccagaagta aaactgatct
2941 tgggtttcct attctgcctc ccctagaagg gcaggggtga taacccagct acagggaaat
3001 cccggcccag ctttccacag gcatcacagg catcttccgc ggattctagg gtgggctgcc
3061 cagccttctg gtctgaggcg cagctccctc tgcccaggtg ctgtgcctat tcaagtggcc
3121 ttcaggcaga gcagcaagtg gcccttagcg ccccttccca taagcagctg tggtggcagt
3181 gagggaggtt gggtagccct ggactggtcc cctcctcaga tcacccttgc aaatctggcc
3241 tcatcttgta ttccaacccg acatccctaa aagtacctcc acccgttccg ggtctggaag
3301 gcgttggcac cacaagcact gtccctgtgg gaggagcaca accttctcgg gacaggatct
3361 gatggggtct tgggctaaag gaggtccctg ctgtcctgga gaaagtccta gaggttatct
3421 caggaatgac tggtggccct gccccaacgt ggaaaggtgg gaaggaagcc ttctcccatt
3481 agccccaatg agagaactca acgtgccgga gctgagtggg ccttgcacga gacactggcc
3541 ccactttcag gcctggagga agcatgcaca catggagacg gcgcctgcct gtagatgttt
3601 ggatcttcga gatctcccca ggcatcttgt ctcccacagg atcgtgtgtg taggtggtgt
3661 tgtgtggttt tcctttgtga aggagagagg gaaactattt gtagcttgtt ttataaaaaa
3721 taaaaaatgg gtaaatcttg

By "tri-methylated histone H3 at lysine 27 (H3K27me3)" is meant the trimethylation of lysine 27 on histone H3 protein subunit. The H3K27me3 modification is generally associated with gene repression.

By "agent" is meant a peptide, nucleic acid molecule, or small compound.

By "allele" is meant one of two or more alternative forms of a gene that are found at the same place on a chromosome.

By "alteration" is meant a change (increase or decrease) in the expression levels or activity of a gene or polypeptide as detected by standard art known methods such as those described herein. As used herein, an alteration includes a 10% change in expression levels, preferably a 25% change, more preferably a 40% change, and most preferably a 50% or greater change in expression levels. "

By "ameliorate" is meant decrease, suppress, attenuate, diminish, arrest, or stabilize the development or progression of a disease.

In this disclosure, "comprises," "comprising," "containing" and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Detect" refers to identifying the presence, absence or amount of the analyte to be detected.

By "disease" is meant any condition or disorder that damages, or interferes with the normal function of a cell, tissue, or organ. Examples of disorders include those associated with undesirable repression of an allele by H3K27me3-dependent imprinting. Microphthalmia exemplary disorder associated with H3K27me3-dependent imprinting relating to imprinting disorders.

By "DNA" is meant deoxyribonucleic acid. In various embodiments, the term DNA refers to genomic DNA, recombinant DNA, or cDNA. In particular embodiments, the DNA comprises a "target region." DNA libraries contemplated herein include genomic DNA libraries, and cDNA libraries constructed from RNA, *e.g.,* an RNA expression library. In various embodiments, the DNA libraries comprise one or more additional DNA sequences and/or tags.

By "effective amount" is meant the amount of a required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, or 100, 200, 300, 400, 500, 600, 700, 800, 900, or 1000 nucleotides or amino acids.

The terms "isolated," "purified," or "biologically pure" refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of this invention is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

By "isolated polynucleotide" is meant a nucleic acid (e.g., a DNA) that is free of the genes which, in the naturally-occurring genome of the organism from which the nucleic acid molecule of the invention is derived, flank the gene. The term therefore includes, for example, a recombinant DNA that is incorporated into a vector; into an autonomously replicating plasmid or virus; or into the genomic DNA of a prokaryote or eukaryote; or that exists as a separate molecule (for example, a cDNA or a genomic or cDNA fragment produced by PCR or restriction endonuclease digestion) independent of other sequences. In addition, the term includes an RNA molecule that is transcribed from a DNA molecule, as well as a recombinant DNA that is part of a hybrid gene encoding additional polypeptide sequence.

By an "isolated polypeptide" is meant a polypeptide of the invention that has been separated from components that naturally accompany it. Typically, the polypeptide is isolated when it is at least 60%, by weight, free from the proteins and naturally-occurring organic molecules with which it is naturally associated. Preferably, the preparation is at least 75%, more preferably at least 90%, and most preferably at least 99%, by weight, a polypeptide of the invention. An isolated polypeptide of the invention may be obtained, for example, by extraction from a natural source, by expression of a recombinant nucleic acid encoding such a polypeptide; or by chemically synthesizing the protein. Purity can be measured by any appropriate method, for example, column chromatography, polyacrylamide gel electrophoresis, or by HPLC analysis.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

By "reduces" is meant a negative alteration of at least 10%, 25%, 50%, 75%, or 100%.

By "reference" is meant a standard or control condition.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 .mu.g/ml denatured salmon sperm DNA (ssDNA). In a most preferred embodiment, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and even more preferably of at least about 68° C. In a preferred embodiment, wash steps will occur at 25° C in 30 mM NaCl, 3 mM trisodium citrate, and 0.1% SDS. In a more preferred embodiment, wash steps will occur at 42 C in 15 mM NaCl, 1.5 mM trisodium citrate, and 0.1% SDS. In a more preferred embodiment, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

By "Somatic Cell Nuclear Transfer" or "SCNT" is meant the transfer of a donor nucleus from a somatic cell into an enucleated oocyte. The process can be used in either reproductive or therapeutic cloning and may be accomplished by fusion of the somatic cell with the enucleated oocyte, injection of the nucleus into the enucleated oocyte, or by any other method. In the context of the present technology, the somatic cell and the enucleated oocyte are non-human.

The nucleus of the somatic cell provides the genetic information, while the oocyte provides the nutrients and other energy-producing materials that are necessary for development of an embryo. Once fusion has occurred, the cell is totipotent, and eventually develops into a blastocyst, at which point the inner cell mass is isolated.

The term "nuclear transfer" as used herein refers to a gene manipulation technique allowing an identical characteristics and qualities acquired by artificially combining an enucleated oocytes with a cell nuclear genetic material or a nucleus of a somatic cell. In some embodiments, the nuclear transfer procedure is where a nucleus or nuclear genetic material from a donor somatic cell is transferred into an enucleated egg or oocyte (an egg or oocyte from which the nucleus/pronuclei have been removed). The donor nucleus can come from a somatic cell. In the context of the present technology, the somatic cell and the enucleated oocyte are non-human.

The term "nuclear genetic material" refers to structures and/or molecules found in the nucleus which comprise polynucleotides (e.g., DNA) which encode information about the individual. Nuclear genetic material includes the chromosomes and chromatin. The term also refers to nuclear genetic material (e.g., chromosomes) produced by cell division such as the division of a parental cell into daughter cells. Nuclear genetic material does not include mitochondrial DNA.

The term "SCNT embryo" refers to a cell, or the totipotent progeny thereof, of an enucleated oocyte which has been fused with the nucleus or nuclear genetic material of a somatic cell. The SCNT embryo can develop into a blastocyst and develop post-implantation into living offspring. The SCNT embryo can be a 1-cell embryo, 2-cell embryo, 4-cell embryo, or any stage embryo prior to becoming a blastocyst. In the context of the present technology, the SCNT embryo is non-human,

The term "somatic cell" refers to a plant or animal cell which is not a reproductive cell or reproductive cell precursor. In some embodiments, a differentiated cell is not a germ cell. A somatic cell does not relate to pluripotent or totipotent cells. In some embodiments the somatic cell is a "non-embryonic somatic cell", by which is meant a somatic cell that is not present in or obtained from an embryo and does not result from proliferation of such a cell *in vitro.* In some embodiments the somatic cell is an "adult somatic cell", by which is meant a cell that is present in or obtained from an organism other than an embryo or a fetus or results from proliferation of such a cell *in vitro.* In the context of the present invention, the somatic cell is non-human.

The term "oocyte" as used herein refers to a mature oocyte which has reached metaphase II of meiosis. An oocyte is also used to describe a female gamete or germ cell involved in reproduction, and is commonly also called an egg. A mature egg has a single set of maternal chromosomes (23, X in a human primate) and is halted at metaphase II. In the context of the present technology, the oocyte is non-human.

A "hybrid oocyte" refers to an enucleated oocyte that has the cytoplasm from a first human oocyte (termed a "recipient") but does not have the nuclear genetic material of the recipient oocyte; it has the nuclear genetic material from another human cell, termed a "donor." In some embodiments, the hybrid oocyte can also comprise mitochondrial DNA (mtDNA) that is not from the recipient oocyte, but is from a donor cell (which can be the same donor cell as the nuclear genetic material, or from a different donor, e.g., from a donor oocyte). In the context of the present technology, the oocyte is non-human.

The term "enucleated oocyte" as used herein refers to a non-human oocyte which its nucleus has been removed.

The term "enucleation" as used herein refers to a process whereby the nuclear material of a cell is removed, leaving only the cytoplasm. When applied to an egg, enucleation refers to the removal of the maternal chromosomes, which are not surrounded by a nuclear membrane. The term "enucleated oocyte" refers to an oocyte where the nuclear material or nuclei is removed.

The term "fusion" as used herein refers to a combination of a nuclear donor cell and a lipid membrane of a recipient oocyte. For example, the lipid membrane may be the plasma membrane or nuclear membrane of a cell. Fusion may occur upon application of an electrical stimulus between a nuclear donor cell and a recipient oocyte when they are placed adjacent to each other or when a nuclear donor cell is placed in a perivitelline space of a recipient oocyte.

The term "living offspring" as used herein means a non-human animal that can survive *ex utero.* Preferably, it is an animal that can survive for one second, one minute, one day, one week, one month, six months or more than one year. The animal may not require an in utero environment for survival.

The term "prenatal" refers to existing or occurring before birth. Similarly, the term "postnatal" is existing or occurring after birth.

The term "blastocyst" as used herein refers to a preimplantation non-human embryo in placental mammals (about 3 days after fertilization in the mouse, about 5 days after fertilization in humans) of about 30-150 cells. The blastocyst stage follows the morula stage, and can be distinguished by its unique morphology. The blastocyst consists of a sphere made up of a layer of cells (the trophectoderm), a fluid-filled cavity (the blastocoel or blastocyst cavity), and a cluster of cells on the interior (the inner cell mass, or ICM). The ICM, consisting of undifferentiated cells, gives rise to what will become the fetus if the blastocyst is implanted in a uterus. These same ICM cells, if grown in culture, can give rise to embryonic stem cell lines. At the time of implantation the mouse blastocyst is made up of about 70 trophoblast cells and 30 ICM cells.

The term "blastula" as used herein refers to an early stage in the development of a non-human embryo consisting of a hollow sphere of cells enclosing a fluid-filled cavity called the blastocoel. The term blastula sometimes is used interchangeably with blastocyst.

The term "blastomere" is used throughout to refer to at least one blastomere (e.g., 1, 2, 3, 4, etc.) obtained from a non-human preimplantation embryo. The term "cluster of two or more blastomeres" is used interchangeably with "blastomere-derived outgrowths" to refer to the cells generated during the *in vitro* culture of a blastomere. For example, after a blastomere is obtained from a SCNT embryo and initially cultured, it generally divides at least once to produce a cluster of two or more blastomeres (also known as a blastomere-derived outgrowth). The cluster can be further cultured with embryonic or fetal cells. Ultimately, the blastomere-derived outgrowths will continue to divide. From these structures, ES cells, totipotent stem (TS) cells, and partially differentiated cell types will develop over the course of the culture method.

The term "cloned (or cloning)" as used herein refers to a gene manipulation technique for preparing a new individual unit to have a gene set identical to another individual unit. In the present invention, the term "cloned" as used herein refers to a non-human cell, embryonic cell, fetal cell, and/or animal cell has a nuclear DNA sequence that is substantially similar or identical to the nuclear DNA sequence of another non-human cell, embryonic cell, fetal cell, differentiated cell, and/or animal cell. The terms "substantially similar" and "identical" are described herein. The cloned SCNT embryo can arise from one nuclear transfer, or alternatively, the cloned SCNT embryo can arise from a cloning process that includes at least one re-cloning step.

The term "transgenic organism" as used herein refers to a non-human organism into which genetic material from another organism has been experimentally transferred, so that the host acquires the genetic traits of the transferred genes in its chromosomal composition.

The term "implanting" as used herein in reference to SCNT embryos as disclosed herein refers to impregnating a surrogate non-human female animal with a SCNT embryo described herein. This technique is well known to a person of ordinary skill in the art. See, e.g., Seidel and Elsden, 1997, Embryo Transfer in Dairy Cattle, W. D. Hoard & Sons, Co., Hoards Dairyman. The embryo may be allowed to develop in utero, or alternatively, the fetus may be removed from the uterine environment before parturition.

By "subject" is meant a non-human mammal, including, but not limited toan agriculturally significant mammal (e.g., bovine, equine, ovine, porcine), a pet (e.g., canine, feline), or a rare or endangered mammal (e.g., panda).

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

Unless specifically stated or obvious from context, as used herein, the term "or" is understood to be inclusive. Unless specifically stated or obvious from context, as used herein, the terms "a", "an", and "the" are understood to be singular or plural (*i.e.,* at least one). By way of example, "an element" means one element or more than one element.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

### BRIEF DESCRIPTION OF THE DRAWINGS

FIGs. 1A-1F show that the combined use of *Xist* KO donor cells and *Kdm4d* mRNA injection does not completely restore developmental potential of SCNT embryos.
FIG. 1A comprises representative images of IVF and SCNT blastocysts stained with anti-H3K27me3, anti-Cdx2, anti-Oct4 antibodies and DAPI. Arrows indicate punctate H3K27me3 signals representing ectopically inactivated X chromosomes. Note that the ectopic XCIs can be observed regardless of *Kdm4d* mRNA injection. Scale bar, 50 µm.
FIG. 1B provides bar graphs showing the ratio of cells with or without punctate H3K27me3 signals (represent inactivated X chromosomes) in IVF and SCNT blastocysts. Each column represents a single blastocyst.
FIG. 1C provides bar graphs showing the pup rate of SCNT embryos examined by caesarian section on E19.5. Note that a combination of using *Xist* KO donor cells with *Kdm4d* mRNA injection additively improves term rate of SCNT embryos with cumulus cells, Sertoli cells and MEF cells as donors.
FIG. 1D shows an image of an adult male mouse derived by SCNT using *Xist* KO Sertoli cell combined with *Kdm4d* mRNA injection, and its pups generated through natural mating with a wild-type female.
FIG. 1E provides box plots showing weight of placenta examined by caesarian section on E19.5. The whiskers represent the maximum and minimum. ***p < 0.001. ns, not significant.
FIG. 1F provides representative images of histological sections of term placenta stained with Periodic acid-Schiff (PAS: right). Note that the PAS-positive spongiotrophoblast layer has invaded into labyrinthine layer in SCNT placenta regardless of the genotype of *Xist* allele in donor cells. Scale bar, 1 mm.
FIGs. 2A-2C show the postimplantation developmental arrest of SCNT embryos.

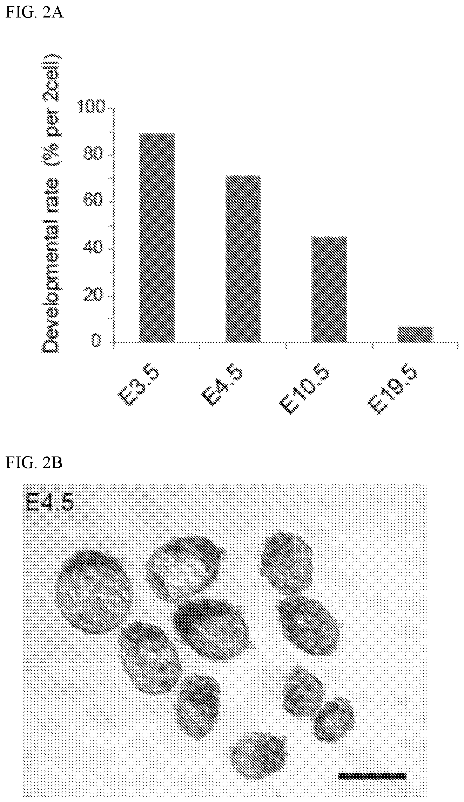

FIG. 2A provides bar graphs showing developmental rate of SCNT embryos generated using Xist KO MEF cells combined with Kdm4d mRNA injection at the indicated time points.
FIG. 2B is an image of SCNT embryos collected at E4.5.

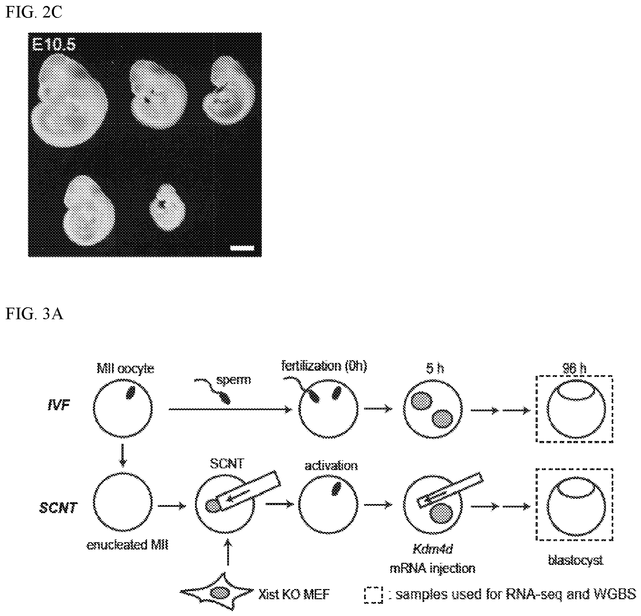

FIG. 2C is an image of SCNT embryos collected at E10.5. Note that SCNT embryos exhibit big variation in embryo/body size at each stage. Scale bars, 100 µm in (FIG. 2B) and 1 mm in (FIG. 2C).
FIGs. 3A-D show extensive reprogramming of DNA methylation in SCNT blastocysts.
FIG. 3A isa schematic illustration of the experimental approach. Blastocysts generated by IVF or SCNT (combination of *Xist* KO donor and *Kdm4d* injection) were used for whole-genome bisulfite sequencing (WGBS) and RNA-seq.

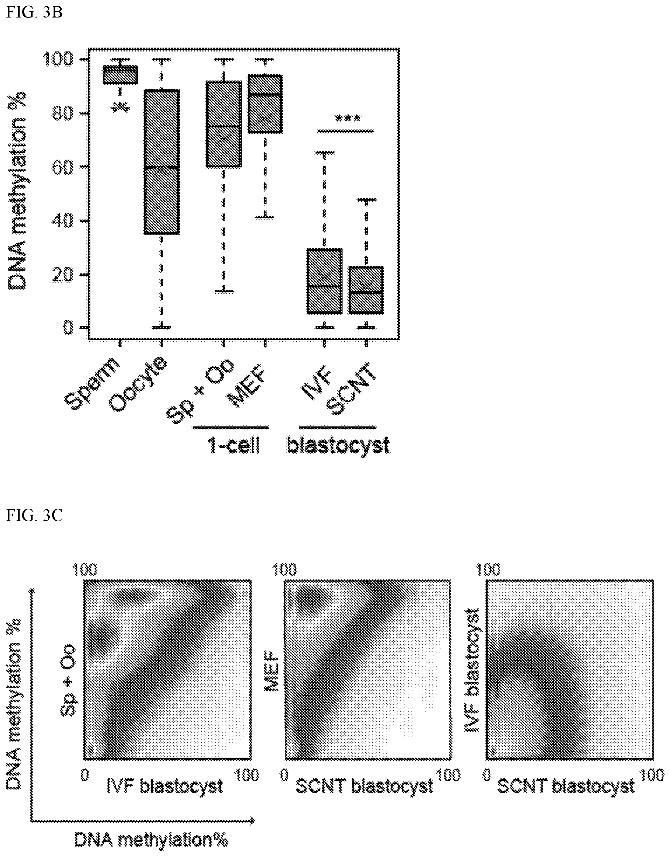

FIG. 3B comprises box plots comparing the DNA methylation levels of all covered CpGs across the genome of SCNT and IVF blastocysts, as well as MEFs, zygotes, sperm and oocytes. Thick lines in boxes indicate the medians, and crosses stand for the mean. The whiskers represent the 2.5th and 97.5th percentiles. Sp+Oo represents the average value of sperm and oocyte. WGBS datasets of MEF, sperm and oocyte were obtained from GSE56151 and GSE56697.
FIG. 3C is a plot comparing the DNA methylation levels between each sample. Note that heavily methylated donor MEF cell genome is globally reprogrammed by SCNT resulting in a similar DNA methylation profile as that of IVF blastocyst.

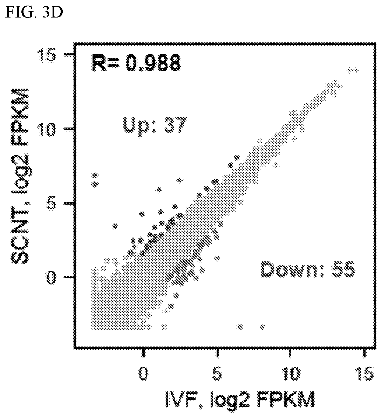

FIG. 3D is a scatter plot comparing gene expression profiles of IVF and SCNT blastocysts. Up-regulated genes (n=37 (dark colored cluster); fold change (FC) > 3.0) and down-regulated genes (n=55, lighter colored cluster; FC > 3.0) in SCNT embryos.

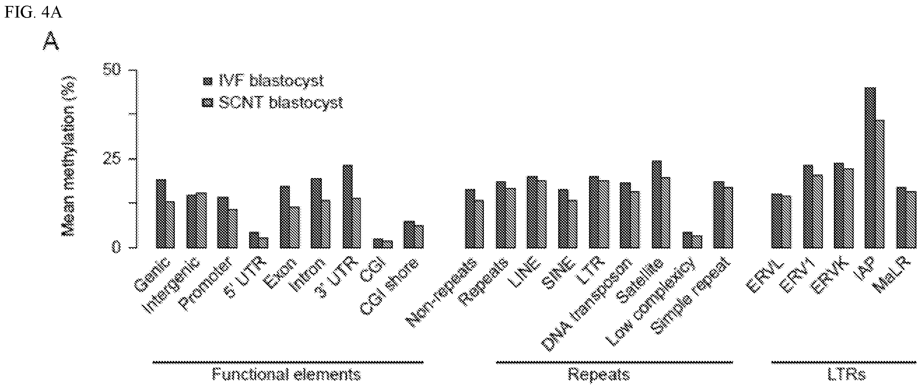

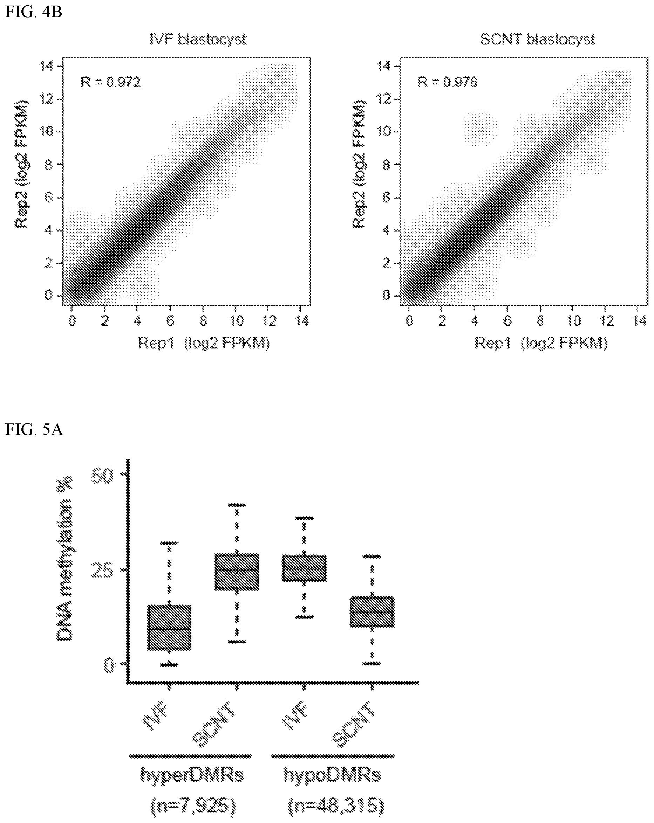

FIGs. 4A and 4B show that SCNT and IVF blastocysts have similar DNA methylome and transcriptome.
FIG. 4A provides a bar graph comparing mean methylation levels at various genomic features including repeats in IVF and SCNT blastocysts.
FIG. 4B comprises scatter plots comparing transcriptomes of biological replicates of IVF and SCNT blastocysts.
FIGs. 5A-5H shows the identification and characterization of differentially methylated regions (DMRs) in SCNT blastocysts.
FIG. 5A shows box plots showing the DNA methylation levels of SCNT and IVF blastocysts at hyper- and hypo-DMRs. Thick lines in boxes indicate the medians, and crosses represent the mean. The number of DMRs are also indicated.

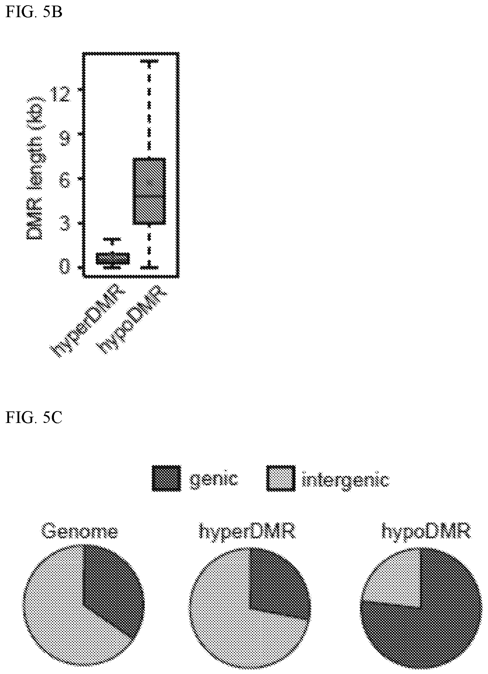

FIG. 5B comprises box plots comparing the lengths of hyper- and hypo-DMRs.
FIG. 5C is a pie chart distribution of hyper- and hypo-DMRs in the genome.

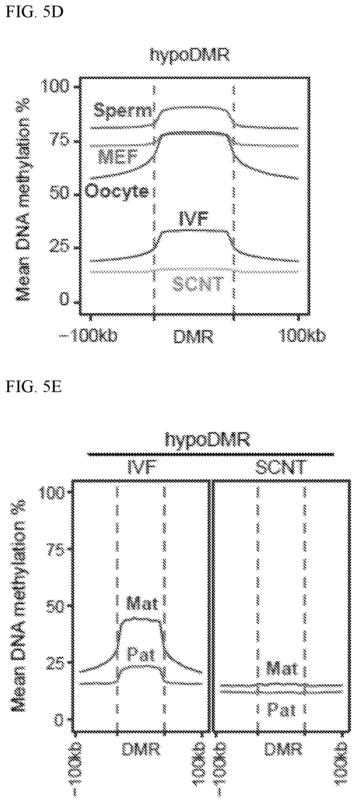

FIG. 5D is a graph showing average DNA methylation levels of the indicated samples at hypoDMRs compared with their flanking regions.
FIG. 5E is a graph showing Paternal (Pat) and maternal (Mat) allele-specific DNA methylation levels of IVF and SCNT blastocysts at hypoDMRs compared with their flanking regions.

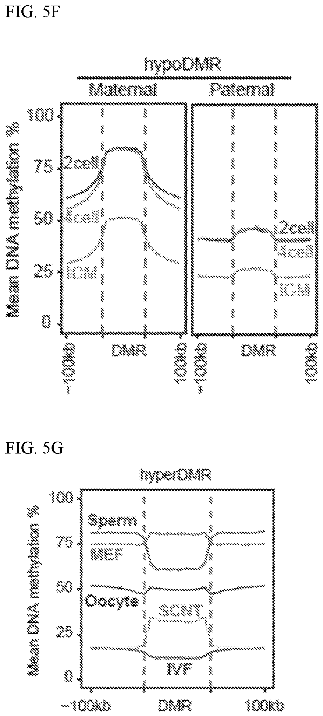

FIG. 5F is a graph showing paternal and maternal allele-specific DNA methylation levels of IVF and SCNT embryos at the indicated developmental stages at hypoDMRs compared with their flanking regions.
FIG. 5G is a graph showing average DNA methylation levels of the indicated samples at hyperDMRs compared with their flanking regions.

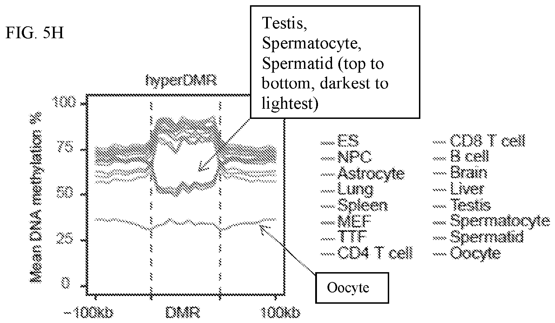

FIG. 5H is a graph showing average DNA methylation levels of the indicated samples at hyperDMRs compared with their flanking regions. Datasets used were from GSE11034.

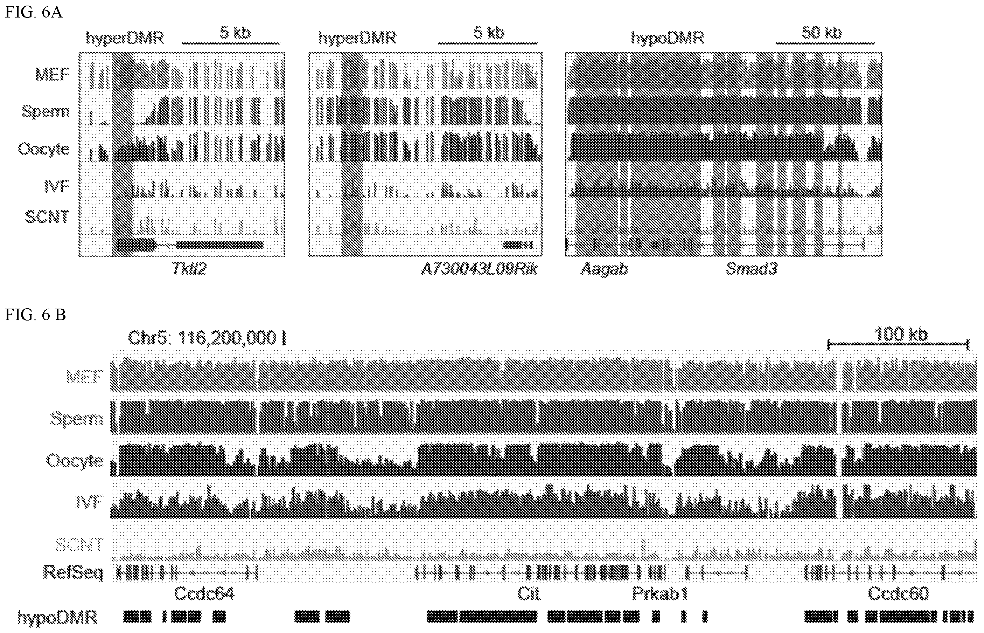

FIGs. 6A-6D provides features of hypo- and hyper-DMRs in SCNT blastocysts.
FIG. 6A is a representative genome browser view of hyper- and hypo-DMRs.
FIG. 6B is a representative genome browser view showing methylation peaks in oocytes overlap with those in IVF blastocysts.

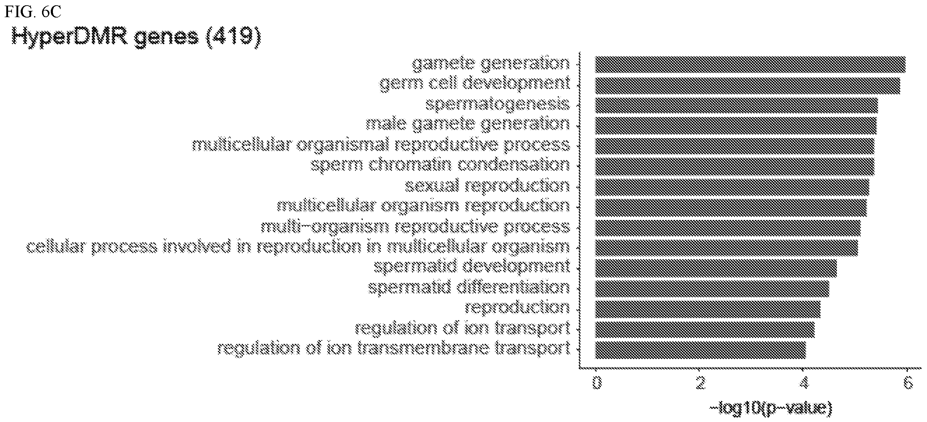

FIG. 6C is a gene ontology analysis of the hyperDMR-accociated genes.

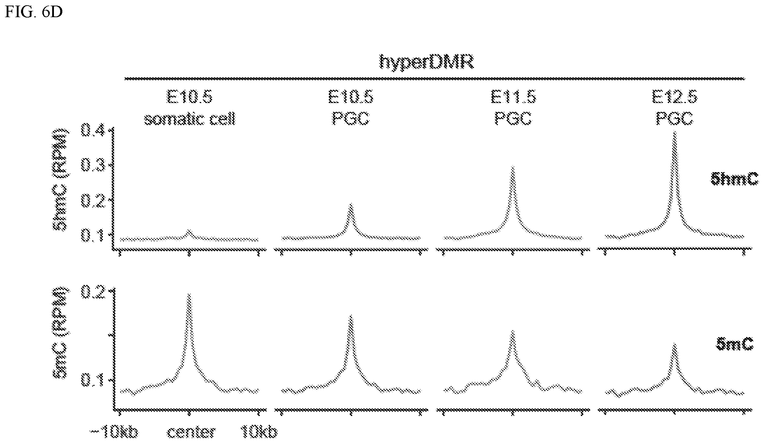

FIG. 6D comprises peak plots showing mean methylation (5mC) and hydroxymethylation (5hmC) levels at hyperDMRs during PGC development.

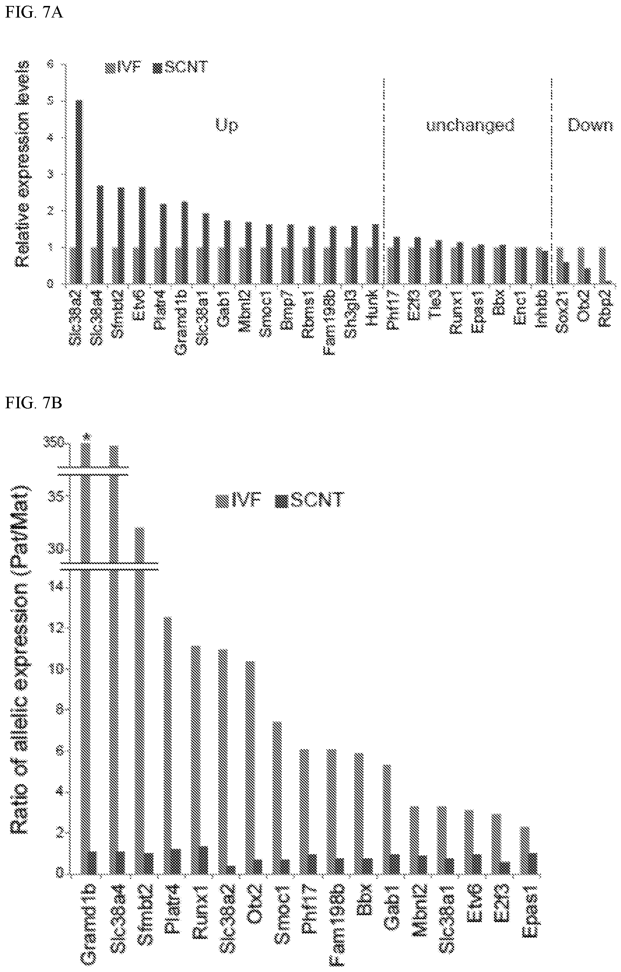

FIGs. 7A-D show loss of H3K27me3-dependent imprinting in SCNT blastocyst.
FIG. 7A provides bar graphs showing relative gene expression levels of H3K27me3-imprinted genes in SCNT blastocysts. Shown are the 26 genes expressed in IVF blastocyst at a reliably detectable level (fragments per kilobase of exon per million mapped fragments (FPKM) > 1). The expression level of IVF blastocysts was set as 1. Genes were classified to up, down and unchanged by expression changes in SCNT compared to that in IVF blastocysts (FC >1.5).
FIG. 7B provides bar graphs showing the ratio (Pat/Mat) of allelic expression of the H3K27me3-imprinted genes in IVF and SCNT blastocysts. Among the 26 expressed genes (FPKM >1), 17 genes with >10 SNP reads in either sample are shown. Asterisk represents 100% biased to paternal allele. Note that all 17 genes lost their paternal allelic bias in SCNT blastocysts.

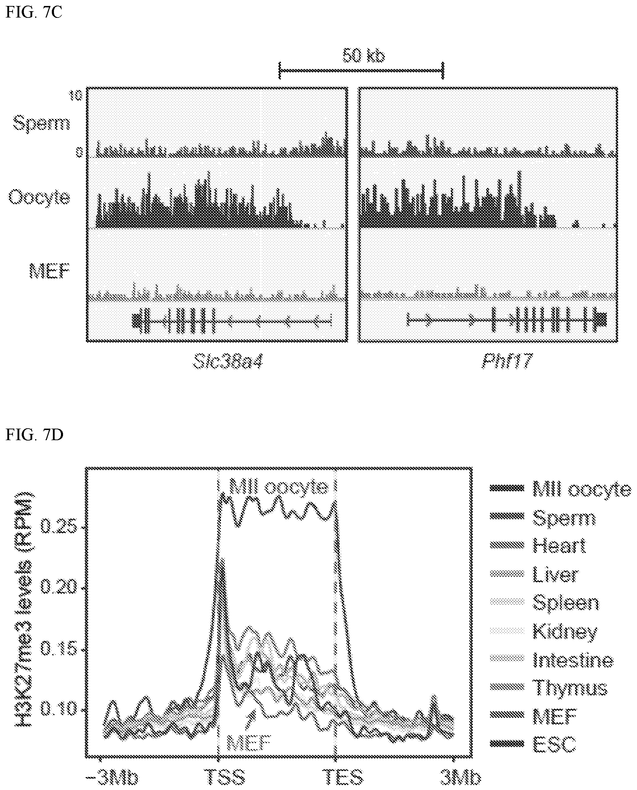

FIG. 7C shows genome browser views of H3K27me3 ChIP-seq signals at two representative H3K27me3-imprinted genes.
FIG. 7D shows the average H3K27me3 ChIP-seq intensity of various cell types (oocytes, sperm, MEFs, ESCs) and tissues at the 76 H3K27me3-imprinted genes compared with 3 Mb flanking regions.

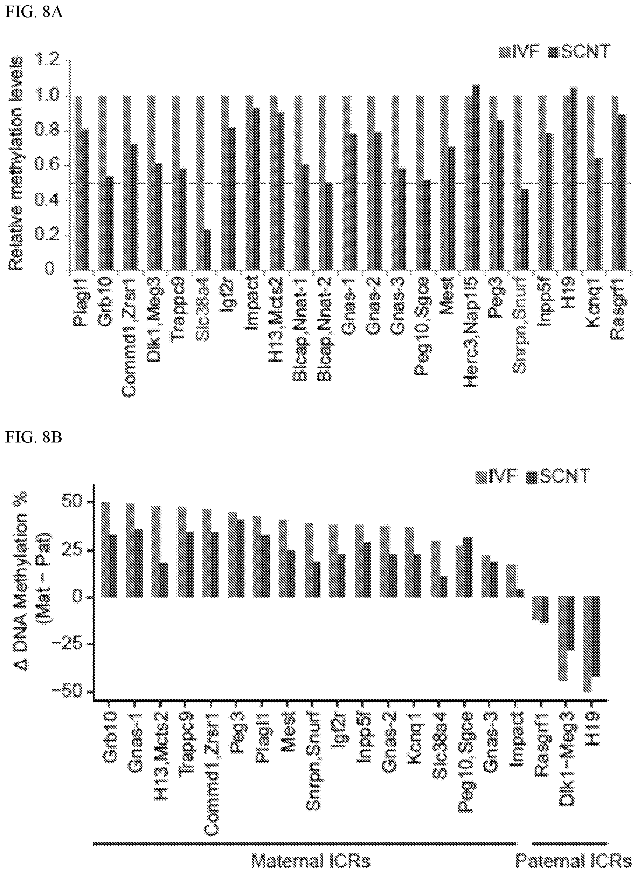

FIGs. 8A-8F illustrates the imprinting status of the known 126 imprinted genes and their known ICRs.
FIG. 8A provides bar graphs showing relative DNA methylation levels of the 23 known imprinting control regions (ICRs) in SCNT blastocysts. The methylation level of IVF blastocysts was set as 1. Dashed line indicates 50% of the IVF blastocysts methylation level. Note that 21 out of 23 ICRs maintained at least 50% that of the IVF methylation levels in SCNT blastocysts, but *Slc38a4* and *Snrpn* ICRs (marked as red) showed less than 50% that of the IVF level.
FIG. 8B provides bar graph showing allelic bias of DNA methylation at 20 ICRs with sufficient allele-specific methylation information (> 5 detected CpG in both alleles of both IVF and SCNT blastocysts). Note that all 20 ICRs maintained allelic biased DNA methylation in SCNT blastocysts.

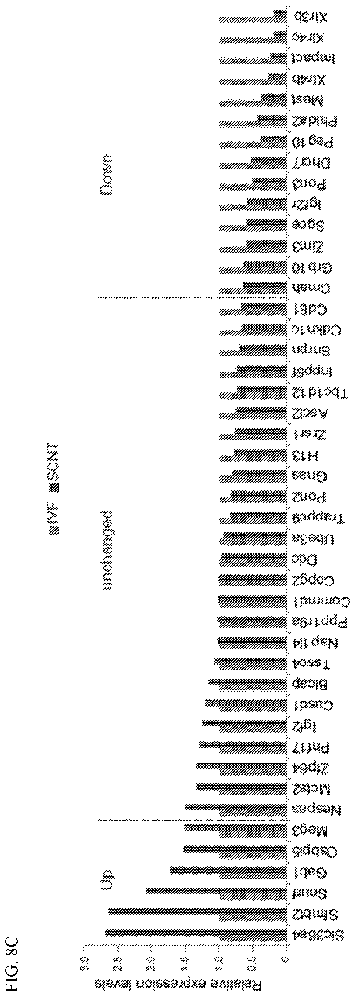

FIG. 8C provides bar graphs showing relative gene expression levels of known imprinted genes in SCNT blastocysts. Shown are 45 imprinted genes reliably detectable in IVF blastocysts (FPKM > 1). The expression level of IVF blastocysts was set as 1. Genes were classified as up, down, and unchanged based on their expression levels in SCNT embryos compared to IVF embryos (FC >1.5).

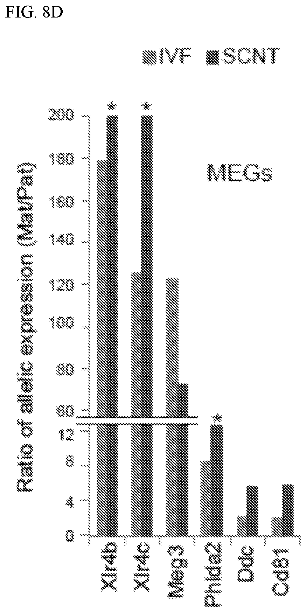

FIG. 8D provides bar graphs showing the ratio of allelic expression (Mat/Pat) of known imprinted genes in IVF and SCNT blastocysts. Shown are 6 maternally expressed genes (MEGs; Mat/Pat > 2.0) that are expressed at a reliably detectable level with sufficient SNP tracked reads (FPKM>1, mean SNP reads > 10 in either sample) in IVF blastocysts. Asterisk represents 100% bias to maternal allele. Note that all 6 MEGs maintained maternal allelic bias in SCNT blastocysts.

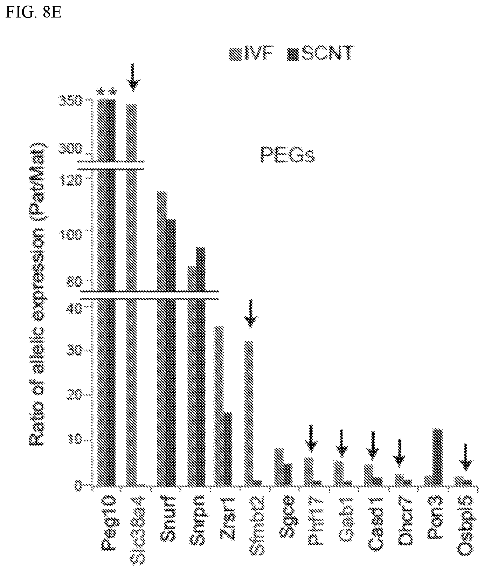

FIG. 8E provides bar graphs showing the ratio of allelic expression (Pat/Mat) of known imprinted genes in IVF and SCNT blastocysts. Shown are 13 paternally expressed genes (PEGs; Pat/Mat > 2.0) that are expressed at a reliably detectable level with sufficient SNP tracked reads (FPKM>1, mean SNP reads > 10 in either sample) in IVF blastocysts. Asterisk represents 100% bias to paternal allele. Arrows indicate genes that lost paternal biased expression in SCNT blastocysts. Slc38a4, Sfmbt2, Phf17, and Gab1 are H3K27me3-dependent imprinted genes.

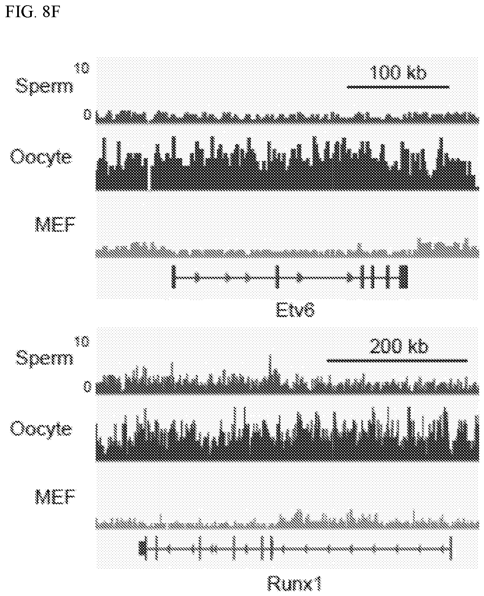

FIG. 8F presents representative genome browser views of H3K27me3 ChIP-seq signals at non-canonical imprinted genes.

### DETAILED DESCRIPTION

The invention provides methods for improving non-human cloning efficiency. In particular embodiments, the invention provides methods for improving non-human somatic cell nuclear transfer efficiency that involve *Kdm4d* overexpression is an *Xist* knockout donor cell.

The invention is based, at least in part, on the discovery that *Xist* knockout donor cells coupled with *Kdm4d* mRNA injection can improve somatic cell nuclear transfer efficiency. This combined approach resulted in the highest efficiency ever reported in mouse cloning using differentiated somatic donor cells. However, many of the SCNT embryos still exhibit postimplantation developmental arrest and the surviving embryos have abnormally large placenta, suggesting some reprogramming defects still persist. Comparative methylome and transcriptome analysis revealed abnormal DNA methylation and loss of H3K27me3-dependent imprinting in SCNT blastocyst embryos, which are likely the cause of the observed developmental defects.

### H3K27me3 is a DNA methylation-independent imprinting mechanism

Mammalian sperm and oocytes have different epigenetic landscapes and are organized in different fashion. Following fertilization, the initially distinct parental epigenomes become largely equalized with the exception of certain loci including imprinting control regions (ICRs). How parental chromatin becomes equalized and how ICRs escape from this reprogramming is largely unknown. Here parental allele-specific DNase I hypersensitive sites (DHSs) was characterized in mouse zygotes and morula embryos, and the epigenetic mechanisms underlying allelic DHSs was investigated. Integrated analyses of DNA methylome and H3K27me3 ChIP-seq data sets revealed 76 genes (Table 1) with paternal allele-specific DHSs that were devoid of DNA methylation, but harbored maternal allele-specific H3K27me3.

**Table 1: H3K27me3-dependent imprinted genes**

| gene name | gene chr | gene start | gene end |
|---|---|---|---|
| Rbp2 | chr9 | 98390956 | 98410190 |
| Runx1 | chr16 | 92601711 | 92826311 |
| Sfmbt2 | chr2 | 10292078 | 10516880 |
| Slc38a2 | chr15 | 96517823 | 96530129 |
| Slc38a4 | chr15 | 96825254 | 96886387 |
| Gramd1b | chr9 | 40105492 | 40263349 |
| Bbx | chr16 | 50191957 | 50432502 |
| Sox21 | chr14 | 118632456 | 118636252 |
| Mbnl2 | chr14 | 120674891 | 120830920 |
| Prdm11 | chr2 | 92815063 | 92886301 |
| 1700067G17Rik | chr1 | 90912688 | 90918785 |
| 1700095B10Rik | chr5 | 113222312 | 113230721 |
| Mir692-2b | chr4 | 125181992 | 125182101 |
| Sh3gl3 | chr7 | 89319728 | 89455927 |
| Etv6 | chr6 | 133985725 | 134220165 |
| Tle3 | chr9 | 61220173 | 61266304 |
| Hunk | chr16 | 90386642 | 90499798 |
| Gab1 | chr8 | 83288333 | 83404378 |
| Matn1 | chr4 | 130500300 | 130511391 |
| Chst1 | chr2 | 92439864 | 92455409 |
| Clic6 | chr16 | 92498392 | 92541486 |
| 1700110K17Rik | chr9 | 40141057 | 40150922 |
| Foxl1 | chr8 | 123651585 | 123654544 |
| Mir6241 | chr14 | 118657855 | 118657958 |
| Otog | chr7 | 53496357 | 53566804 |
| 1700017J07Rik | chr2 | 168803769 | 168804406 |
| 4930404H11Rik | chr12 | 72641594 | 72657120 |
| Gm5086 | chr13 | 98329955 | 98353949 |
| Tshz2 | chr2 | 169459146 | 169714004 |
| Bmp7 | chr2 | 172695189 | 172765794 |
| G730013B05Rik | chr16 | 50526358 | 50559572 |
| Rftn1 | chr17 | 50132632 | 50329822 |
| C430002E04Rik | chr3 | 41291603 | 41297121 |
| Myoz2 | chr3 | 122709124 | 122737905 |
| Six3os1 | chr17 | 86001272 | 86017736 |
| Slc38a1 | chr15 | 96401849 | 96473344 |
| Rbms1 | chr2 | 60590010 | 60801261 |
| Flt1 | chr5 | 148373772 | 148537564 |
| Sall3 | chr18 | 81163113 | 81183317 |
| Otx2os1 | chr14 | 49288963 | 49413023 |
| 1700006F04Rik | chr14 | 120148449 | 120150786 |
| 2300005B03Rik | chr15 | 74573269 | 74577117 |
| 4931430N09Rik | chr6 | 118830176 | 118835561 |
| Gas7 | chr11 | 67346500 | 67502494 |
| Phf17 | chr3 | 41359656 | 41420786 |
| Igsf21 | chr4 | 139582767 | 139802726 |
| Otx2 | chr14 | 49277859 | 49282547 |
| Klhdc7a | chr4 | 139518088 | 139523941 |
| 1700125H03Rik | chr8 | 70892358 | 70899609 |
| Lpar3 | chr3 | 145883925 | 145949178 |
| Mir6239 | chr14 | 118352964 | 118353069 |
| Epas1 | chr17 | 87153204 | 87232750 |
| Slc6a1 | chr6 | 114232629 | 114267519 |
| Cdh26 | chr2 | 178165312 | 178222071 |
| 1700025C18Rik | chr2 | 164904193 | 164916250 |
| Prox1 | chr1 | 191945658 | 191994559 |
| 1700121N20Rik | chr12 | 107680862 | 107685876 |
| Adamts2 | chr11 | 50415587 | 50617551 |
| Gadl1 | chr9 | 115818573 | 115985294 |
| Dnase2b | chr3 | 146244337 | 146278562 |
| Inhbb | chr1 | 121312042 | 121318825 |
| E2f3 | chr13 | 29998444 | 30077932 |
| Ajap1 | chr4 | 152747330 | 152856939 |
| BC049762 | chr11 | 51067153 | 51076453 |
| Edn3 | chr2 | 174586274 | 174609543 |
| Enc1 | chr13 | 98011060 | 98022995 |
| 4930465M20Rik | chr12 | 108961953 | 108973698 |
| 9630028H03Rik | chr2 | 135406266 | 135408956 |
| Cd44 | chr2 | 102651300 | 102741822 |
| Epgn | chr5 | 91456543 | 91464238 |
| Syt13 | chr2 | 92755258 | 92796208 |
| Myb | chr10 | 20844736 | 20880790 |
| Lrig3 | chr10 | 125403275 | 125452415 |
| Fam198b | chr3 | 79689852 | 79750200 |
| Smoc1 | chr12 | 82127795 | 82287401 |
| 1700084F23Rik | chr13 | 70142928 | 70167226 |

Interestingly, these genes are paternally expressed in preimplantation embryos, and ectopic removal of H3K27me3 induced maternal allele expression. H3K27me3-dependent imprinting was largely lost in the embryonic cell lineage, but at least 5 genes maintained their imprinting in the extra-embryonic cell lineage. The 5 genes include all previously identified DNA methylation-independent imprinted autosomal genes. Maternal H3K27me3 is a DNA methylation-independent imprinting mechanism. In one embodiment, the methods of the invention involve the use of an H3K27me3 selective methylase.

### H3K27me3 is important for X chromosome inactivation

In females of certain therian mammals including rodents, one of the two X chromosomes is inactivated to achieve gene dosage compensation. This phenomenon, called X chromosome inactivation (XCI), provides an excellent model for understanding mechanisms of epigenetic silencing. During development, XCI can take place in either imprinted or random manners. For imprinted XCI, the paternal X chromosome (Xp) is selectively inactivated during preimplantation development. Although imprinted XCI is maintained in the extra-embryonic cell lineage, it is lost in the inner cell mass (ICM) of late blastocysts. At peri-implantation stage, epiblast cells undergo random XCI resulting in the silencing of either Xp or maternal X chromosome (Xm). Previous studies have demonstrated a critical role *of Xist,* an X-linked long non-coding RNA, in both imprinted and random XCI. The *Xist* RNA participates in XCI by coating and inactivating X chromosome *in cis.*

Genomic imprinting allows parent-of-origin specific gene regulation. To selectively silence the Xp during imprinted XCI, the *Xist* gene is imprinted for silencing in the Xm with a long sought-after, but yet-to-be-identified, mechanism. Previous studies using nuclear transfer approaches have suggested that genomic imprinting of *Xist* is established during oogenesis, like that of autosomal imprinted genes. In mouse preimplantation embryos and extra-embryonic cells, only the paternal X chromosome (Xp) is inactivated. Central to the imprinted paternal X chromosome inactivation (XCI) is a long non-coding RNA, *Xist,* which is expressed from Xp and acts *in cis* to coat and silence the entire Xp. To achieve Xp-specific inactivation, the maternal *Xist* gene must be silenced, yet the silencing mechanism is not yet clear. As described but not specifically claimed herein, the *Xist* locus is coated with a broad H3K27me3 domain in mouse oocytes, which persists through preimplantation development. Ectopic removal of H3K27me3 induces maternal *Xist* expression and maternal XCI. Thus, maternal H3K27me3 serves as the imprinting mark *of Xist.*

In some embodiments as described but not specifically claimed herein, the methods of the invention involve administering a pharmaceutical composition comprising a selective H3K27me3 demethylase inhibitor.

### H3K9me3 and SCNT

Histone H3 lysine 9 trimethylation (H3K9me3) in donor somatic cells is an epigenetic barrier for SCNT reprogramming. H3K9me3 in donor cells prevents transcriptional activation of the associated regions at zygotic genome activation and leads to developmental arrest of SCNT embryos at preimplantation stages in both mouse and human. Importantly, removal of the H3K9me3 barrier by overexpressing a H3K9me3-specific demethylase, *Kdm4d,* allows SCNT embryos to develop to the blastocyst stage at a rate similar to that of IVF. Consequently, the overall cloning efficiency for term rate is increased 8-9 fold. Although the use of *Kdm4d* in SCNT results in an implantation rate comparable to that of IVF, less than 15% of the implanted SCNT embryos develop to term. Moreover, abnormally large placentas are still observed in *Kdm4d-*injected SCNT embryos. These results, as described but not specifically claimed herein, suggest that the H3K9me3 reprogramming barrier mainly impedes preimplantation development and other barriers affect postimplantation development.

*Xist* is important for postimplantation development of mouse SCNT embryos. Abnormal expression *of Xist* from maternal X chromosome leads to ectopic X chromosome inactivation (XCI) and global transcriptional alteration in preimplantation embryos, resulting in postimplantation developmental failure of SCNT embryos. Importantly, this developmental failure caused by ectopic *Xist* expression can be overcome by using *Xist* knockout (KO) somatic cells as donor cells or by injecting small interfering RNA against *Xist* into 1-cell male SCNT embryos leading to an 8-10 fold increase of term rate.

### Inhibitory Nucleic Acids

Inhibitory nucleic acid molecules are those oligonucleotides that inhibit the expression or activity of a polypeptide or polynucleotide (e.g., an Xist polynucleotide). Such oligonucleotides include single and double stranded nucleic acid molecules (e.g., DNA, RNA, and analogs thereof) that bind a nucleic acid molecule that encodes an Xist polynucleotide (e.g., antisense molecules, siRNA, shRNA).

### siRNA

Short twenty-one to twenty-five nucleotide double-stranded RNAs are effective at down-regulating gene expression (Zamore et al., Cell 101: 25-33; Elbashir et al., Nature 411: 494-498, 2001). The effectiveness of an siRNA approach in mammals was demonstrated *in vivo* by McCaffrey et al. (Nature 418: 38-39.2002).

Given the sequence of a target gene, siRNAs may be designed to inactivate that gene. Such siRNAs, for example, could be administered directly to an affected tissue, or administered systemically. The nucleic acid sequence of a gene can be used to design small interfering RNAs (siRNAs). The 21 to 25 nucleotide siRNAs may be used, for example, to reduce Xist expression.

The inhibitory nucleic acid molecules of the present invention may be employed as double-stranded RNAs for RNA interference (RNAi)-mediated knock-down of expression. In one embodiment, expression of an Xist gene is reduced in a somatic cell. RNAi is a method for decreasing the cellular expression of specific proteins of interest (reviewed in Tuschl, Chembiochem 2:239-245, 2001; Sharp, Genes & Devel. 15:485-490, 2000; Hutvagner and Zamore, Curr. Opin. Genet. Devel. 12:225-232, 2002; and Hannon, Nature 418:244-251, 2002). The introduction of siRNAs into cells either by transfection of dsRNAs or through expression of siRNAs using a plasmid-based expression system is increasingly being used to create loss-of-function phenotypes in mammalian cells.

In one embodiment of the invention, a double-stranded RNA (dsRNA) molecule is made that includes between eight and nineteen consecutive nucleobases of a nucleobase oligomer of the invention. The dsRNA can be two distinct strands of RNA that have duplexed, or a single RNA strand that has self-duplexed (small hairpin (sh)RNA). Typically, dsRNAs are about 21 or 22 base pairs, but may be shorter or longer (up to about 29 nucleobases) if desired. dsRNA can be made using standard techniques (e.g., chemical synthesis or *in vitro* transcription). Kits are available, for example, from Ambion (Austin, Tex.) and Epicentre (Madison, Wis.). Methods for expressing dsRNA in mammalian cells are described in Brummelkamp et al. Science 296:550-553, 2002; Paddison et al. Genes & Devel. 16:948-958, 2002. Paul et al. Nature Biotechnol. 20:505-508, 2002; Sui et al. Proc. Natl. Acad. Sci. USA 99:5515-5520, 2002; Yu et al. Proc. Natl. Acad. Sci. USA 99:6047-6052, 2002; Miyagishi et al. Nature Biotechnol. 20:497-500, 2002; and Lee et al. Nature Biotechnol. 20:500-505 2002.

Small hairpin RNAs (shRNAs) comprise an RNA sequence having a stem-loop structure. A "stem-loop structure" refers to a nucleic acid having a secondary structure that includes a region of nucleotides which are known or predicted to form a double strand or duplex (stem portion) that is linked on one side by a region of predominantly single-stranded nucleotides (loop portion). The term "hairpin" is also used herein to refer to stem-loop structures. Such structures are well known in the art and the term is used consistently with its known meaning in the art. As is known in the art, the secondary structure does not require exact base-pairing. Thus, the stem can include one or more base mismatches or bulges. Alternatively, the base-pairing can be exact, i.e. not include any mismatches. The multiple stem-loop structures can be linked to one another through a linker, such as, for example, a nucleic acid linker, a miRNA flanking sequence, other molecule, or some combination thereof.

As used herein, the term "small hairpin RNA" includes a conventional stem-loop shRNA, which forms a precursor miRNA (pre-miRNA). While there may be some variation in range, a conventional stem-loop shRNA can comprise a stem ranging from 19 to 29 bp, and a loop ranging from 4 to 30 bp. "shRNA" also includes micro-RNA embedded shRNAs (miRNA-based shRNAs), wherein the guide strand and the passenger strand of the miRNA duplex are incorporated into an existing (or natural) miRNA or into a modified or synthetic (designed) miRNA. In some instances the precursor miRNA molecule can include more than one stem-loop structure. MicroRNAs are endogenously encoded RNA molecules that are about 22-nucleotides long and generally expressed in a highly tissue- or developmental-stage-specific fashion and that post-transcriptionally regulate target genes. More than 200 distinct miRNAs have been identified in plants and animals. These small regulatory RNAs are believed to serve important biological functions by two prevailing modes of action: (1) by repressing the translation of target mRNAs, and (2) through RNA interference (RNAi), that is, cleavage and degradation of mRNAs. In the latter case, miRNAs function analogously to small interfering RNAs (siRNAs). Thus, one can design and express artificial miRNAs based on the features of existing miRNA genes.

shRNAs can be expressed from DNA vectors to provide sustained silencing and high yield delivery into almost any cell type. In some embodiments as described but not specifically claimed herein, the vector is a viral vector. Exemplary viral vectors include retroviral, including lentiviral, adenoviral, baculoviral and avian viral vectors, and including such vectors allowing for stable, single-copy genomic integrations. Retroviruses from which the retroviral plasmid vectors can be derived include, but are not limited to, Moloney murine leukemia virus, spleen necrosis virus, Rous sarcoma virus, Harvey sarcoma virus, avian leukosis virus, gibbon ape leukemia virus, human immunodeficiency virus, myeloproliferative sarcoma virus, and mammary tumor virus. A retroviral plasmid vector can be employed to transduce packaging cell lines to form producer cell lines. Examples of packaging cells which can be transfected include, but are not limited to, the PE50l, PA3l7, R-2, R-AM, PA12, T19-14x, VT-19-17-H2, RCRE, RCRIP, GP+E-86, GP+envAm12, and DAN cell lines as described in Miller, Human Gene Therapy 1:5-14 (1990). The vector can transduce the packaging cells through any means known in the art. A producer cell line generates infectious retroviral vector particles which include polynucleotide encoding a DNA replication protein. Such retroviral vector particles then can be employed, to transduce eukaryotic cells, either in vitro or in vivo. The transduced eukaryotic cells will express a DNA replication protein.

Catalytic RNA molecules or ribozymes that include an antisense sequence of the present invention can be used to inhibit expression of a nucleic acid molecule *in vivo* (e.g., Xist). The inclusion of ribozyme sequences within antisense RNAs confers RNA-cleaving activity upon them, thereby increasing the activity of the constructs. The design and use of target RNA-specific ribozymes is described in Haseloff et al., Nature 334:585-591. 1988, and U.S. Patent Application Publication No. 2003/0003469 A1.

Accordingly, the invention as described but not specifically claimed herein also features a catalytic RNA molecule that includes, in the binding arm, an antisense RNA having between eight and nineteen consecutive nucleobases. In preferred embodiments of this invention as described but not specifically claimed herein, the catalytic nucleic acid molecule is formed in a hammerhead or hairpin motif. Examples of such hammerhead motifs are described by Rossi et al., Aids Research and Human Retroviruses, 8:183, 1992. Example of hairpin motifs are described by Hampel et al., "RNA Catalyst for Cleaving Specific RNA Sequences," filed Sep. 20, 1989, which is a continuation-in-part of U.S. Ser. No. 07/247,100 filed Sep. 20, 1988; Hampel and Tritz, Biochemistry, 28:4929, 1989; and Hampel et al., Nucleic Acids Research, 18: 299, 1990. These specific motifs are not limiting in the invention and those skilled in the art will recognize that all that is important in an enzymatic nucleic acid molecule of this invention is that it has a specific substrate binding site which is complementary to one or more of the target gene RNA regions and that it have nucleotide sequences within or surrounding that substrate binding site which impart an RNA cleaving activity to the molecule.

Essentially any method for introducing a nucleic acid construct into cells can be employed. Physical methods of introducing nucleic acids include injection of a solution containing the construct, bombardment by particles covered by the construct, soaking a cell, tissue sample or organism in a solution of the nucleic acid, or electroporation of cell membranes in the presence of the construct. A viral construct packaged into a viral particle can be used to accomplish both efficient introduction of an expression construct into the cell and transcription of the encoded shRNA. Other methods known in the art for introducing nucleic acids to cells can be used, such as lipid-mediated carrier transport, chemical mediated transport, such as calcium phosphate, and the like. Thus the shRNA-encoding nucleic acid construct can be introduced along with components that perform one or more of the following activities: enhance RNA uptake by the cell, promote annealing of the duplex strands, stabilize the annealed strands, or otherwise increase inhibition of the target gene.

For expression within cells, DNA vectors, for example plasmid vectors comprising either an RNA polymerase II or RNA polymerase III promoter can be employed. Expression of endogenous miRNAs is controlled by RNA polymerase II (Pol II) promoters and in some cases, shRNAs are most efficiently driven by Pol II promoters, as compared to RNA polymerase III promoters (Dickins et al., 2005, Nat. Genet. 39: 914-921). In some embodiments as described but not specifically claimed herein, expression of the shRNA can be controlled by an inducible promoter or a conditional expression system, including, without limitation, RNA polymerase type II promoters. Examples of useful promoters in the context of the invention are tetracycline-inducible promoters (including TRE-tight), IPTG-inducible promoters, tetracycline transactivator systems, and reverse tetracycline transactivator (rtTA) systems. Constitutive promoters can also be used, as can cell- or tissue-specific promoters. Many promoters will be ubiquitous, such that they are expressed in all cell and tissue types. A certain embodiment as described but not specifically claimed herein uses tetracycline-responsive promoters, one of the most effective conditional gene expression systems in in vitro and in vivo studies. See International Patent Application PCT/US2003/030901 (Publication No. WO 2004-029219 A2) and Fewell et al., 2006, Drug Discovery Today 11: 975-982, for a description of inducible shRNA.

### Delivery of Polynucleotides

Naked polynucleotides, or analogs thereof, are capable of entering mammalian cells and inhibiting expression of a gene of interest. Nonetheless, it may be desirable to utilize a formulation that aids in the delivery of oligonucleotides or other nucleobase oligomers to cells (see, e.g., U.S. Pat. Nos. 5,656,611; 5,753,613; 5,785,992; 6,120,798; 6,221,959; 6,346,613; and 6,353,055).

### Oligonucleotides and other Nucleobase Oligomers

At least two types of oligonucleotides induce the cleavage of RNA by RNase H: polydeoxynucleotides with phosphodiester (PO) or phosphorothioate (PS) linkages. Although 2'-OMe-RNA sequences exhibit a high affinity for RNA targets, these sequences are not substrates for RNase H. A desirable oligonucleotide is one based on 2'-modified oligonucleotides containing oligodeoxynucleotide gaps with some or all internucleotide linkages modified to phosphorothioates for nuclease resistance. The presence of methylphosphonate modifications increases the affinity of the oligonucleotide for its target RNA and thus reduces the IC₅₀. This modification also increases the nuclease resistance of the modified oligonucleotide. It is understood that the methods and reagents of the present invention may be used in conjunction with any technologies that may be developed, including covalently-closed multiple antisense (CMAS) oligonucleotides (Moon et al., Biochem J. 346:295-303, 2000; PCT Publication No. WO 00/61595), ribbon-type antisense (RiAS) oligonucleotides (Moon et al., J. Biol. Chem. 275:4647-4653, 2000; PCT Publication No. WO 00/61595), and large circular antisense oligonucleotides (U.S. Patent Application Publication No. US 2002/0168631 A1).

As is known in the art, a nucleoside is a nucleobase-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn, the respective ends of this linear polymeric structure can be further joined to form a circular structure; open linear structures are generally preferred. Within the oligonucleotide structure, the phosphate groups are commonly referred to as forming the backbone of the oligonucleotide. The normal linkage or backbone of RNA and DNA is a 3' to 5' phosphodiester linkage.

As described but not specifically claimed herein, specific examples of preferred nucleobase oligomers useful in this invention include oligonucleotides containing modified backbones or non-natural internucleoside linkages. As defined in this specification, nucleobase oligomers having modified backbones include those that retain a phosphorus atom in the backbone and those that do not have a phosphorus atom in the backbone. For the purposes of this specification, modified oligonucleotides that do not have a phosphorus atom in their internucleoside backbone are also considered to be nucleobase oligomers.

Nucleobase oligomers that have modified oligonucleotide backbones include, for example, phosphorothioates, chiral phosphorothioates, phosphorodithioates, phosphotriesters, aminoalkyl-phosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity, wherein the adjacent pairs of nucleoside units are linked 3'-5' to 5'-3' or 2'-5' to 5'-2'. Various salts, mixed salts and free acid forms are also included. Representative United States patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

Nucleobase oligomers having modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH.sub.2 component parts. Representative United States patents that teach the preparation of the above oligonucleotides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

In other nucleobase oligomers, both the sugar and the internucleoside linkage, i.e., the backbone, are replaced with novel groups. The nucleobase units are maintained for hybridization with an *Xist* gene listed. One such nucleobase oligomer, is referred to as a Peptide Nucleic Acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Methods for making and using these nucleobase oligomers are described, for example, in "Peptide Nucleic Acids: Protocols and Applications" Ed. P. E. Nielsen, Horizon Press, Norfolk, United Kingdom, 1999. Representative United States patents that teach the preparation of PNAs include, but are not limited to, U.S. Pat. Nos. 5,539,082; 5,714,331; and 5,719,262. Further teaching of PNA compounds can be found in Nielsen et al., Science, 1991, 254, 1497-1500.

In particular embodiments of the invention as described but not specifically claimed herein, the nucleobase oligomers have phosphorothioate backbones and nucleosides with heteroatom backbones, and in particular -CH₂₋NH-O-CH₂-, -CH₂-N(CH₃)-O-CH₂- (known as a methylene (methylimino) or MMI backbone), -CH₂-O-N(CH₃)-CH₂-, -CH₂-N(CH₃)-N(CH₃)-CH₂-, and -O-N(CH₃)-CH₂-CH₂-. In other embodiments as described but not specifically claimed herein, the oligonucleotides have morpholino backbone structures described in U.S. Pat. No. 5,034,506.

Nucleobase oligomers as described but not specifically claimed herein may also contain one or more substituted sugar moieties. Nucleobase oligomers comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl, and alkynyl may be substituted or unsubstituted C₁ to C₁₀ alkyl or C₂ to C₁₀ alkenyl and alkynyl. Particularly preferred are O[(CH₂)ₙO]ₙCH₃, O(CH₂)ₙOCH₃, O(CH₂)ₙNH₂, O(CH₂)ₙCH₃, O(CH₂)ₙONH₂, and O(CH₂)nON[(CH₂)ₙCH₃)]₂, where n and m are from 1 to about 10. Other preferred nucleobase oligomers include one of the following at the 2' position: C₁ to C₁₀ lower alkyl, substituted lower alkyl, alkaryl, aralkyl, O-alkaryl, or O-aralkyl, SH, SCH₃, OCN, Cl, Br, CN, CF₃, OCF₃, SOCH₃, SO₂CH₃, ONO₂, NO₂, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of a nucleobase oligomer, or a group for improving the pharmacodynamic properties of an nucleobase oligomer, and other substituents having similar properties. Preferred modifications are 2'-O-methyl and 2'-methoxyethoxy (2'-O-CH₂CH₂OCH₃, also known as 2'-O-(2-methoxyethyl) or 2'-MOE). Another desirable modification is 2'-dimethylaminooxyethoxy (i.e., O(CH₂) ₂ON(CH₃)₂), also known as 2'-DMAOE. Other modifications include, 2'-aminopropoxy (2'-OCH₂CH₂CH₂NH₂) and 2'-fluoro (2'-F). Similar modifications may also be made at other positions on an oligonucleotide or other nucleobase oligomer, particularly the 3' position of the sugar on the 3' terminal nucleotide or in 2'-5' linked oligonucleotides and the 5' position of 5' terminal nucleotide. Nucleobase oligomers may also have sugar mimetics such as cyclobutyl moieties in place of the pentofuranosyl sugar. Representative United States patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

Nucleobase oligomers may also include nucleobase modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenine and guanine; 2-propyl and other alkyl derivatives of adenine and guanine; 2-thiouracil, 2-thiothymine and 2-thiocytosine; 5-halouracil and cytosine; 5-propynyl uracil and cytosine; 6-azo uracil, cytosine and thymine; 5-uracil (pseudouracil); 4-thiouracil; 8-halo, 8-amino, 8-thiol, 8-thioalkyl, 8-hydroxyl and other 8-substituted adenines and guanines; 5-halo (e.g., 5-bromo), 5-trifluoromethyl and other 5-substituted uracils and cytosines; 7-methylguanine and 7-methyladenine; 8-azaguanine and 8-azaadenine; 7-deazaguanine and 7-deazaadenine; and 3-deazaguanine and 3-deazaadenine. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are particularly useful for increasing the binding affinity of an antisense oligonucleotide of the invention. These include 5-substituted pyrimidines, 6-azapyrimidines, and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y. S., Crooke, S. T. and Lebleu, B., eds., Antisense Research and Applications, CRC Press, Boca Raton, 1993, pp. 276-278) and are desirable base substitutions, even more particularly when combined with 2'-O-methoxyethyl or 2'-O-methyl sugar modifications. Representative United States patents that teach the preparation of certain of the above noted modified nucleobases as well as other modified nucleobases include U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,681,941; and 5,750,692.

As described but not specifically claimed herein, another modification of a nucleobase oligomer of the invention involves chemically linking to the nucleobase oligomer one or more moieties or conjugates that enhance the activity, cellular distribution, or cellular uptake of the oligonucleotide. Such moieties include but are not limited to lipid moieties such as a cholesterol moiety (Letsinger et al., Proc. Natl. Acad. Sci. USA, 86:6553-6556, 1989), cholic acid (Manoharan et al., Bioorg. Med. Chem. Let, 4:1053-1060, 1994), a thioether, e.g., hexyl-S-tritylthiol (Manoharan et al., Ann. N.Y. Acad. Sci., 660:306-309, 1992; Manoharan et al., Bioorg. Med. Chem. Let., 3:2765-2770, 1993), a thiocholesterol (Oberhauser et al., Nucl. Acids Res., 20:533-538: 1992), an aliphatic chain, e.g., dodecandiol or undecyl residues (Saison-Behmoaras et al., EMBO J., 10:1111-1118, 1991; Kabanov et al., FEBS Lett., 259:327-330, 1990; Svinarchuk et al., Biochimie, 75:49-54, 1993), a phospholipid, e.g., di-hexadecyl-rac-glycerol or triethylammonium 1,2-di-O-hexadecyl-rac-glycero-3-H-phosphonate (Manoharan et al., Tetrahedron Lett., 36:3651-3654, 1995; Shea et al., Nucl. Acids Res., 18:3777-3783, 1990), a polyamine or a polyethylene glycol chain (Manoharan et al., Nucleosides & Nucleotides, 14:969-973, 1995), adamantane acetic acid (Manoharan et al., Tetrahedron Lett., 36:3651-3654, 1995), a palmityl moiety (Mishra et al., Biochim. Biophys. Acta, 1264:229-237, 1995), or an octadecylamine or hexylamino-carbonyl-oxycholesterol moiety (Crooke et al., J. Pharmacol. Exp. Ther., 277:923-937, 1996. Representative United States patents that teach the preparation of such nucleobase oligomer conjugates include U.S. Pat. Nos. 4,587,044; 4,605,735; 4,667,025; 4,762,779; 4,789,737; 4,824,941; 4,828,979; 4,835,263; 4,876,335; 4,904,582; 4,948,882; 4,958,013; 5,082,830; 5,109,124; 5,112,963; 5,118,802; 5,138,045; 5,214,136; 5,218,105; 5,245,022; 5,254,469; 5,258,506; 5,262,536; 5,272,250; 5,292,873; 5,317,098; 5,371,241, 5,391,723; 5,414,077; 5,416,203, 5,451,463; 5,486,603; 5,510,475; 5,512,439; 5,512,667; 5,514,785; 5,525,465; 5,541,313; 5,545,730; 5,552,538; 5,565,552; 5,567,810; 5,574,142; 5,578,717; 5,578,718; 5,580,731; 5,585,481; 5,587,371; 5,591,584; 5,595,726; 5,597,696; 5,599,923; 5,599,928; 5,608,046; and 5,688,941.

As described but not specifically claimed herein, the present invention also includes nucleobase oligomers that are chimeric compounds. "Chimeric" nucleobase oligomers are nucleobase oligomers, particularly oligonucleotides, that contain two or more chemically distinct regions, each made up of at least one monomer unit, i.e., a nucleotide in the case of an oligonucleotide. These nucleobase oligomers typically contain at least one region where the nucleobase oligomer is modified to confer, upon the nucleobase oligomer, increased resistance to nuclease degradation, increased cellular uptake, and/or increased binding affinity for the target nucleic acid. An additional region of the nucleobase oligomer may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of nucleobase oligomer inhibition of gene expression. Consequently, comparable results can often be obtained with shorter nucleobase oligomers when chimeric nucleobase oligomers are used, compared to phosphorothioate deoxyoligonucleotides hybridizing to the same target region.

Chimeric nucleobase oligomers of the invention may be formed as composite structures of two or more nucleobase oligomers as described above. Such nucleobase oligomers, when oligonucleotides, have also been referred to in the art as hybrids or gapmers. Representative United States patents that teach the preparation of such hybrid structures include U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922.

As described but not specifically claimed herein, the nucleobase oligomers used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives.

The nucleobase oligomers of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

### Genome Editing to Knockout Xist

Gene editing is a major focus of biomedical research, embracing the interface between basic and clinical science. The development of novel "gene editing" tools provides the ability to manipulate the DNA sequence of a cell at a specific chromosomal locus, without introducing mutations at other sites of the genome. This technology effectively enables the researcher to manipulate the genome of a subject's cells *in vitro* or *in vivo.* In the context of SCNT, non-human cells comprising a KO in Xist are generated using CRISPR.

In one embodiment, gene editing involves targeting an endonuclease (an enzyme that causes DNA breaks internally within a DNA molecule) to a specific site of the genome and thereby triggering formation of a chromosomal double strand break (DSB) at the chosen site. If, concomitant with the introduction of the chromosome breaks, a donor DNA molecule is introduced (for example, by plasmid or oligonucleotide introduction), interactions between the broken chromosome and the introduced DNA can occur, especially if the two sequences share homology. In this instance, a process termed "gene targeting" can occur, in which the DNA ends of the chromosome invade homologous sequences of the donor DNA by homologous recombination (HR). By using the donor plasmid sequence as a template for HR, a seamless knock out of the gene of interest can be accomplished. Importantly, if the donor DNA molecule includes a deletion within the target gene (e.g., *Xist*), HR-mediated DSB repair will introduce the donor sequence into the chromosome, resulting in the deletion being introduced within the chromosomal locus. By targeting the nuclease to a genomic site that contains the target gene, the concept is to use DSB formation to stimulate HR and to thereby replace the functional target gene with a deleted form of the gene. The advantage of the HR pathway is that it has the potential to generate seamlessly a knockout of the gene in place of the previous wild-type allele.

Current genome editing tools use the induction of double strand breaks (DSBs) to enhance gene manipulation of cells. Such methods include zinc finger nucleases (ZFNs; described for example in U.S. Patent Nos. 6,534,261; 6,607,882; 6,746,838; 6,794,136; 6,824,978; 6,866,997; 6,933,113; 6,979,539; 7,013,219; 7,030,215; 7,220,719; 7,241,573; 7,241,574; 7,585,849; 7,595,376; 6,903,185; and 6,479,626; and U.S. Pat. Publ. Nos. 20030232410 and US2009020314), Transcription Activator-Like Effector Nucleases (TALENs; described for example in U.S. Patent Nos. 8,440,431, 8,440,432, 8,450,471, 8,586,363, and 8,697,853, and U.S. Pat. Publ. Nos. 20110145940, 20120178131, 20120178169, 20120214228, 20130122581, 20140335592, and 20140335618), and the CRISPR (Clustered Regularly Interspaced Short Palindromic Repeats)/Cas9 system (described for example in U.S. Patent Nos. 8,697,359, 8,771,945, 8,795,965, 8,871,445, 8,889,356, 8,906,616, 8,932,814, 8,945,839, 8,993,233, and 8,999,641, and U.S. Pat. Publ. Nos. 20140170753, 20140227787, 20140179006, 20140189896, 20140273231, 20140242664, 20140273232, 20150184139, 20150203872, 20150031134, 20150079681, 20150232882, and 20150247150). For example, ZFN DNA sequence recognition capabilities and specificity can be unpredictable. Similarly, TALENs and CRISPR/Cas9 cleave not only at the desired site, but often at other "off-target" sites, as well. These methods have significant issues connected with off-target double-stranded break induction and the potential for deleterious mutations, including indels, genomic rearrangements, and chromosomal rearrangements, associated with these off-target effects. ZFNs and TALENs entail use of modular sequence-specific DNA binding proteins to generate specificity for ~18 bp sequences in the genome.

RNA-guided nucleases-mediated genome editing, based on Type 2 CRISPR (Clustered Regularly Interspaced Short Palindromic Repeat)/Cas (CRISPR Associated) systems, offers a valuable approach to alter the genome. In brief, Cas9, a nuclease guided by single-guide RNA (sgRNA), binds to a targeted genomic locus next to the protospacer adjacent motif (PAM) and generates a double-strand break (DSB). The DSB is then repaired either by non-homologous end joining (NHEJ), which leads to insertion/deletion (indel) mutations, or by homology-directed repair (HDR), which requires an exogenous template and can generate a precise modification at a target locus (Mali et al., Science. 2013 Feb 15;339(6121):823-6). Unlike other gene therapy methods, which add a functional, or partially functional, copy of a gene to a patient's cells but retain the original dysfunctional copy of the gene, this system can remove the defect. Genetic correction using engineered nucleases has been demonstrated in tissue culture cells and rodent models of rare diseases.

CRISPR has been used in a wide range of organisms including bakers yeast (S. cerevisiae), zebra fish, nematodes (C. elegans), plants, mice, and several other organisms. Additionally CRISPR has been modified to make programmable transcription factors that allow scientists to target and activate or silence specific genes. Libraries of tens of thousands of guide RNAs are now available.

Since 2012, the CRISPR/Cas system has been used for gene editing (silencing, enhancing or changing specific genes) that even works in eukaryotes like mice and primates. By inserting a plasmid containing cas genes and specifically designed CRISPRs, an organism's genome can be cut at any desired location.

CRISPR repeats range in size from 24 to 48 base pairs. They usually show some dyad symmetry, implying the formation of a secondary structure such as a hairpin, but are not truly palindromic. Repeats are separated by spacers of similar length. Some CRISPR spacer sequences exactly match sequences from plasmids and phages, although some spacers match the prokaryote's genome (self-targeting spacers). New spacers can be added rapidly in response to phage infection.

CRISPR-associated (cas) genes are often associated with CRISPR repeat-spacer arrays. As of 2013, more than forty different Cas protein families had been described. Of these protein families, Cas1 appears to be ubiquitous among different CRISPR/Cas systems. Particular combinations of cas genes and repeat structures have been used to define 8 CRISPR subtypes (Ecoli, Ypest, Nmeni, Dvulg, Tneap, Hmari, Apern, and Mtube), some of which are associated with an additional gene module encoding repeat-associated mysterious proteins (RAMPs). More than one CRISPR subtype may occur in a single genome. The sporadic distribution of the CRISPR/Cas subtypes suggests that the system is subject to horizontal gene transfer during microbial evolution.

Exogenous DNA is apparently processed by proteins encoded by Cas genes into small elements (about 30 base pairs in length), which are then somehow inserted into the CRISPR locus near the leader sequence. RNAs from the CRISPR loci are constitutively expressed and are processed by Cas proteins to small RNAs composed of individual, exogenously-derived sequence elements with a flanking repeat sequence. The RNAs guide other Cas proteins to silence exogenous genetic elements at the RNA or DNA level. Evidence suggests functional diversity among CRISPR subtypes. The Cse (Cas subtype Ecoli) proteins (called CasA-E in E. coli) form a functional complex, Cascade, that processes CRISPR RNA transcripts into spacer-repeat units that Cascade retains. In other prokaryotes, Cas6 processes the CRISPR transcripts. Interestingly, CRISPR-based phage inactivation in E. coli requires Cascade and Cas3, but not Cas1 and Cas2. The Cmr (Cas RAMP module) proteins found in Pyrococcus furiosus and other prokaryotes form a functional complex with small CRISPR RNAs that recognizes and cleaves complementary target RNAs. RNA-guided CRISPR enzymes are classified as type V restriction enzymes.

See also U.S. Patent Publication 2014/0068797

### Cas9

Cas9 is a nuclease, an enzyme specialized for cutting DNA, with two active cutting sites, one for each strand of the double helix. The team demonstrated that they could disable one or both sites while preserving Cas9's ability to home located its target DNA. Jinek et al. (2012) combined tracrRNA and spacer RNA into a "single-guide RNA" molecule that, mixed with Cas9, could find and cut the correct DNA targets. It has been proposed that such synthetic guide RNAs might be able to be used for gene editing (Jinek et al., Science. 2012 Aug 17;337(6096):816-21).

Cas9 proteins are highly enriched in pathogenic and commensal bacteria. CRISPR/Cas-mediated gene regulation may contribute to the regulation of endogenous bacterial genes, particularly during bacterial interaction with eukaryotic hosts. For example, Cas protein Cas9 of Francisella novicida uses a unique, small, CRISPR/Cas-associated RNA (scaRNA) to repress an endogenous transcript encoding a bacterial lipoprotein that is critical for F. novicida to dampen host response and promote virulence. Coinjection of Cas9 mRNA and sgRNAs into the germline (zygotes) generated mice with mutations. Delivery of Cas9 DNA sequences also is contemplated.

### gRNA

As an RNA guided protein, Cas9 requires a short RNA to direct the recognition of DNA targets. Though Cas9 preferentially interrogates DNA sequences containing a PAM sequence NGG it can bind here without a protospacer target. However, the Cas9-gRNA complex requires a close match to the gRNA to create a double strand break. CRISPR sequences in bacteria are expressed in multiple RNAs and then processed to create guide strands for RNA. Because Eukaryotic systems lack some of the proteins required to process CRISPR RNAs the synthetic construct gRNA was created to combine the essential pieces of RNA for Cas9 targeting into a single RNA expressed with the RNA polymerase type 2I promoter U6). Synthetic gRNAs are slightly over 100 bp at the minimum length and contain a portion which is targets the 20 protospacer nucleotides immediately preceding the PAM sequence NGG; gRNAs do not contain a PAM sequence.

In one approach, one or more cells of a subject are altered to delete or inactivate *Xist* using a CRISPR-Cas system. Cas9 can be used to target an *Xist* gene. Upon target recognition, Cas9 induces double strand breaks in the *Xist* target gene. Homology-directed repair (HDR) at the double-strand break site can allow insertion of an inactive or deleted form of the *Xist* sequence.

Representative US patents and patent publications include: Patent No. 8,697,359, 20140170753, 20140179006, 20140179770, 20140186843, 20140186958, 20140189896, 20140227787, 20140242664, 20140248702, 20140256046, 20140273230, 20140273233, 20140273234, 20140295556, 20140295557, 20140310830, 20140356956, 20140356959, 20140357530, 20150020223, 20150031132, 20150031133, 20150031134, 20150044191, 20150044192, 20150045546, 20150050699, 20150056705, 20150071898, 20150071899, 20150071903, 20150079681, 20150159172, 20150165054, 20150166980, and 20150184139.

### Therapeutic Methods

Agents modulating H3K27me3 imprinting present in an imprinting control region are useful in generating cloned full term non-human organisms using SCNT. Agents that add H3K27me3 imprinting can be used in combination with an Xist KO cell injected with a *Kdm4d* polynucleotide.

In one approach as described but not specifically claimed herein, an agent that inhibits H3K27me3 demethylase is used in combination with SCNT. The dosage of the administered agent depends on a number of factors, including the size and health of the individual patient. For any particular subject, the specific dosage regimes should be adjusted over time according to the individual need and the professional judgement of the person administering or supervising the administration of the compositions.

The following examples are put forth so as to provide those of ordinary skill in the art with a complete disclosure and description of how to make and use the assay, screening, and therapeutic methods of the invention, and are not intended to limit the scope of what the inventors regard as their invention.

### Somatic cell Nuclear Transfer

Within the context of the present technology, the animals, cells, oocytes, and embryos referred to in the following paragraphs are non-human. Somatic cell nuclear transfer (SCNT) is a technique that may be used, for example, for the reproductive cloning of livestock (e.g., cows, horses, sheep, goats, pigs) or for therapeutic cloning, in which desired tissues are produced for cell replacement therapy. Unfortunately cloned animals suffer from certain defects arising from improper imprinting, such as a deficiency in trimethylation of lysine 27 on histone H3 protein subunit. This deficiency can be remedied by providing an mRNA encoding an enzyme that carries out the trimethylation event during the SCNT procedure. In one embodiment as described but not specifically claimed herein, an mRNA encoding an enzyme capable of carrying out the trimethylation event (e.g., EZH1, EZH2, PRC2) is injected into the recipient cell or the nuclear donor cell prior to or during the SCNT procedure.

Somatic cell nuclear transfer involves obtaining a nuclear donor cell, then fusing this nuclear donor cell into an enucleated recipient cell, most preferably an enucleated oocyte, to form a nuclear transfer embryo, activating this embryo, and finally culturing the embryo or transferring this embryo into a maternal host. During nuclear transfer a full complement of nuclear DNA from one cell is introduced to an enucleated cell. Nuclear transfer methods are well known to a person of ordinary skill in the art. See, U.S. Pat. No. 4,994,384 to Prather et al., entitled "Multiplying Bovine Embryos," issued on Feb. 19, 1991; U.S. Pat. No. 5,057,420 to Massey, entitled "Bovine Nuclear Transplantation," issued on Oct. 15, 1991; U.S. Pat. No. 5,994,619, issued on Nov. 30, 1999 to Stice et al., entitled "Production of Chimeric Bovine or Porcine Animals Using Cultured Inner Cell Mass Cells; U.K. Patents Nos. GB 2,318,578 GB 2,331,751, issued on Jan. 19, 2000 to Campbell et al. and Wilmut et al., respectively, entitled "Quiescent Cell Populations For Nuclear Transfer"; U.S. Pat. No. 6,011,197 to Strelchenko et al., entitled "Method of Cloning Bovines Using Reprogrammed Non-Embryonic Bovine Cells," issued on Jan. 4, 2000; and in U.S. patent application Ser. No. 09/753,323 entitled "Method of Cloning Porcine Animals (attorney docket number 030653.0026.CIP1, filed Dec. 28, 2000). Nuclear transfer may be accomplished by using oocytes that are not surrounded by a zona pellucida.

In a nuclear transfer procedure, a nuclear donor cell, or the nucleus thereof, is introduced into a recipient cell. A recipient cell is preferably an oocyte and is preferably enucleated. However, the invention relates in part to nuclear transfer, where a nucleus of an oocyte is not physically extracted from the oocyte. It is possible to establish a nuclear transfer embryo where nuclear DNA from the donor cell is replicated during cellular divisions. See, e.g., Wagoner et al., 1996, "Functional enucleation of bovine oocytes: effects of centrifugation and ultraviolet light," Theriogenology 46: 279-284. In addition, nuclear transfer may be accomplished by combining one nuclear donor and more than one enucleated oocyte. Also, nuclear transfer may be accomplished by combining one nuclear donor, one or more enucleated oocytes, and the cytoplasm of one or more enucleated oocytes. The resulting combination of a nuclear donor cell and a recipient cell can be referred to as a "hybrid cell."

The term "nuclear donor" as used herein refers to any cell, or nucleus thereof, having nuclear DNA that can be translocated into an oocyte. A nuclear donor may be a nucleus that has been isolated from a cell. Multiple techniques are available to a person of ordinary skill in the art for isolating a nucleus from a cell and then utilizing the nucleus as a nuclear donor. See, e.g., U.S. Pat. Nos. 4,664,097, 6,011,197, and 6,107,543. Any type of cell can serve as a nuclear donor. Examples of nuclear donor cells include, but are not limited to, cultured and non-cultured cells isolated from an embryo arising from the union of two gametes in vitro or in vivo; embryonic stem cells (ES cells) arising from cultured embryonic cells (e.g., pre-blastocyst cells and inner cell mass cells); cultured and non-cultured cells arising from inner cell mass cells isolated from embryos; cultured and non-cultured pre-blastocyst cells; cultured and non-cultured fetal cells; cultured and non-cultured adult cells; cultured and non-cultured primordial germ cells; cultured and non-cultured germ cells (e.g., embryonic germ cells); cultured and non-cultured somatic cells isolated from an animal; cultured and non-cultured cumulus cells; cultured and non-cultured amniotic cells; cultured and non-cultured fetal fibroblast cells; cultured and non-cultured genital ridge cells; cultured and non-cultured differentiated cells; cultured and non-cultured cells in a synchronous population; cultured and non-cultured cells in an asynchronous population; cultured and non-cultured serum-starved cells; cultured and non-cultured permanent cells; and cultured and non-cultured totipotent cells. See, e.g., Piedrahita et al., 1998, Biol. Reprod. 58: 1321-1329; Shim et al., 1997, Biol. Reprod. 57: 1089-1095; Tsung et al., 1995, Shih Yen Sheng Wu Hsueh Pao 28: 173-189; and Wheeler, 1994, Reprod. Fertil. Dev. 6: 563-568. In addition, a nuclear donor may be a cell that was previously frozen or cryopreserved.

Hybrid cells made by the process of nuclear transfer may be used, for example, in reproductive cloning or in regenerative cloning.

SCNT experiments showed that nuclei from adult differentiated somatic cells can be reprogrammed to a totipotent state. Accordingly, a SCNT embryo generated using the methods as disclosed herein can be cultured in a suitable in vitro culture medium for the generation of totipotent or embryonic stem cell or stem-like cells and cell colonies. Culture media suitable for culturing and maturation of embryos are well known in the art. Examples of known media, which may be used for bovine embryo culture and maintenance, include Ham's F-10+10% fetal calf serum (FCS), Tissue Culture Medium-199 (TCM-199)+10% fetal calf serum, Tyrodes-Albumin-Lactate-Pyruvate (TALP), Dulbecco's Phosphate Buffered Saline (PBS), Eagle's and Whitten's media. One of the most common media used for the collection and maturation of oocytes is TCM-199, and 1 to 20% serum supplement including fetal calf serum, newborn serum, estrual cow serum, lamb serum or steer serum. A preferred maintenance medium includes TCM-199 with Earl salts, 10% fetal calf serum, 0.2 Ma pyruvate and 50 ug/ml gentamicin sulphate. Any of the above may also involve co-culture with a variety of cell types such as granulosa cells, oviduct cells, BRL cells and uterine cells and STO cells.

In particular, epithelial cells of the endometrium secrete leukemia inhibitory factor (LIF) during the preimplantation and implantation period. Therefore, in some embodiments, as described but not specifically claimed herein, the addition of LIF to the culture medium is encompassed to enhancing the in vitro development of the SCNT-derived embryos. The use of LIF for embryonic or stem-like cell cultures has been described in U.S. Pat. No. 5,712,156.

Another maintenance medium is described in U.S. Pat. No. 5,096,822 to Rosenkrans, Jr. et al.. This embryo medium, named CR1, contains the nutritional substances necessary to support an embryo. CR1 contains hemicalcium L-lactate in amounts ranging from 1.0 mM to 10 mM, preferably 1.0 mM to 5.0 mM. Hemicalcium L-lactate is L-lactate with a hemicalcium salt incorporated thereon. Also, suitable culture medium for maintaining human embryonic stem cells in culture as discussed in Thomson et al., Science, 282:1145-1147 (1998) and Proc. Natl. Acad. Sci., USA, 92:7844-7848 (1995).

In some embodiments as described but not specifically claimed herein, the feeder cells will comprise mouse embryonic fibroblasts. Means for preparation of a suitable fibroblast feeder layer are described in the example which follows and is well within the skill of the ordinary artisan.

Methods of deriving ES cells (e.g., NT-ESCs or hNT-ESCs) from blastocyst-stage SCNT embryos (or the equivalent thereof) are well known in the art. Such techniques can be used to derive ES cells (e.g., hNT-ESCs) from SCNT embryos, where the SCNT embryos used to generate hNT-ESCs have a reduced level of H3K9me3 in the nuclear genetic material donated from the somatic donor cell, as compared to SCNTs which were not treated with a member of the KDM4 demethylase family and/or an inhibitor of the histone methyltransferase SUV39h1/SUV39h2. Additionally or alternatively, hNT-ESCs can be derived from cloned SCNT embryos during earlier stages of development.

In certain embodiments as described but not specifically claimed herein, blastomeres generated from SCNT embryos generated using the methods, compositions and kits as disclosed herein can be dissociated using a glass pipette to obtain totipotent cells. In some embodiments as described but not specifically claimed herein, dissociation may occur in the presence of 0.25% trypsin (Collas and Robl, 43 BIOL. REPROD. 877-84, 1992; Stice and Robl, 39 BIOL. REPROD. 657-664, 1988; Kanka et al., 43 MOL. REPROD. DEV. 135-44, 1996).

In certain embodiments, the resultant blastocysts, or blastocyst-like clusters from the SCNT embryos can be used to obtain embryonic stem cell lines, eg., nuclear transfer ESC (ntESC) cell lines. Such lines can be obtained, for example, according to the culturing methods reported by Thomson et al., Science, 282:1145-1147 (1998) and Thomson et al., Proc. Natl. Acad. Sci., USA, 92:7544-7848 (1995).

Pluripotent embryonic stem cells can also be generated from a single blastomere removed from a SCNT embryo without interfering with the embryo's normal development to birth. See U.S. application Nos. 60/624,827, filed Nov. 4, 2004; 60/662,489, filed Mar. 14, 2005; 60/687,158, filed Jun. 3, 2005; 60/723,066, filed Oct. 3, 2005; 60/726,775, filed Oct. 14, 2005; 11/267,555 filed Nov. 4, 2005; PCT application no. PCT/US05/39776, filed Nov. 4, 2005; see also Chung et al., Nature, Oct. 16, 2005 (electronically published ahead of print) and Chung et al., Nature V. 439, pp. 216-219 (2006)). In such a case, an SCNT embryo is not destroyed for the generation of pluripotent stem cells.

In one aspect of the invention as described but not specifically claimed herein, the method comprises the utilization of cells derived from the SCNT embryo in research and in therapy. Such pluripotent stem cells (PSCs) or totipotent stem cells (TSC) can be differentiated into any of the cells in the body including, without limitation, skin, cartilage, bone, skeletal muscle, cardiac muscle, renal, hepatic, blood and blood forming, vascular precursor and vascular endothelial, pancreatic beta, neurons, glia, retinal, inner ear follicle, intestinal, or lung cells.

In another embodiment of the invention as described but not specifically claimed herein, the SCNT embryo, or blastocyst, or pluripotent or totipotent cells obtained from a SCNT embryo (e.g., NT-ESCs), can be exposed to one or more inducers of differentiation to yield other therapeutically-useful cells such as retinal pigment epithelium, hematopoietic precursors and hemangioblastic progenitors as well as many other useful cell types of the ectoderm, mesoderm, and endoderm. Such inducers include but are not limited to: cytokines such as interleukin-alpha A, interferon-alpha A/D, interferon-beta, interferon-gamma, interferon-gamma-inducible protein-10, interleukin-1-17, keratinocyte growth factor, leptin, leukemia inhibitory factor, macrophage colony-stimulating factor, and macrophage inflammatory protein-1 alpha, 1-beta, 2, 3 alpha, 3 beta, and monocyte chemotactic protein 1-3, 6kine, activin A, amphiregulin, angiogenin, B-endothelial cell growth factor, beta cellulin, brain-derived neurotrophic factor, C10, cardiotrophin-1, ciliary neurotrophic factor, cytokine-induced neutrophil chemoattractant-1, eotaxin, epidermal growth factor, epithelial neutrophil activating peptide-78, erythropoietin, estrogen receptor-alpha, estrogen receptor-beta, fibroblast growth factor (acidic and basic), heparin, FLT-3/FLK-2 ligand, glial cell line-derived neurotrophic factor, Gly-His-Lys, granulocyte colony stimulating factor, granulocytemacrophage colony stimulating factor, GRO-alpha/MGSA, GRO-beta, GRO-gamma, HCC-1, heparin-binding epidermal growth factor, hepatocyte growth factor, heregulin-alpha, insulin, insulin growth factor binding protein-1, insulin-like growth factor binding protein-1, insulin-like growth factor, insulin-like growth factor II, nerve growth factor, neurotophin-3,4, oncostatin M, placenta growth factor, pleiotrophin, rantes, stem cell factor, stromal cell-derived factor 1B, thromopoietin, transforming growth factor--(alpha, beta 1,2,3,4,5), tumor necrosis factor (alpha and beta), vascular endothelial growth factors, and bone morphogenic proteins, enzymes that alter the expression of hormones and hormone antagonists such as 17B-estradiol, adrenocorticotropic hormone, adrenomedullin, alpha-melanocyte stimulating hormone, chorionic gonadotropin, corticosteroid-binding globulin, corticosterone, dexamethasone, estriol, follicle stimulating hormone, gastrin 1, glucagons, gonadotropin, L-3,3',5'-triiodothyronine, leutinizing hormone, L-thyroxine, melatonin, MZ-4, oxytocin, parathyroid hormone, PEC-60, pituitary growth hormone, progesterone, prolactin, secretin, sex hormone binding globulin, thyroid stimulating hormone, thyrotropin releasing factor, thyroxin-binding globulin, and vasopressin, extracellular matrix components such as fibronectin, proteolytic fragments of fibronectin, laminin, tenascin, thrombospondin, and proteoglycans such as aggrecan, heparan sulphate proteoglycan, chontroitin sulphate proteoglycan, and syndecan. Other inducers include cells or components derived from cells from defined tissues used to provide inductive signals to the differentiating cells derived from the reprogrammed cells of the present invention. Such inducer cells may derive from human, non-human mammal, or avian, such as specific pathogen-free (SPF) embryonic or adult cells.

Blastomere Culturing. In one embodiment as described but not specifically claimed herein, the SCNT embryos can be used to generate blastomeres and utilize in vitro techniques related to those currently used in pre-implantation genetic diagnosis (PGD) to isolate single blastomeres from a SCNT embryo, generated by the methods as disclosed herein, without destroying the SCNT embryos or otherwise significantly altering their viability. As demonstrated herein, pluripotent embryonic stem (hES) cells and cell lines can be generated from a single blastomere removed from a SCNT embryo as disclosed herein without interfering with the embryo's normal development to birth.

The discoveries of Wilmut et al. (Wilmut, et al, Nature 385, 810 (1997) in sheep cloning of "Dolly", together with those of Thomson et al. (Thomson et al., Science 282, 1145 (1998)) in deriving hESCs, have generated considerable enthusiasm for regenerative cell transplantation based on the establishment of patient-specific hESCs derived from SCNT-embryos or SCNT-engineered cell masses generated from a patient's own nuclei. This strategy, aimed at avoiding immune rejection through autologous transplantation, is perhaps the strongest clinical rationale for SCNT. By the same token, derivations of complex disease-specific SCNT-hESCs may accelerate discoveries of disease mechanisms. For cell transplantations, innovative treatments of murine SCID and PD models with the individual mouse's own SCNT-derived mESCs are encouraging (Rideout et al, Cell 109, 17 (2002); Barberi, Nat. Biotechnol. 21, 1200 (2003)). Ultimately, the ability to create banks of SCNT-derived stem cells with broad tissue compatibility would reduce the need for an ongoing supply of new oocytes.

In certain embodiments of the invention as described but not specifically claimed herein, pluripotent or totipotent cells obtained from a SCNT embryo (e.g., hNT-ESCs) can be optionally differentiated, and introduced into the tissues in which they normally reside in order to exhibit therapeutic utility. For example, pluripotent or totipotent cells obtained from a SCNT embryo can be introduced into the tissues. In certain other embodiments as described but not specifically claimed herein, pluripotent or totipotent cells obtained from a SCNT embryo can be introduced systemically or at a distance from a cite at which therapeutic utility is desired. In such embodiments, the pluripotent or totipotent cells obtained from a SCNT embryo can act at a distance or may hone to the desired cite.

In certain embodiments of the invention as described but not specifically claimed herein, cloned cells, pluripotent or totipotent cells obtained from a SCNT embryo can be utilized in inducing the differentiation of other pluripotent stem cells. The generation of single cell-derived populations of cells capable of being propagated in vitro while maintaining an embryonic pattern of gene expression is useful in inducing the differentiation of other pluripotent stem cells. Cell-cell induction is a common means of directing differentiation in the early embryo. Many potentially medically-useful cell types are influenced by inductive signals during normal embryonic development including spinal cord neurons, cardiac cells, pancreatic beta cells, and definitive hematopoietic cells. Single cell-derived populations of cells capable of being propagated in vitro while maintaining an embryonic pattern of gene expression can be cultured in a variety of in vitro, in ovo, or in vivo culture conditions to induce the differentiation of other pluripotent stem cells to become desired cell or tissue types.

The pluripotent or totipotent cells obtained from a SCNT embryo (e.g., ntESCs) can be used to obtain any desired differentiated cell type. Therapeutic usages of such differentiated cells are unparalleled. As discussed herein, the donor cell, or the recipient oocyte, the hybrid oocyte or SCNT embryo can be treated with a KDM4 histone dimethylase activator and/or H3K9 methyltransferase inhibitor according to the methods as disclosed herein.

Alternatively, the donor cells can be adult somatic cells from a subject with a disorder, and the generated SCNT embryos can be used to produce animal models of disease or disease-specific pluripotent or totipotent cells which can be cultured under differentiation conditions to produce cell models of disease. The great advantage of the present invention is that by increasing the efficiency of SCNT, it provides an essentially limitless supply of isogenic or syngeneic ES cells, particularly pluripotent that are not induced pluripotent stem cells (e.g., not iPSCs). Such NT-ESCs have advantages over iPSCs and are suitable for transplantation, as they do not partially pluripotent, and do not have viral transgenes or forced expression of reprogramming factors to direct their reprogramming.

In some embodiments as described but not specifically claimed herein, the NT-ESCs generated from the SCNTs are patient-specific pluripotent obtained from SCNT embryos, where the donor cell was obtained from a subject to be treated with the pluripotent stem cells or differentiated progeny thereof. Therefore, it will obviate the significant problem associated with current transplantation methods, i.e., rejection of the transplanted tissue which may occur because of host-vs-graft or graft-vs-host rejection. Conventionally, rejection is prevented or reduced by the administration of anti-rejection drugs such as cyclosporin. However, such drugs have significant adverse side-effects, e.g., immunosuppression, carcinogenic properties, as well as being very expensive. The present invention should eliminate, or at least greatly reduce, the need for anti-rejection drugs, such as cyclosporine, imulan, FK-506, glucocorticoids, and rapamycin, and derivatives thereof.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the invention, and, as such, may be considered in making and practicing the invention. Particularly useful techniques for particular embodiments will be discussed in the sections that follow.

The following examples are put forth so as to provide those of ordinary skill in the art with a complete disclosure and description of how to make and use the assay, screening, and therapeutic methods of the invention, and are not intended to limit the scope of what the inventors regard as their invention.

### EXAMPLES

### Example 1: Kdm4d injection does not alleviate SCNT-associated abnormal Xist activation

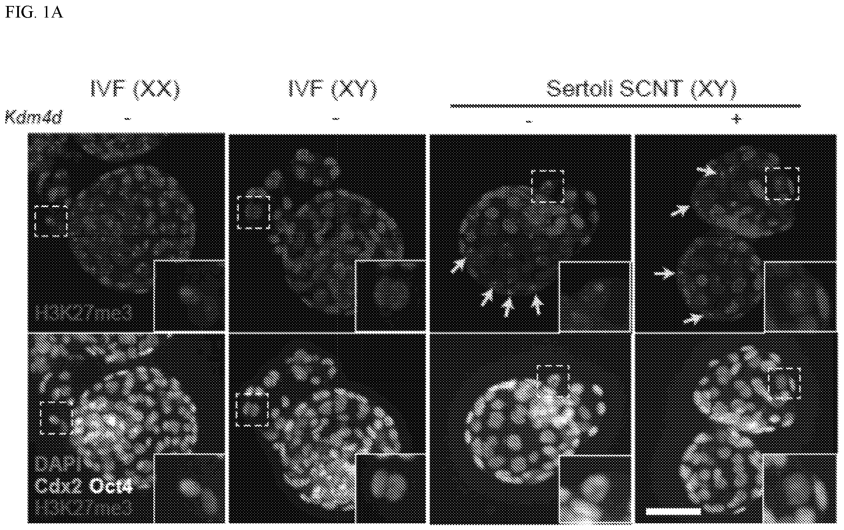

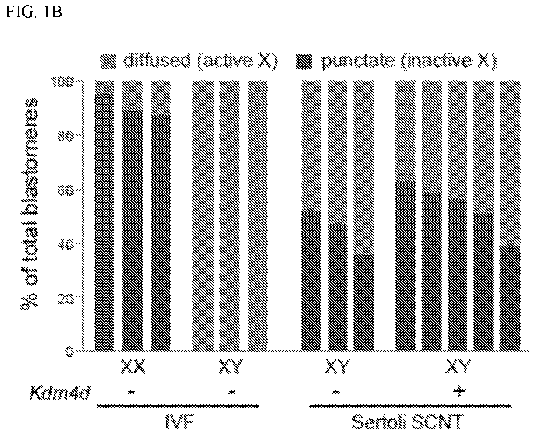

The inactive X chromosome is marked by its punctate staining with an anti-H3K27me3 antibody. Consistently, such punctate staining is detectable only in female (XX) cells but not in male (XY) cells in *in vitro* fertilized (IVF) embryos. In contrast, such punctate staining was observed in SCNT embryos when male Sertoli cells were used as donor cells (FIG. 1A), suggesting abnormal *Xist* activation in SCNT embryos. Importantly, *Kdm4d* injection does not alter the punctate staining pattern or its frequency compared to no-injection control SCNT embryos (FIGs. 1A, 1B). These results demonstrated that H3K9me3 in donor cells and ectopic activation of *Xist* in SCNT embryos are two independent barriers in SCNT reprogramming.

### Example 2: Combinational use of Xist mutant donor cell and Kdm4d mRNA injection greatly improves cloning efficiency

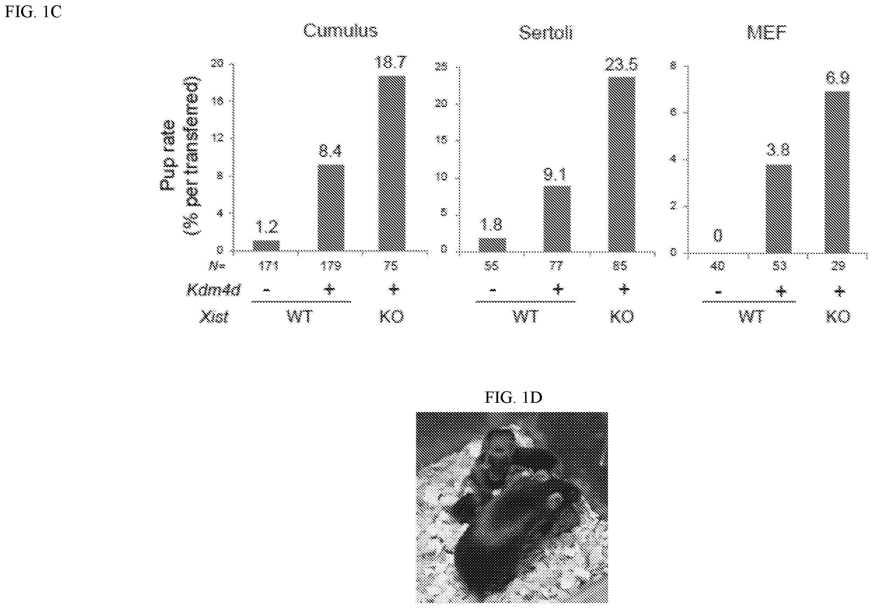

The fact that the two reprogramming barriers are independent of each other prompted the question of whether a combined approach of using *Xist* KO donor cells and injecting *Kdm4d* will have a synergistic or additive effect to achieve increased cloning efficiency. SCNT was attempted using cumulus cells as donors. In wildtype control with B6D2F1 background, only 1.2% of embryos transferred to surrogate mothers reached term (FIG. 1C; Table 2).

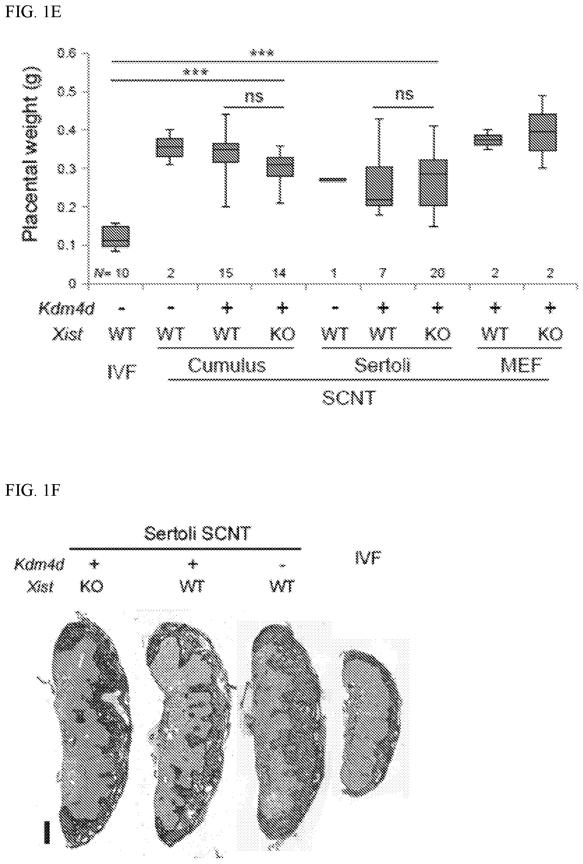

**Table 2. Postimplantation development of SCNT embryos, Related to Figure 1**

| No. of recipients | No. of 2-cell embryos transferred | No. of implanted (% per ET) | No. of pups (% per ET) | Body weight at birth (g ± SD) | Placenta weight at birth (g ± SD) |
|---|---|---|---|---|---|
| 6 | 171 | 52 (30.4) | 2 (1.2) | 1.56 ± 0.04 | 0.36 ± 0.06 |
| 8 | 179 | 110 (61.5) | 15 (8.4) | 1.59 ± 0.14 | 0.34 ± 0.06 |
| 4 | 75 | 46 (61.3) | 14 (18.7) | 1.50 ± 0.12 | 0.30 ± 0.04 |
| 3 | 55 | 25 (45.5) | 1 (1.8) | 1.50 | 0.28 |
| 4 | 77 | 47 (61.0) | 7 (9.1) | 1.48 ± 0.11 | 0.26 ± 0.10 |
| 4 | 85 | 57 (67.1) | 20 (23.5) | 1.46 ± 0.16 | 0.27 ± 0.08 |
| 5 | 40 | 15 (37.5) | 0 (0.0) | N/A | N/A |
| 5 | 53 | 36 (67.9) | 2 (3.8) | 1.70 ± 0.18 | 0.38 ± 0.04 |
| 5 | 29 | 23 (79.3) | 2 (6.9) | 1.89 ± 0.25 | 0.40 ± 0.13 |

| | | | | | |
|---|---|---|---|---|---|
| Concentration of injected Kdm4d mRNA was 1500 ng/ul. N/A, not applicable. ET, embryo transfer. | | | | | |

*Kdm4d* mRNA injection increased the pup rate to 8.4%, consistent with previous observations (Matoba et al., Proc. Natl. Acad. Sci. U. S. A. 108, 20621-20626, 2014). When cumulus cells derived from *Xist* heterozygous mice were used as donor cells and combined with *Kdm4d* mRNA injection, the pup rate increased to 18.7% (FIG. 1C; Table S1). Similarly, the pup rate of Sertoli cell-derived SCNT embryos was improved from 1.8% to 9.1% by *Kdm4d* mRNA injection, and further increased to 23.5% by combining *Xist* KO with *Kdm4d* mRNA injection (FIG. 1C; Table 2), which is the highest mouse cloning rate ever reported (Ogura et al., Phil. Trans. R. Soc. B 368, 20110329, 2013). Importantly, this additive effect was also observed using MEF cells of a different *Xist* mutant line in a hybrid (129S1/Svj x CAST/EiJ) genetic background (FIG. 1C; Table 2). The pups generated using the combined approach grew up to adulthood and showed normal fertility (FIG. 1D).

The above results indicate that the invention provides the highest mouse cloning efficiency by using the combined approach. However, even the highest cloning efficiency of 23.5% is still less than half of the IVF pup rate, which is more than 50% of transferred embryos. Indeed, 65% (37 out of 57) of the implanted embryos arrested during postimplantation development in the combined Sertoli cell SCNT group (Table 2). A careful morphological examination at different embryonic stages revealed that the embryo arrest starts just after implantation and gradually increases as development proceeds (FIG. 2A). Moreover, morphological and histological analyses revealed that the large placental phenotype (FIG. 1E), which is associated with invasion of the PAS-positive spongiotroblast cells into the labyrinth layer, was not rescued by *Kdm4d* mRNA injection or the combined approach (FIGs. 1E and 1F; Table 2). Thus, despite the combined positive effect of *Xist* KO and *Kdm4d* mRNA injection in improving SCNT embryo development, other reprogramming barriers may contribute to the developmental failure of these SCNT embryos.

### Example 3: Extensive DNA methylation reprogramming is achieved in combinational reprogrammed SCNT blastocysts

Since the combinational reprogrammed SCNT embryos begin to exhibit developmental defects right after implantation (FIGs. 2A, 2B, 2C), it was postulated that reprogramming related epigenetic defects would have already existed in the SCNT blastocysts although they appear morphologically normal. To identify such epigenetic defects, whole genome bisulfite sequencing (WGBS) data of both SCNT blastocysts derived from *Xist* KO MEF cells (129S1/Svj x CAST/EiJ background) combined with *Kdm4d* mRNA injection was generated and compared with that of genetically matched IVF blastocysts (FIG. 3A).

DNA methylation information of 20.6 and 20.9 million CpG sites from IVF and SCNT blastocysts, respectively (Table 3).

**Table 3. Summary of WGBS libraries, Related to Figure 2**

| Samples | Total sequencing reads | Mapped reads | Percentage of mapped reads (%) | 1x covered CpGs | 5x covered CpGs | Bisulfite conversion rate (%) |
|---|---|---|---|---|---|---|
| IVF blastocyst | 416,465,544 | 297,678,300 | 71.5 | 20,611,901 | 12,717,260 | 99.2 |
| SCNT blastocyst | 412,706,010 | 298,966,969 | 72.4 | 20,676,648 | 12,506,862 | 99.2 |

For comparison, WGBS datasets were also obtained for MEF (Yu et al., Proc. Natl. Acad. Sci. U. S. A. 111, 5890-5895, 2014), sperm and oocyte (Wang et al., Cell 157, 979-991, 2014) from public database. First, the average DNA methylation level of all covered CpGs was calculated. It was found that the average methylation levels in sperm and oocyte are 82.2% and 58.8%, respectively. The average methylation level of sperm and oocyte, which serves as the starting methylation level of IVF zygotes, is 70.5% (FIG. 3B). However, the highly methylated gametes were globally reprogrammed to a low methylation level by the blastocyst stage (19.1%) likely due to the active and passive demethylation processes taking place during preimplantation development.

Next, the DNA methylation levels of MEF cells and SCNT blastocysts of the commonly covered CpGs was calculated, and it was found that the donor MEF cells are highly methylated (78.0%), but the SCNT blastocysts were lowly methylated with a methylation level (15.6%) similar to that of the IVF blastocysts (19.1%) indicating successful global reprogramming of DNA methylation state (FIG. 3B). Indeed, pairwise comparisons of DNA methylation revealed both IVF and SCNT blastocysts possess extremely low DNA methylation compared to that in gametes or MEF cells (FIG. 3C). Not only the global methylation level, but also the distribution of methylated CpG is similar (FIG. 5A). Consistent with similar global DNA methylation pattern, RNA-seq revealed highly similar transcriptomes between IVF and SCNT blastocysts (R = 0.988) (FIGs. 3D, 4A, 4B, and 5B). Indeed, among the 8,921 genes detected (FPKM>3 in at least one sample), only 92 genes were differentially expressed (FC>3) in SCNT blastocysts (FIG. 5D, Table 4).

**Table 4: Differentially Regulated Genes in SCNT Blastocysts**

| **symbol** | **state** | **Chr** |
|---|---|---|
| Msc | down | Chr1 |
| Crygd | down | Chr1 |
| Ren1 | down | Chr1 |
| Mael | down | Chr1 |
| Car4 | down | Chr11 |
| Cbr2 | down | Chr11 |
| Cd7 | down | Chr11 |
| Scama3b | down | Chr12 |
| Acot1 | down | Chr12 |
| Cox8c | down | Chr12 |
| Pi1 | down | Chr13 |
| Bhmt2 | down | Chr13 |
| Sult4a1 | down | Chr15 |
| Tnp2 | down | Chr16 |
| Fetub | down | Chr16 |
| Kng1 | down | Chr16 |
| Impact | down | Chr18 |
| Pde6a | down | Chr18 |
| 1700123I 01Rik | down | Chr19 |
| Fabp9 | down | Chr3 |
| Fabp4 | down | Chr3 |
| Slc25a31 | down | Chr3 |
| S100a3 | down | Chr3 |
| Tdpoz4 | down | Chr3 |
| Gstm6 | down | Chr3 |
| Scama2 | down | Chr3 |
| Fam154a | down | Chr4 |
| Cox7b2 | down | Chr5 |
| Asz1 | down | Chr6 |
| Npy | down | Chr6 |
| Tuba3b | down | Chr6 |
| Tex101 | down | Chr7 |
| Klk7 | down | Chr7 |
| Rbmxl2 | down | Chr7 |
| Adrb3 | down | Chr8 |
| Tat | down | Chr8 |
| Tex12 | down | Chr9 |
| 2410076I 21Rik | down | Chr9 |
| Rbp2 | down | Chr9 |
| AU0227 51 | down | ChrX |
| 1700013 H16Rik | down | ChrX |
| Xlr | down | ChrX |
| Gm773 | down | ChrX |
| 4930550 L24Rik | down | ChrX |
| Xlr3a | down | ChrX |
| Xlr5a | down | ChrX |
| Xlr5b | down | ChrX |
| Xlr3b | down | ChrX |
| Xlr4b | down | ChrX |
| Xlr4c | down | ChrX |
| Xlr3c | down | ChrX |
| Magea8 | down | ChrX |
| Magea5 | down | ChrX |
| Gm6432 | down | Chr9 |
| LOC100 861571 | down | Chr12 |
| Scarna3a | up | Chr1 |
| Derl3 | up | Chr10 |
| Ddit3 | up | Chr10 |
| Krt19 | up | Chr11 |
| Jdp2 | up | Chr12 |
| Serpina3 m | up | Chr12 |
| BC05166 5 | up | Chr13 |
| Slc39a2 | up | Chr14 |
| Slc7a8 | up | Chr14 |
| Entpd1 | up | Chr19 |
| Aif11 | up | Chr2 |
| Elf5 | up | Chr2 |
| Chac1 | up | Chr2 |
| Trib3 | up | Chr2 |
| Defb25 | up | Chr2 |
| Myl9 | up | Chr2 |
| Ptk6 | up | Chr2 |
| Glipr2 | up | Chr4 |
| Tspan1 | up | Chr4 |
| Plac8 | up | Chr5 |
| Apoc2 | up | Chr7 |
| LOC100 302567 | up | Chr7 |
| Parva | up | Chr7 |
| Nupr1 | up | Chr7 |
| H19 | up | Chr7 |
| Gm514 | up | Chr9 |
| Ostb | up | Chr9 |
| Praf2 | up | ChrX |
| Rhox6 | up | ChrX |
| Plac1 | up | ChrX |
| 0610009 L18Rik | up | Chr11 |
| 1700019 E08Rik | up | Chr2 |
| 4930461 G14Rik | up | Chr9 |
| 4930591 | up | Chr2 |
| A17Rik | | |
| AF35742 6 | up | Chr12 |
| Gm5480 | up | Chr16 |
| LOC100 861570 | up | Chr12 |

These results indicate that DNA methylation and transcriptome are largely reprogrammed by SCNT at the blastocyst stage.

### Example 4: Identifying and characterizing differentially methylated regions (DMRs) in SCNT blastocysts

Despite successful global reprogramming of the DNA methylome, the mean methylation level of SCNT blastocysts (15.6%) was slightly but significantly lower (p value < 2.2e-16) than that of the IVF blastocysts (19.1%) (FIG. 5B). Genome-wide scanning analysis (10 CpGs as a minimum window size) was performed to identify differentially methylated regions (DMRs) between SCNT and IVF blastocysts, which uncovered 56,240 DMRs with absolute methylation difference greater than 10% (FIG. 5A). The majority of these DMRs (48,315: 85.9%) showed lower DNA methylation in SCNT compared to that of IVF and were termed hypoDMRs. 7,925 regions with higher DNA methylation in SCNT compared to that of IVF were identified and were termed hyperDMRs. Interestingly, the average length of hyperDMRs (741 bp) is much shorter than that of hypoDMRs (5,743 bp) (FIG. 5B). Indeed, a representative hyperDMR is present in the promoter/enhancer region as a sharp peak, while a representative hypoDMR covers an entire gene-coding region (FIGs. 6A, 6B). Consistently, hyperDMRs and hypoDMRs exhibit distinct genomic distributions with hyperDMR enriched in intergenic regions, while hypoDMR enriched in gene body (FIG. 5C).

To understand how these DMRs are formed and whether they could contribute to the post-implantation developmental failure of the SCNT embryos, the analysis focused on the hypoDMRs. In sperm, the methylation level at hypoDMRs was significantly higher than the flanking regions (~90% vs ~80%) (FIG. 5D). In oocyte, the methylation difference between hypoDMRs and the flanking regions is even greater (~75% vs ~60%) (FIG. 3D). In contrast, the methylation difference between hypoDMRs and the flanking regions is much smaller in MEFs (FIG. 5D). Thus, the hypoDMRs of SCNT blastocyst correlate well with their relatively higher DNA methylation levels in gametes, which remain to be at a higher level in the IVF blastocysts should both SCNT and IVF embryos go through the same number of replication-dependent dilution. Consistent with this notion, a visual inspection of representative hypoDMRs in genome browser view revealed that the methylation peaks in oocytes clearly overlap with those in IVF blastocysts (FIG. 6B). Allelic DNA methylation analysis also supports this notion as the methylation pattern in IVF blastocysts are strongly biased to maternal allele (FIG. 5E). Indeed, analysis of published WGBS datasets of preimplantation embryos (Wang et al., Cell 157, 979-991, 2014) revealed that the maternal allele maintains its high DNA methylation level at hypoDMRs until 4 cell-stage, while paternal allele quickly loses its methylation at these regions (FIG. 5F).

Next, the hyperDMRs were analyzed. The methylation levels at hyperDMRs and flanking regions are similar (~50%) in oocytes, while it is significantly lower than the flanking regions (~55% vs ~80%) in sperm (FIG. 5G). Despite their difference in methylation levels, both hyperDMRs and flanking regions were demethylated to a very low level (~20%) in IVF blastocysts (FIG. 5G). In contrast, hyperDMRs were heavily methylated (~80%) with even higher methylation level than that of the flanking regions in MEFs (FIG. 5G). The fact that hyperDMRs were heavily methylated in MEF but not in gametes suggest that low methylation at these regions might be a unique feature of germline. Indeed, analyses of public DNA methylome datasets of different cell types revealed that hyperDMRs are indeed heavily methylated in all somatic cell types analyzed, but are less methylated only in spermatocyte, spermatid and oocyte (FIG. 5H). Consistently, GO analysis of the genes associated with hyperDMRs revealed significant enrichment of germline related functions such as spermatogenesis and gametogenesis (FIG. 6C). HyperDMRs appear to be demethylated during primordial germ cell (PGC) development by Tet1 (Yamaguchi et al., Nature 504, 460-464, 2013) as hydroxymethylcytosines (5hmC) was significantly enriched in the hyperDMRs in PGCs (FIG. 6D). This suggests that hyperDMRs are mostly related to germline development but not embryonic development.

### Example 5: Loss of H3K27me3-dependent imprinting in SCNT blastocysts

Defective placental development is a central feature in SCNT embryos. Previous studies have established that genomic imprinting plays a critical role in placental development. Therefore, besides the identified DRMs that can potentially contribute to the post-implantation defects of the SCNT embryos, it is important to evaluate the impact of a combinatorial approach on genomic imprinting. To this end, the DNA methylation level of the 23 known imprinting control regions (ICRs) was analyzed as allelic expression of imprinted genes is largely controlled by allelic ICR methylation. Comparative DNA methylation analysis revealed that although DNA methylation levels are slightly reduced at most ICRs, 21 out of the 23 ICRs maintained at least half that of the IVF blastocysts level (FIG. 8A), indicating DNA methylation-mediated genomic imprinting was largely maintained. Indeed, all the 20 ICRs with sufficient allele specific methylation information (> 5 detected CpG in both alleles of both IVF and SCNT blastocysts) showed consistent allele specific DNA methylation between IVF and SCNT blastocysts (FIG. 8B).

To further evaluate the potential role of DNA methylation-mediated genomic imprinting in the SCNT defects, RNAseq datasets were analyzed focusing on the 126 known imprinted genes. Of the 45 imprinted genes reliably detectable in IVF blastocysts (FPKM > 1), only 6 were significantly upregulated in SCNT blastocysts compared to that in IVF blastocysts (FC > 1.5) (FIG. 8C). Allelic expression analysis (FPKM>1, mean SNP reads > 10 in either sample) revealed that among the 36 imprinted genes with sufficient number of SNP reads, 6 showed maternal-allele biased (Mat/Pat > 2.0) and 13 showed paternal-allele biased (Pat/Mat >2.0) expression in IVF blastocysts (FIGs. 8D and 8E, lighter bars). All 6 maternally expressed genes (MEGs) maintained their maternal biased expression in SCNT blastocysts (FIG. 8D). Among the 13 paternally expressed genes (PEGs), 7 lost allelic bias and become biallelically expressed in SCNT blastocysts (arrows in FIG. 8E). Interestingly, the 7 PEGs that lost imprinted expression in SCNT blastocysts include *Slc38a4, Sfmbt2, Phf17* and *Gab1* (darker bars in FIG. 8E) whose imprinted expression is known to be independent of DNA methylation, but dependent on maternally deposited H3K27me3 (Inoue et al., Nature 547, 419-424, 2017).

The analysis focused on the H3K27me3-dependent imprinted genes that were recently identified, but that are not included in the above analysis. Among the 76 genes that exhibit H3K27me3-dependent imprinted expression in the morula embryos (Inoue et al., Nature 547, 419-424, 2017), 26 are expressed at a reliably detectable level (FPKM >1) in IVF blastocysts. Interestingly, the majority of them (15/26) are significantly upregulated in SCNT blastocysts (FC>1.5) (FIG. 7A). Allelic expression analysis revealed that, of the 23 genes with sufficient SNP reads (FPKM>1, mean SNP reads > 10 in either sample), 17 showed paternally biased expression (Pat/Mat > 2.0) in IVF blastocysts under the genetic background analyzed (129S1/Svj x CAST/EiJ). Strikingly, all the 17 PEGs lost paternal allele-biased expression and showed biallelic expression (FIG. 7B). These results clearly demonstrated that H3K27me3-dependent imprinted genes completely lose their imprinting in SCNT blastocysts.

Why do these H3K27me3-dependent imprinted genes lose imprinting in SCNT blastocysts? Since imprinting status of these genes is regulated by maternal allele-specific H3K27me3 domains deposited during oogenesis, it is possible that the H3K27me3 pattern in donor MEFs may differ from that in oocytes. Analysis of available H3K27me3 ChIP-seq datasets of fully grown oocyte and MEF cells revealed that the H3K27me3 domains at these imprinted genes in oocyte were completely absent in MEF cells (FIGs. 7C and 8F). The analysis was expanded to include other somatic cell types. This analysis found that the H3K27me3 domains in these imprinted genes were generally absent from the somatic cell types analyzed and therefore were unique to the oocyte genome (FIG. 8D). These results indicate that lack of H3K27me3 methylation at the maternal allele of these imprinted genes in donor somatic cells is likely the cause of loss-of-imprinting (LOI) after SCNT.

Despite successful cloning of more than 20 mammalian species by SCNT =, the cloning efficiency is uniformly low and developmental abnormalities including placenta overgrowth are observed in essentially all cloned mammalian species. It has been speculated that epigenetic abnormalities are responsible for the developmental failure of the cloned animals. In this study, two approaches were combined to overcome two previously identified reprogramming barriers that impede development of mouse SCNT embryos. The combinatorial use *of Xist* KO donor somatic cells and *Kdm4d* mRNA injection indeed increased the overall cloning efficiency (term rate) by 20-fold to generate the highest pup rate (e.g., 23% using Sertoli cells) ever reported in mouse reproductive cloning using somatic donor cells. This efficiency is remarkable as it is close to that of intra-cytoplasmic sperm- or round spermatid-injection (ICSI/ROSI) where similar nuclear injection is involved (Ogonuki et al., PLoS One 5, 2010; Ogura et al., International Review of Cytology, pp. 189-2292005). This achievement clearly demonstrates that H3K9me3 in donor cells and abnormal activation *of Xist* represent two major barriers for successful cloning, and therefore establishes a foundation for understanding the molecular mechanisms of SCNT-mediated reprogramming.

Despite the remarkable improvement, many of the SCNT embryos generated using the combinational approach failed to develop after implantation. Moreover, placental overgrowth was still observed regardless of the donor cell types indicating additional barriers exist for high efficiency animal cloning. To identify these additional reprogramming barriers, w the time when the developmental failure begins was identified and the first WGBS dataset was generated from SCNT blastocysts right before the developmental phenotype appears. Comparative DNA methylome analysis revealed successful global DNA methylome reprogramming by SCNT, indicating that the DNA demethylation machineries in ooplasm and cleavage embryos are similarly functional in SCNT embryos compared to that in IVF embryos. Nevertheless, detailed comparative analysis of DNA methylomes revealed many DMRs across the genome between IVF and SCNT blastocysts. Interestingly, hyperDMRs are enriched in genomic regions demethylated in germline, which is consistent with the fact that germ cell-specific genes are demethylated by Tet1 during germ cell development, particularly at the primordial germ cell (PGC) stage. Yet SCNT bypasses this demethylation processes. The list of hyperDMR- associated genes does not include a few germline genes reported to be quickly demethylated at 1cell SCNT embryos, indicating that some germline genes, but not the majority, are subjected to demethylation after SCNT. On the other hand, hypoDMRs mainly overlap with regions that are methylated in oocytes. Maternal DNA methylation at these regions appears to escape the demethylation processes particularly before the 8-cell stage in IVF embryos. The underlying mechanism for the maternal allele-specific maintenance of DNA methylation before the 8-cell stage is of interest for future study. Collectively, it appears that the SCNT specific DMRs, either high or low, are formed due to the unique feature of gametogenesis which are inherited to the blastocysts through normal fertilization. Maternal DNA methylation passed down from oocyte to embryos through fertilization has been shown to play important roles in early stage trophoblast development (Branco et al., Dev. Cell 36, 152-163, 2016). Therefore, loss of oocyte-like DNA methylation pattern in SCNT blastocysts may contribute to developmental phenotypes of SCNT embryos.

As reported herein, DNA methylation and transcriptome analysis of DNA methylation-imprinted genes revealed that most ICRs largely maintain their normal imprinting status and that most canonical imprinted genes indeed maintain allelic expression pattern in SCNT blastocysts. In contrast, the recently discovered H3K27me3-mediated non-canonical imprinted genes (Inoue et al., 2017) are totally dysregulated and exhibit biallelic expression in SCNT blastocysts. The list of dysregulated non-canonical imprinted genes in SCNT blastocysts included *Slc38a4, Sfmbt2* and *Gab1,* consistent with a previous report of loss-of-imprint (LOI) of these three genes in placenta of E13.5 SCNT embryos (Okae et al., Hum. Mol. Genet. 23, 992-1001, 2014). Given all of the three genes have been shown to play important roles in placental growth, LOI of these genes likely contribute to the placenta overgrowth phenotype of SCNT embryos. Moreover, *Runx1, Otx2* and *Etv6* have been shown to play critical roles in mouse early embryonic development, therefore LOI of these genes at the blastocyst stage may contribute to embryonic lethality phenotype of postimplantation SCNT embryos. The causes of LOI of non-canonical imprinted genes in SCNT are most likely due to the absence of H3K27me3 at these loci in the donor somatic cells. Further detailed analysis on the regulatory mechanisms of the H3K27me3-imprinted genes will provide clues for improving SCNT embryo development.

In summary, in addition to establishing the most efficient mouse cloning method by combining *Kdm4d* mRNA injection with the use of *Xist* KO donor cells, H3K27me3 imprinting was uncovered as a potential barrier preventing efficient animal cloning. Without intending to be bound by theory, based on the clear association of LOI at the H3K27me3-dependent imprinted genes in mouse SCNT blastocysts and their critical functions in embryonic development, LOI at the H3K27me3-imprinted genes most likely accounted for the postimplantation phenotypes of SCNT embryos, although the possibility of potential contribution of abnormal DNA methylation identified in this study cannot be excluded. Given that defective postimplantation development and abnormal placental phenotypes in SCNT embryos are commonly observed in mammalian species, investigation of H3K27me3-dependent imprinting status in cloned embryos of other species may warrant future investigation.

The results described were obtained using the following methods and materials.

### Isolation of maternal and paternal pronuclei from PN5 stage zygotes

All animal studies were performed in accordance with guidelines of the Institutional Animal Care and Use Committee at Harvard Medical School. MII-stage oocytes were collected from 8 week-old B6D2F1/J (BDF1) females superovulated by injecting 7.5 I.U. of PMSG (Millipore) and hCG (Millipore). For *in vitro* fertilization (IVF), MII oocytes were inseminated with activated spermatozoa obtained from the caudal epididymis of adult BDF1 male mice in HTF medium supplemented with 10 mg/ml bovine serum albumin (BSA; Sigma-Aldrich). Spermatozoa capacitation was attained by 1 h incubation in the HTF medium. Zygotes were cultured in a humidified atmosphere with 5% CO₂/95% air at 37.8°C. At 10 hours post-fertilization (hpf), zygotes were transferred into M2 media containing 10 µg/ml cytochalasin B (Sigma-Aldrich). Zona pellucidae were cut by a Piezo impact-driven micromanipulator (Prime Tech Ltd., Ibaraki, Japan) and the pronuclei were isolated from the zygotes. At 12 hpf (PN5-stage), isolated pronuclei were washed with 0.2% BSA/PBS, transferred into Eppendorf LoBind 1.5 ml tubes, and placed on ice until DNase I treatment. For each experiment, 150-200 pronuclei were collected and prepared for liDNase-seq. The parental pronuclei were distinguished by (1) the distance from the second polar body and (2) the size of the pronucleus.

### Preparation of androgenetic (AG) and gynogenetic (GG) embryos

MII oocytes were collected from 8 week-old superovulated BDF1 females and inseminated with BDF1 sperm. At 7 hpf, zygotes were transferred into M2 media containing 5 µg/ml cytochalasin B, and parental pronuclei were exchanged by using a Piezo impact-driven micromanipulator. The sendai virus (HVJ, Cosmo-bio) was used for fusing karyoplasts with cytoplasms as previously described. After reconstruction, embryos were cultured in KSOM.
When collecting embryos for RNA-seq or/and liDNase-seq, zona pellucida (ZP) was removed by a brief exposure to Acid tyrode's solution (Sigma-Aldrich), then the embryos were washed with M2 media, and then 0.2% BSA/PBS. For liDNase-seq, 10 morula embryos were transferred into an Eppendorf LoBind 1.5 ml tube, and placed on ice until DNase I treatment. For RNA-seq, seven to ten embryos were transferred into a thin-walled RNase-free PCR tubes (Ambion). The 2-cell and morula embryos were collected at 30 and 78 hpf, respectively. When preparing α-amanitin treated 2-cell embryos, 5 hpf zygotes were transferred into KSOM containing 25 µg/ml α-amanitin (Sigma-Aldrich) and cultured in the presence of α-amanitin until collection (30 hpf). ICM and TE were isolated. Briefly, AG and GG embryos at 120 hpi were treated with Acid tyrode's solution to remove ZP. After being washed in M2 media, the embryos were incubated in KSOM containing rabbit anti-mouse lymphocyte serum (Cedarlane, 1:8 dilution) for 45 min at 37°C. After being washed in M2 media, they were transferred into KSOM containing guinea pig complement (MP Biomedicals, 1:3.3 dilution). After incubation for 30 min at 37°C, lysed TE cells were removed by pipetting with a glass capillary. The remaining ICM clumps were incubated in 0.25% Trypsin/EDTA (Thermo Fisher, 25200) for 10 min at 37°C, and then dissociated into single cells to avoid contamination of lysed TE cells. 100-200 cells were collected for RNA-seq.

### Isolation of GV nuclei from fully-grown oocytes

Fully-grown GV-stage oocytes were obtained from 3-week-old BDF1 mice 44-48 h after injection with 5 I.U. PMSG. The ovaries were transferred to M2 media. The ovarian follicles were punctured with a 30-gauge needle, and the cumulus cells were gently removed from the cumulus-oocyte complexes using a narrow-bore glass pipette. The oocytes were then transferred into α-MEM (Life technologies, 12571-063) supplemented with 5% Fetal Bovine Serum (FBS) (Sigma-Aldrich, F0926), 10 ng/ml Epidermal Growth Factor (Sigma-Aldrich, E4127), and 0.2 mM 3-isobutyl-1-methylxanthine (IBMX; Sigma-Aldrich). One hour after collection, GV oocytes exhibiting visible perivitelline spaces, which have the surrounding-nucleolus (SN)-type chromatin, were culled. They were then incubated in M2 media containing 10 µg/ml cytochalasin B, 0.1 µg/ml colcemid (Sigma-Aldrich), and 0.2 mM IBMX for 15 min. Then, GV nuclei were isolated by using a Piezo-driven micromanipulator. After washing with 0.2% BSA/PBS, the GV nuclei were transferred into an Eppendorf LoBind 1.5 ml tube. For each experiment, 115-150 GV nuclei were collected for liDNase-seq.

### Dissection of E6.5 embryos and FACS sorting of GFP-positive E9.5 placental cells

To obtain C57BL6(B6)/PWK hybrid embryos, a natural mating scheme was used. To obtain PWK/B6 hybrid embryos, *in vitro* fertilization of PWK oocytes with B6 sperm was used, and the 2-cell embryos were transferred into surrogate ICR strain mothers. Dissection of E6.5 embryos into EPI, EXE, and VE was performed. To collect E9.5 placental cells, the B6^{GFP} mice from Jackson laboratory were purchased [C57BL/6-Tg(CAG-EGFP)131Osb/LeySopJ, Stock number 006567]. MII oocytes and sperms were collected from superovulated 8-week old B6^{GFP} or PWK mice. After *in vitro* fertilization, the 2-cell embryos were transferred into surrogate ICR strain mothers. At E9.5, placentae were harvested, cut into ~0.5 mm pieces, transferred into 50 ml tubes, and treated with 2 ml of 0.25% Trypsin-EDTA (Thermo Fisher Scientific, 25200) at 30°C for 15 min in a shaker at 200 rpm to dissociate placental cells. Trypsin treatment was stopped by the addition of 2 ml DMEM containing 10% FBS. After pipetting, the tubes were centrifuged and the pelleted cells were washed with 0.2%BSA/PBS three times. DAPI was added at the final concentration of 1 µM in the final cell suspension. The GFP-positive cells were sorted using a BD FACSaria machine (BD Biosciences) with DAPI positive cells excluded as dead cells. Approximately 10,000-20,000 GFP-positive cells were collected from each placenta, which corresponded to 40-60% of total placental cells.

### Plasmid construction and mRNA preparation

To generate the *Kdm6b^{WT}* construct, the cDNA encoding the carboxyl-terminal part containing the catalytic domain (amino acid 1025-End) was amplified. The PCR amplicon was cloned between a Flag tag and poly(A) of the pcDNA3.1-Flag-poly(A)83 plasmid. The H1390A *Kdm6b^{MUT}* construct were generated by using PrimeSTAR mutagenesis (TAKARA). Primers used for the mutagenesis are 5'-CCAGGCgctCAAGAGAATAACAATTTCTGCTCAGTCAACATCAAC-3' and 5'-CTCTTGagcGCCTGGCGTTCGGCTGCCAGGGACCTTCATG-3'. All constructs were verified by DNA sequencing. The plasmids for wild-type and H189A mutant *Kdm4d* were previously described.

After linearization by a restriction enzyme, the construct was purified with phenol-chloroform extraction. mRNA was synthesized by *in vitro* transcription using a mMESSAGE mMACHINE T7 Ultra Kit (Life technologies) according to manufacturer's instructions. The synthesized mRNA was purified by lithium chloride precipitation and diluted with nuclease-free water. mRNA aliquots were stored in -80°C until use.

### mRNA injection

MII oocytes were collected from superovulated 8 week-old BDF1 females and inseminated with BDF1 sperm. At 2.5 hpf, fertilized oocytes were transferred into M2 media and mRNA was injected using a Piezo impact-driven micromanipulator. mRNA injection was completed by 4 hpf. The mRNA concentrations of *Kdm6b^{WT}* and *Kdm6b^{MUT}* were 1.8 µg/µl, and those of *Kdm4d^{WT}* and *Kdm4d^{MUT}* were 1.5 µg/µl. When preparing *Kdm6b-injected* PG embryos, MII oocytes were chemically activated by treating with 3 mM SrCl₂ in Ca²⁺-free KSOM containing 5 µg/ml cytochalasin B. At 4 hrs post-activation (hpa), the embryos were washed with KSOM. At 5 hpa, they were injected with mRNA.

### Whole mount immunostaining

Zygotes were fixed in 3.7% paraformaldehyde (PFA) in PBS containing 0.2% Triton for 20 min. After 4x washes with PBS containing 10 mg/ml BSA (PBS/BSA), zygotes were treated with primary antibodies at 4°C overnight. The primary antibodies used in this study were mouse-anti-H3K27me3 (1/500, Active Motif, 61017), rabbit anti-H3K9me3 (1/500, Millipore, 07-442), and rabbit anti-FLAG (1/2000, Sigma-Aldrich, F7524). After 3x washes with PBS/BSA, samples were incubated with a 1:250 dilution of fluorescein isothiocyanate-conjugated anti-mouse IgG (Jackson Immuno-Research) or Alexa Flour 568 donkey anti-rabbit IgG (Life technologies) for 1 h. The zygotes were then mounted on a glass slide in Vectashield anti-bleaching solution with 4',6-diamidino-2-phenylindole (DAPI) (Vector Laboratories, Burlingame, CA). Fluorescence was detected under a laser-scanning confocal microscope with a spinning disk (CSU-10, Yokogawa) and an EM-CCD camera (ImagEM, Hamamatsu) or Zeiss LSM800.

All images were acquired and analyzed using the Axiovision software (Carl Zeiss). The fluorescent signal intensity was quantified with the Axiovision software. Briefly, the signal intensity within the maternal pronuclei was determined, and the cytoplasmic signal was subtracted as background. Then, the averaged signal intensity of the no-injection control zygotes was set as 1.0.

### Low-input DNase-seq

Low-input DNase-seq libraries were prepared as previously described with minor modifications. Embryos or nuclei collected in 1.5 ml tubes were resuspended in 36 µl lysis buffer (10 mM Tris-HCl, pH 7.5, 10 mM NaCl, 3 mM MgCl2, 0.1% Triton X-100) and incubated on ice for 5 min. DNase I (10 U/µl, Roche) was added to the final concentration of 80 U/ml (for the GV nucleus sample) or 40 U/ml (for all the other samples) and incubated at 37 °C for exactly 5 min. The reaction was stopped by adding 80 µl Stop Buffer (10 mM Tris-HCl, pH 7.5, 10 mM NaCl, 0.15% SDS, 10 mM EDTA) containing 2 µl Proteinase K (20 mg/ml, Life technologies). Then 20 ng of a circular carrier DNA [a pure plasmid DNA without any mammalian genes purified with 0.5x Beckman SPRIselect beads (Beckman Coulter) to remove small DNA fragments] was added. The mixture was incubated at 50 °C for 1 hr, then DNA was purified by extraction with phenol-chloroform and precipitated by ethanol in the presence of linear acrylamide (Life technologies) overnight at -20 °C. Precipitated DNA was resuspended in 50 µl TE (2.5 mM Tris, pH 7.6, 0.05 mM EDTA), and the entire volume was used for sequencing library construction.

Sequencing library was prepared using NEBNext Ultra II DNA Library Prep Kit for Illumina (New England Biolabs) according to the manufactures' instruction with the exception that the adaptor ligation was performed with 0.03 µM adaptor in the ligation reaction for 30 minutes at 20 °C and that PCR amplification was performed using Kapa Hifi hotstart readymix (Kapa Biosystems) for 8-cycles. The PCR products were purified with x1.3 volume of SPRIselect beads (Beckman Coulter) and then size selected with x0.65 volume followed by x0.7 volume of SPRIselect beads. The sample was eluted in 24 µl TE. The number of cycles needed for the second PCR amplification was determined by qPCR using 1 µl of the 1:1,000 diluted samples. The remaining 23 µl of the samples was then amplified with Kapa Hifi hotstart readymix (we used 7 cycles for all samples in this study). The PCR product was purified with x1.3 volume of SPRIselect beads and then size selected with x0.65 volume followed by x0.7 volume of SPRIselect beads. The DNA was eluted in 30 µl of TE and quantified by Qubit dsDNA HS assay kit (Thermo Fisher Scientific, Q32854) and Agilent high sensitivity assay kit (Agilent Technologies). The libraries were sequenced on a Hiseq2500 with single-end 100 bp reads (Illumina).

### RNA-sequencing

RNA-seq libraries were prepared as previously described. Briefly, reverse transcription and cDNA amplification were performed using whole embryo lysates with SMARTer Ultra Low Input RNA cDNA preparation kit (Clontech, 634890). When processing 2-cell AG, GG and α-amanitin-treated IVF embryo samples, 1 µl of 1:40,000 diluted ERCC (External RNA Controls Consortium) standard RNA (Life technologies) was added to each of the tubes at the step of cell lysis. cDNAs were then fragmented using the Covaris M220 sonicator (Covaris) with microTUBE-50 (Covaris) into average 150-160 bp fragments. The fragmented cDNAs were end-repaired, adaptor ligated and amplified using NEBNext Ultra DNA Library Prep Kit for Illumina according to the manufacturer's instruction (New England Biolabs). Single end 100 bp sequencing was performed on a HiSeq2500 sequencer (Illumina).

### liDNase-seq data analysis

Reads of liDNase-seq data were firstly trimmed of low quality and adapter with trim_galore, and then mapped to the mouse genome (mm9) using Bowtie v0.12.9. '-m 1' parameter to keep unique mapping hits. The reads with mapping quality (MAPQ) ≤ 10 or redundant reads that mapped to the same location with the same orientation were removed with SAMtools. The DHS peaks in liDNase-seq data were identified by Hotspot program with FDR <= 0.01. The DHS peaks from all 33 libraries were merged using 'bedtools merge' from bedtools. The number of reads in each DHS for each library was calculated using 'multiBamSummary' from deepTools and normalized to the total number of mapped reads and to the length of DHS (possibility of a tag located on a position per 1 kb per million mapped reads). Reads of sex chromosomes were removed because the number of sex chromosomes is different between the parental pronuclei and between androgenetic and gynogenetic embryos. The Pearson correlation coefficient (r) of tag densities at genome-wide DHSs was calculated to measure the correlation between replicates. For identification of parental allele-specific DHSs in zygotes and morula embryos, a stringent cutoff was used (RPKM mean>2, RPKM>1 in all replicates in a biased allele, and mean value fold change larger than 4 between the two alleles). The 431 most reliable Ps-DHSs were identified by applying an additional criterion 'RPKM>1 in all replicates of paternal PNs of microinjected zygotes' to Ps-DHSs. The RefSeq gene assembly (mm9) from the UCSC Genome Browser database and CGIs previously defined were used as genomic feature distribution analysis in FIGs. 2D and 2E.

### RNA-seq data analysis

A custom reference sequence combining mouse genome (mm9) with the ERCC control was constructed. Reads of RNA-seq were mapped to the reference genome with TopHat v2.0.6 or STAR (github.com/alexdobin/STAR). All programs were run with default parameters unless otherwise specified. Uniquely mapped reads were subsequently assembled into transcripts guided by the reference annotation (UCSC gene models) with featureCounts from subread-v1.5.1. For all 2-cell RNA-seq libraries, library size factors were estimated with 'estimateSizeFactors' function form R package DESeq only using ERCC read counts. After the library size was normalized, the expression level of each gene was quantified with normalized FPKM (fragments per kilobase of exon per million mapped fragments). The Pearson correlation coefficient (r) of gene expression level was calculated to indicate the correlation between duplicates. For identification of newly synthesized transcripts at the 2-cell stage, statistically non-significant genes were filtered out between AG or GG and α-amanitin treated 2-cell embryo. To this end, adjusted P value was calculated with 'nbinomTest' function form R pakage DESeq using a negative binomial model, and only genes with FDR<0.05 were selected. Additional cutoffs [Mean FPKM (AG or GG)>2 and fold-change (FC) (AG/Ama or GG/Ama)>2] were then applied. As a result, 4,381 and 3,916 genes were identified as newly synthesized genes in AG and GG 2-cell embryos, respectively. For identifying AG- and GG-specific DEGs in 2-cell embryos, the gene expression level (FPKM) of each gene in α-amanitin 2-cell embryos was subtracted from that of AG and GG embryos. Genes showing FC (AG/GG or GG/AG)>10 were identified as DEGs.

### WGBS and H3K27me3 ChIP-seq data analyses

The DNA methylation level at DHSs was calculated using methpipe v3.4.2. When calculating the DNA methylation level at each DHS, to get enough coverage of WGBS reads, each DHS was extended to both up and downstream 2 kb to include more nearby CpG sites. The oocyte-methylated gDMR was defined by >80% methylation in oocytes and <20% in sperm. For FIG. 5A, "bedtools makewindows" were used to generate a set of non-overlapped 1 kb bins for the ±100 kb flanking region of Ps-DHSs. For H3K27me3 ChIP-seq analysis, Bed files were downloaded from Zheng et al., 2016 and converted to the bigWig format using 'bedClip' and 'bedGraphToBigWig' from UCSC Genome Browser database. 'multiBigwigSummary' from deepTools was used to compute H3K27me3 signal over the DHS and surrounding region.

### Statistical analyses and data visualization

Statistical analyses were implemented with R (www.r-project.org/). Pearson's r coefficient was calculated using the 'cor' function with default parameters. FIGs. 6B and 10D were generated with R function 'heatmap.2'. FIGs. 7D, 10C, and 12A-12D were generated with R function 'pheatmap'. FIGs. 1B and 7B were generated using 'computeMatrix' and 'plotHeatmap' function in deepTools. Position-wise coverage of the genome by sequencing reads was determined by normalizing to the total unique mapped reads in the library using macs2 v2.1.0 and visualized as custom tracks in the IGV genome browser.

### Known imprinting gene information

Known imprinting information was downloaded from www.geneimprint.com/site/genes-by-species.Mus+musculus.

### Code availability

A customized pipeline was used to split the hybrid RNA-seq data to their parental origin based on SNP information. The code can be found at github.com/lanjiangboston/UniversalSNPsplit.

### Data availability statement

All the liDNase-seq and RNA-seq datasets generated in this study were deposited at GEO database under accession number GSE92605. Sperm liDNase-seq datasets were from a previously publication (GSE76642). WGBS datasets for sperm and GV oocytes were downloaded from www.nodai-genome.org/mouse.html?lang=en. H3K27me3 ChIP-seq datasets of sperm, MII oocytes, and SNP-tracked maternal and paternal alleles of 1-cell embryos were downloaded from a previous publication (GSE76687).

### Collection of mouse preimplantation embryos

All animal studies were performed in accordance with guidelines of the Institutional Animal Care and Use Committee at Harvard Medical School. MII-stage oocytes were collected from 8 week-old B6D2F1/J (BDF1) females superovulated by injecting 7.5 I.U. of PMSG (Millipore) and hCG (Millipore). For *in vitro* fertilization (IVF), MII oocytes were inseminated with activated spermatozoa obtained from the caudal epididymis of adult BDF1 or PWK (Jackson Laboratory, 003715) males in HTF medium supplemented with 10 mg/ml bovine serum albumin (BSA; Sigma-Aldrich). Spermatozoa capacitation was attained by 1 h incubation in the HTF medium. Zygotes were transferred to KSOM and cultured in a humidified atmosphere with 5% CO₂/95% air at 37.8°C.

### mRNA injection

At 4 hrs post-fertilization (hpf), zygotes were transferred into M2 media and mRNA was injected using a Piezo impact-driven micromanipulator (Prime Tech Ltd., Ibaraki, Japan). The construction and preparation of mRNA were described above. The concentrations of injected mRNA of *Kdm6b^{WT}* and *Kdm6b^{MUT}* were 1.8 µg/µl, and those of *Kdm4d^{WT}* and *Kdm4d^{MUT}* were 1.5 µg/µl.

### Probe for fluorescent in situ hybridization

A probe for *Xist* RNA was prepared by using Nick translation reagent kit (Abbott Molecular, 07J00-001) with Cy3-dCTP (GE healthcare, PA53021), according to the manufacturer's instruction. The template DNA used for the probe preparation was a plasmid coding the full-length mouse *Xist* gene, a gift from Rudolf Jaenisch (pCMV-Xist-PA, 26760) (Wutz and Jaenisch, 2000). A probe for DNA FISH was prepared using the same kit with Green-dUTP (Abbott Molecular, 02N32-050). The template DNA was a BAC clone containing the *Rnf12* locus (RP23-36C20). The fluorescent probes were ethanol precipitated with 5 µg Cot-1 DNA (Life technologies), 5 µg herring sperm DNA (Thermo Fisher Scientific), and 2.5 ng yeast tRNA (Thermo Fisher Scientific, AM7119), and then dissolved with 20 µl formamide (Thermo Fisher Scientific, 17899). The probes were stored at 4°C. Before being used, the probes (0.75 µl each) were mixed with 0.75 µl Cot-1 DNA, which had been ethanol precipitated and dissolved in formamide, and 2.25 µl of 4xSSC/20% Dextran (Millipore S4030). The probe mixtures were heated at 80°C for 30 min and then transferred to a 37°C incubator ('pre-annealed probes').

### Whole mount RNA/DNA fluorescent in situ hybridization

Morula embryos were fixed at 78 hpf in 2% paraformaldehyde (PFA) in PBS containing 0.5% Triton X-100 for 20 min at room temperature. After 3x washes with PBS containing 1 mg/ml BSA (PBS/BSA), embryos were treated with 0.1 N HCl containing 0.02% Triton X-100 for 15 min at 4°C. After 3x washes with 2xSSC containing 0.1% BSA, embryos were incubated in 15 µl of 10% formamide/2xSSC in a glass dish (Electron Microscopy Science, 705430-30). All embryos were sunk and attached to the bottom of the glass dish by gentle pipetting. After 5 min, 15 µl of 30% formamide/2xSSC was added. After 5 min, 90 µl of 60% formamide/2xSSC was added to make the final formamide concentration 50%, and embryos were incubated for additional 30 min at room temperature. The formamide solution containing embryos were covered with mineral oil. The samples were heated at 80°C for 30 min, and then transferred to a 37°C incubator for at least 30 min. The embryos were picked in a glass pipette, transferred into 4.5 µl of 'pre-annealed probes' covered with mineral oil on another glass dish, and incubated in 37°C for at least 24 hrs. Embryos were washed with pre-warmed (42°C) 2xSSC containing 0.1%BSA and left in the last drop for 30 min. After 3x wash with 1%BSA/PBS, they were mounted on a glass slide in Vectashield anti-bleaching solution with 4',6-diamidino-2-phenylindole (DAPI) (Vector Laboratories, Burlingame, CA). Fluorescence was detected under a laser-scanning confocal microscope Zeiss LSM800.

### Whole mount immunostaining

The procedure of immunostanining and quantification was described above.

### Computational identification of maternal allele-biased H3K27me3

The bed files including RPKM values in 100 bp bins for H3K27me3 ChIP-seq in inner cell mass (ICM) were downloaded from GEO under the number GSE76687. Bed files labeled maternal or paternal containing RPKM values for two parental alleles and allelic reads were normalized to total reads number. 'bedtools makewindows' was used to generate 1000 bp bins for mm9 genome, then RPKM value for each bin was calculated by 'bedtools map'. All the bins are classified to three categories of no signal, biallelic, maternal bias using a signal cutoff of 1 and a fold change cutoff of 4. A sliding window approach was used to identify windows containing maternal biased H3K27me3 bins with criteria of the window size of 20 kb, the minimum bin number of 3 and the percentage of maternal biased H3K27me3 bins larger than 50%. Overlapped windows were merged with "bedtools merge". A total of 5986 windows were identified in the genome.

### RNA-sequencing

RNA-seq libraries were prepared as described above with minor modifications. Briefly, reverse transcription and cDNA amplification were performed using whole embryo lysates with SMARTer Ultra Low Input RNA cDNA preparation kit (Clontech, 634890). cDNAs were then fragmented using Tagmentation with Nextera XT DNA library prep kit (Illumina). The fragmented cDNAs were amplified using Nextera PCR master mix according to the manufacturer's instruction. Single end 100 bp sequencing was performed on a HiSeq2500 sequencer (Illumina).

### RNA-seq data analysis

Reads of RNA-seq were mapped to the reference genome with STAR (github.com/alexdobin/STAR). All programs were run with default parameters unless otherwise specified. Uniquely mapped reads were subsequently assembled into transcripts guided by the reference annotation (UCSC gene models) with featureCounts from subread-v1.5.1. After the library size was normalized, the expression level of each gene was quantified with normalized FPKM (fragments per kilobase of exon per million mapped fragments). The Pearson correlation coefficient (r) of gene expression level was calculated to indicate the correlation between duplicates.

Statistical analyses were implemented with R (www.r-project.org/). Pearson's r coefficient was calculated using the 'cor' function with default parameters

### Code availability

A customized pipeline was used to split the hybrid RNA-seq data to their parental origin based on SNP information. The code can be found at github.com/lanjiangboston/UniversalSNPsplit.

### Data availability

RNA-seq datasets generated in this study were deposited at GEO database under accession number GSEXXXXX. The WGBS dataset for GV oocytes was downloaded from www.nodai-genome.org/mouse.html?lang=en. WGBS reads from same 100 bp bins were pooled together to calculate the average methylation level and minimal coverage of 10 reads was required. H3K27me3 ChIP-seq datasets of sperm, MII oocytes, and SNP-tracked maternal and paternal alleles of 1-cell, 2-cell, and inner cell mass of blastocyst embryos were downloaded from a previous study (GSE76687). The oocyte DNaseI-seq datasets were from above (GSE92605).

### Mice

B6D2F1/J (BDF1) mice were used for the collection of recipient oocytes for SCNT. For mouse embryonic fibroblast (MEF) cell preparation, *Xist* KO female mice maintained in 129S1/SvImj background (Marahrens et al., 1997) were mated with CAST/EiJ males to generate *Xist* heterozygous KO embryos in 129/CAST F1 background. For cumulus and Sertoli cell preparation, *Xist* KO female mice in C57BL/6N background (Sado et al., 2005) were mated with DBA/2N males to generate *Xist* heterozygous KO embryos in BDF1 background. All animal experiments were approved by the Institutional Animal Care and Use Committees of Harvard Medical School and RIKEN Tsukuba Institute.

### Donor cell preparation

Primary MEF cells were derived from Xist KO male mouse embryos at 13.5 dpc. After removal of head and all organs, minced tissue from remaining corpus was dissociated in 500 µl of 0.25% Trypsin with 1 mM EDTA (Thermo Fisher Scientific # 25200056) for 10 min at 37°C. Cell suspension was diluted with equal amount of DMEM (Thermo Fisher Scientific # 11995-073) containing 10% FBS and Penicillin/Streptomycin (Thermo Fisher Scientific # 15140-022) and pipetted up and down 20 times. The cell suspension was diluted with fresh medium and plated onto 100 mm dishes and cultured at 37°C. Two days later, MEF cells were harvested and frozen. Frozen stocks of MEF cells were thawed and used for experiments after one passage.

Cumulus cells were collected from wildtype (WT) and *Xist* heterozygous KO adult females (RIKEN BioResource Center, RBRC01260) through superovulation by injecting 7.5 IU of pregnant mare serum gonadotropin (PMSG; Millipore # 367222) and 7.5 IU of human chorionic gonadotropin (hCG; Millipore # 230734). Fifteen to seventeen hours after the hCG injection, cumulus-oocyte complexes (COCs) were collected from the oviducts and treated briefly with Hepes-buffered potassium simplex-optimized medium (KSOM) containing 300 U/ml bovine testicular hyaluronidase (Calbiochem # 385931) to obtain dissociated cumulus cells.

Sertoli cells were collected from testes of 3-5 day-old WT or *Xist* KO male mice as described (Matoba et al., 2011). Testicular masses were incubated in PBS containing 0.1 mg/ml collagenase (Thermo Fisher Scientific # 17104-019) for 30 min at 37°C followed by 5 min treatment with 0.25% Trypsin with 1 mM EDTA at room temperature. After washing for four times with PBS containing 3 mg/ml bovine serum albumin, the dissociated cells were suspended in Hepes-KSOM medium.

### Kdm4d mRNA synthesis

*Kdm4d* mRNA was synthesized by *in vitro* transcription (IVT) as described previously (Matoba et al., 2014). Briefly, a pcDNA3.1 plasmid containing full length mouse *Kdm4d* followed by poly(A)83 (Addgene # 61553) was linearized by *Xba*I. After purification, the linearized plasmid DNA was used as a template for IVT using mMESSAGE mMACHINE T7 Ultra Kit (Thermo Fisher Scientific # AM1345). The synthesized mRNA was dissolved in nuclease-free water and quantified by NanoDrop ND-1000 spectrophotometer (NanoDrop Technologies). After the mRNA is diluted to 1500 ng/µl, aliquots were stored at -80°C.

### SCNT

Mouse somatic cell nuclear transfer was carried out as described previously (Matoba et al., 2014; Ogura et al., 2000). Briefly, recipient MII oocytes were collected from adult BDF1 female mice through superovulation by injecting 7.5 IU of PMSG and 7.5 IU of hCG. Fifteen to seventeen hours after the hCG injection, cumulus-oocyte complexes (COCs) were collected from the oviducts and treated briefly with Hepes- KSOM containing 300 U/ml bovine testicular hyaluronidase to obtain MII oocytes. Isolated MII oocytes were enucleated in Hepes-buffered KSOM medium containing 7.5 µg/ml of cytochalasin B (Calbiochem # 250233) by using Piezo-driven micromanipulator (Primetech # PMM-150FU). The nuclei of cumulus or Sertoli cells were injected into the enucleated oocytes. MEF cells were fused with enucleated oocytes by inactivated Sendai virus envelope (GenomOne CF; Ishihara Sangyo #CF001). After 1h incubation in KSOM, reconstructed SCNT oocytes were activated by incubating in Ca-free KSOM containing 3 mM strontium chloride (SrCl2) and 5 µg/ml cytochalasin B for 1 h, and further cultured in KSOM with 5 µg/ml cytochalasin B for 4 h. Activated SCNT embryos were washed 5 h after the onset of SrCl₂ treatment (hours post activation, hpa) and cultured in KSOM in a humidified atmosphere with 5% CO₂ at 37.8°C. Some SCNT embryos were injected with ~10 pl of 1500 ng/µl mouse Kdm4d mRNA at 5-6 hpa by using a Piezo-driven micromanipulator.

### Embryo transfer

Two-cell stage SCNT embryos were transferred to the oviducts of pseudopregnant (E0.5) ICR females. The pups were recovered by caesarian section on the day of delivery (E19.5) and nursed by lactating ICR females. Some females were sacrificed at E4.5 and E10.5 for examining embryonic development.

### Histological analysis of placenta

Placentae at E19.5 were fixed in 4% paraformaldehyde (PFA) 4°C overnight and routinely embedded in paraffin. Serial sections (4 µm in thickness) were subjected to periodic acid Schiff (PAS) staining.

### Immunostaining for H3K27me3 in blastocysts

Blastocysts were fixed with 4% paraformaldehyde (PFA) for 20 min at room temperature. After washing with PBS containing 10 mg/ml BSA (PBS/BSA), the fixed embryos were permeabilized by 15 min incubation with 0.5% Triton-X 100. After blocking in PBS/BSA for 1 h at room temperature, they were incubated in a mixture of primary antibodies including rabbit anti-H3K27me3 antibody (1/500, Millipore, 07-449), goat anti-Oct4 antibody (1/500, SantaCruz, sc-8628) and mouse anti-Cdx2 antibody (1/100, BioGenex, AM392-5M) at 4°C overnight. Following three washes with PBS/BSA, the embryos were incubated with a mixture of secondary antibodies including fluorescein isothiocyanate-conjugated anti-mouse IgG (1/400, Jackson Immuno-Research), Alexa Flour 546 donkey anti-rabbit IgG (1/400, Thermo Fisher Scientific) and Alexa Flour 647 donkey anti-goat IgG (1/400, Thermo Fisher Scientific) for 1 h at room temperature. Finally, they were mounted with Vectashield with 4',6-diamidino-2-phenylindole (DAPI) (Vector Laboratories # H-1200). The fluorescent signals were observed using a laser-scanning confocal microscope (Zeiss LSM510) and an EM-CCD camera (Hamamatsu ImagEM).

### WGBS

IVF and SCNT embryos of the early blastocyst stage (96 hours after fertilization or activation) were directly subjected to bisulfite conversion using the EZ DNA Methylation-Direct Kit (Zymo Research, D5020). Thirty-nine and 36 embryos were used for preparing the IVF and SCNT samples, respectively. A small amount (0.01 ng) of unmethylated Lambda DNA (Promega, D152A) was added to each sample before bisulfite conversion to serve as spike-in controls for evaluating bisulfite conversion efficiency. Sequencing libraries were prepared using the EpiGnome Methyl-Seq kit (Epicenter, EGMK81312) following the manufacturer's instructions. Libraries were only amplified for 12 cycles, and were then purified using Agencourt AMPure XP beads (Beckman Coulter, A63880). Final libraries were subjected to single-read (100 bp) sequencing on a HiSeq 2500 sequencer (Illumina) with PhiX spike-in control.

### RNA-seq

Six IVF or SCNT embryos of the early blastocyst stage (96 hours after fertilization or activation) were directly lysed and used for cDNA synthesis using the SMARTer Ultra Low Input RNA cDNA preparation kit (Clontech, 634936). After amplification, the cDNA samples were fragmented using a Covaris M220 sonicator (Covaris). Sequencing libraries were made with the fragmented cDNA using NEBNext Ultra DNA Library Prep Kit for Illumina according to manufacturer's instructions (New England Biolabs, E7370). Single-read 50 bp sequencing was performed on a HiSeq 2500 sequencer (Illumina).

### QUANTIFICATION AND STATISTICAL ANALYSIS

### WGBS and RRBS data analysis

WGBS and reduced representation bisulfite sequencing (RRBS) reads were first trimmed using trim_galore to remove low-quality sequences and adapter sequences. Bismark (version 0.15.0) was used to align reads to a bisulfite converted reference genome (mm9). The coverage depth and methylation level of each cytosine were extracted from the aligned reads with bismark_methylation_extractor. When calculating methylation level for CpG sites, information from both strands was combined, and a coverage of at least five reads was required. DMRs were identified using methpipe (version 3.4.3) and were further filtered requiring at least 10 CpG sites and at least 10% methylation difference. Functional annotation of DMR associated genes (i.e., genes with a DMR located in the TSS±3kb region) was performed with clusterProfiler (version 2.4.3) in R. For allele specific methylation analysis of known ICRs, all detected CpGs within one ICR were pooled together, and a coverage of at least 5 detected CpGs in both alleles was required for further methylation comparison.

### RNA-seq data analysis

RNA-seq data were mapped to the mouse genome (mm9) with TopHat (version 2.0.14) with parameters "--no-coverage-search --no-novel-juncs --library-type=fr-unstranded". Uniquely mapped reads were subsequently assembled into transcripts guided by the reference annotation (UCSC gene models) with Cufflinks (version 2.2.1). Expression levels of each gene was quantified with normalized FPKM (fragments per kilobase of exon per million mapped fragments). The Pearson correlation coefficient of gene expression level was calculated to indicate the correlation between duplicates.

### Allele specific analysis

After mapping the hybrid WGBS and RNA-seq data (129S1/Svj x CAST/EiJ) to the mouse genome (mm9), custom Perl scripts were used to split mapped reads to their parental origin on the basis of SNP information downloaded from the Mouse Genomes Project (ftp://ftp-mouse.sanger.ac.uk/REL-1211-SNPs_Indels/). The allelic reads were then processed individually.

### ChIP-seq and DIP-seq data analysis

Downloaded ChIP-seq and DIP-seq reads were mapped to the mouse genome (mm9) using Bowtie (version 2.1.0) with parameters "-D 20 -R 3 -N 1 -L 20 -i S,1,0.50" to obtain only those reads that are mapped uniquely with at most 3 mismatches. To visualize the signals in the genome browser, we generated wig track files for each data set with MACS2 (version 2.1.1) by extending the uniquely mapped reads (keeping at most two read at the same genomic position) to 200 bp toward the 3' end and binning the read count to 50 bp intervals. Tag counts were further normalized in each bin to the total number of uniquely mapped reads (reads per million reads, RPM). The 'computeMatrix' program from deepTools was used to compute the ChIP-seq and DIP-seq signals over the DMRs or ICRs and their flanking regions.

### Statistical analysis and data visualization

Statistical analyses and plots were implemented with R (version 3.4.1, http://www.r-project.org). Pearson correlation coefficient was calculated using the 'cor' function with default parameters. Student's *t*-test (two-tailed, equal variance) was performed using the 't.test' function with default parameters. ChIP-seq signals and DNA methylation levels were visualized as custom tracks in the Integrative Genomics Viewer genome browser.

### DATA AND SOFTWARE AVAILABILITY

The WGBS and RNA-seq datasets generated in this study have been deposited in Gene Expression Omnibus (GEO) under the accession number GSE109214.

### Published datasets used in this study

Maternal and paternal DNA methylation of the preimplantation embryos (2-cell, 4-cell, and ICM) was obtained from GSE56697 (Wang et al., 2014). RRBS data of different cells and somatic tissues were obtained from GSE11034 and GSE43719 (Soumillon et al., 2013). H3K27me3 ChIP-seq data were obtained from GSE49847 (Yue et al., 2014) and GSE76687 (Zheng et al., 2016). DIP-seq data of 5mC and 5hmC during PGC development were downloaded from SRP016940 (Hackett et al., 2013).

## Claims

1. A method for obtaining a cloned non-human, mammalian blastocyst, the method comprising:
(a) transferring a donor nucleus obtained from an isolated or cultured non-human, mammalian somatic cell lacking Xist activity into a non-human, mammalian enucleated oocyte; and
(b) overexpressing Kdm4d in the oocyte, thereby obtaining a cloned non-human, mammalian blastocyst,
wherein the somatic cell lacking Xist activity is produced by the use of:
genome editing;
a CRISPR system which introduces a deletion or inactivity mutation in a genomic Xist polynucleotide;
siRNA;
shRNA; or
RNAi.

2. The method of claim 1, wherein the oocyte is injected with a *Kdm4d* mRNA.

3. The method of claim 1, wherein the donor cell nucleus is obtained from a non-human embryonic fibroblast comprising a deletion in Xist or comprising an inactive form of Xist.

4. The method of claim 1, wherein the donor nucleus is obtained from a cat, cow, dog, pig, or horse.

5. A method for obtaining a cell or tissue suitable for transplantation into a non-human, mammalian subject, the method comprising: (a) inactivating *Xist* or reducing Xist activity or expression in a cultured cell obtained from a non-human, mammalian subject; wherein reducing Xist activity or expression is achieved by the use of genome editing; a CRISPR system which introduces a deletion or inactivity mutation in a genomic Xist polynucleotide; siRNA; shRNA; or RNAi;
(b) transferring the nucleus from the cultured cell into non-human, mammalian enucleated oocyte, thereby activating the oocyte; and
(c) injecting the activated non-human, mammalian oocyte obtained in step (b) with a *Kdm4d* mRNA; overexpressing Kdm4d in the oocyte; and culturing the resulting oocyte, thereby obtaining a non-human, mammalian cell or tissue suitable for transplantation into the non-human, mammalian subject.

6. The method of claim 5, wherein *Xist* is inactivated by genome editing of the cultured cell of step (a).

7. The method of claim 5, wherein *Xist* is inactivated by using a CRISPR system to introduce a deletion or inactivating mutation in a genomic *Xist* polynucleotide in the cultured cell of step (a).

8. The method of claim 5, wherein Xist polynucleotide expression or activity is reduced by using siRNA or shRNA in the cultured cell of step (a).

9. A non-human cell comprising a deletion in Xist or having a level of Xist expression reduced by the use of genome editing; a CRISPR system which introduces a deletion or inactivity mutation in a genomic Xist polynucleotide; siRNA; shRNA; or RNAi; and comprising a heterologous polynucleotide encoding Kdm4d, which is expressed at an increased level in the non-human cell.

## Patentansprüche

1. Verfahren zur Gewinnung einer klonierten nicht-humanen Säugetier-Blastozyste, wobei das Verfahren umfasst:
(a) Übertragen eines Donornukleus, der aus einer isolierten oder kultivierten nicht-humanen, somatischen Säugetier-Zelle ohne Xist-Aktivität gewonnen wurde, in eine nicht-humane, enukleierte Säugetier-Oozyte; und
(b) Überexprimieren von Kdm4d in der Oozyte, wodurch eine klonierte, nicht-humane Säugetier-Blastozyste gewonnen wird,
wobei die somatische Zelle ohne Xist-Aktivität erzeugt wird unter Verwendung von:
Genom-Editierung;
einem CRISPR-System, das eine Deletion oder Inaktivitätsmutation in ein genomisches Xist-Polynukleotid einführt;
siRNA;
shRNA; oder
RNAi.

2. Verfahren nach Anspruch 1, wobei Kdm4d-mRNA in die Oozyte injiziert wird.

3. Verfahren nach Anspruch 1, wobei der Donorzellnukleus aus einem nicht-humanen embryonalen Fibroblasten gewonnen wird, der eine Deletion in Xist oder eine inaktive Form von Xist umfasst.

4. Verfahren nach Anspruch 1, wobei der Donornukleus aus einer Katze, einem Rind, einem Hund, einem Schwein oder einem Pferd gewonnen wird.

5. Verfahren zur Gewinnung einer Zelle oder eines Gewebes, das zur Transplantation in ein nicht-humanes Säugetier geeignet ist, wobei das Verfahren umfasst:
(a) Inaktivieren von *Xist* oder Verringern der Xist-Aktivität oder -Expression in einer kultivierten Zelle, die von einem nicht-humanen Säugetier gewonnen wurde; wobei die Verringerung der Xist-Aktivität oder -Expression erreicht wird durch die Verwendung von Genom-Editierung; einem CRISPR-System, das eine Deletion oder Inaktivitätsmutation in ein genomisches Xist-Polynukleotid einführt; siRNA, shRNA oder RNAi;
(b) Übertragen des Nukleus der kultivierten Zelle in eine nicht-humane, enukleierte Säugetierzelle, wodurch die Oozyte aktiviert wird; und
(c) Injizieren einer Kdm4d-mRNA in die aktivierte nicht-humane, Säugetier-Oozyte, die in Schritt (b) gewonnen wurde; Überexprimieren von Kdm4d in der Oozyte; und Kultivieren der resultierenden Oozyte, wodurch eine nicht-humane Säugetierzelle oder ein nicht-humanes Säugetiergewebe gewonnen wird, das zur Transplantation in das nicht-humane Säugetiersubjekt geeignet ist.

6. Verfahren nach Anspruch 5, wobei *Xist* durch Genom-Editierung der kultivierten Zelle aus Schritt (a) inaktiviert wird.

7. Verfahren nach Anspruch 5, wobei *Xist* durch die Verwendung eines CRISPR-Systems inaktiviert wird, um eine Deletion oder eine inaktivierende Mutation in ein genomisches Xist-Polynukleotid in die kultivierte Zelle aus Schritt (a) einzuführen.

8. Verfahren nach Anspruch 5, wobei die *Xist-*Polynukleotid-Expression oder -Aktivität durch die Verwendung von siRNA oder shRNA in der kultivierten Zelle aus Schritt (a) verringert wird.

9. Nicht-humane Zelle, die eine Deletion in Xist umfasst oder eine Menge an Xist-Expression aufweist, die verringert wird durch die Verwendung von Genom-Editierung; einem CRISPR-System, das eine Deletion oder Inaktivitätsmutation in ein genomisches Xist-Polynukleotid einführt; siRNA, shRNA oder RNAi; und umfassend ein heterologes Polynukleotid, das für Kdm4d kodiert, das in der nicht-humanen Zelle in einer höheren Menge exprimiert wird.

## Revendications

1. Procédé d'obtention d'un blastocyste de mammifère non humain cloné, le procédé comprenant :
(a) le transfert d'un noyau donneur obtenu à partir d'une cellule somatique de mammifère non humaine isolée ou de culture dépourvue d'activité de Xist dans un ovocyte énucléé de mammifère non humain ; et
(b) la surexpression de Kdm4d dans l'ovocyte, ce qui permet d'obtenir un blastocyste de mammifère non humain cloné,
dans lequel la cellule somatique dépourvue d'activité Xist est produite au moyen de :
une édition génomique ;
un système CRISPR qui introduit une mutation par délétion ou d'inactivité dans un polynucléotide génomique de Xist ;
un petit ARN interférent ;
un petit ARN en épingle à cheveux ; ou
une interférence ARN.

2. Procédé selon la revendication 1, dans lequel un ARNm *Kdm4d* est injecté dans l'ovocyte.

3. Procédé selon la revendication 1, dans lequel le noyau de cellule donneur est obtenu à partir d'un fibroblaste embryonnaire non humain comprenant une délétion de Xist ou comprenant une forme inactive de Xist.

4. Procédé selon la revendication 1, dans lequel le noyau donneur est obtenu à partir d'un chat, d'une vache, d'un chien, d'un cochon ou d'un cheval.

5. Procédé d'obtention d'une cellule ou d'un tissu adapté à la transplantation chez un sujet mammifère non humain, le procédé comprenant : (a) l'inactivation de *Xist* ou la réduction de l'activité ou de l'expression de Xist dans une cellule de culture obtenue à partir d'un sujet mammifère non humain ; dans lequel la réduction de l'activité ou de l'expression de Xist est obtenue au moyen d'une édition génomique ; d'un système CRISPR qui introduit une mutation par délétion ou d'inactivité dans un polynucléotide génomique de Xist ; d'un petit ARN interférent ; d'un petit ARN en épingle à cheveux ; ou d'une interférence ARN ;
(b) le transfert du noyau de la cellule de culture dans un ovocyte énucléé de mammifère non humain, ce qui permet d'activer l'ovocyte ; et
(c) l'injection d'un ARNm *Kdm4d* dans l'ovocyte de mammifère non humain activé obtenu à l'étape (b) ; la surexpression de Kdm4d dans l'ovocyte ; et la culture de l'ovocyte résultant, ce qui permet d'obtenir une cellule ou un tissu de mammifère non humain adapté à la transplantation chez le sujet mammifère non humain.

6. Procédé selon la revendication 5, dans lequel *Xist* est inactivé par édition génomique de la cellule de culture de l'étape (a).

7. Procédé selon la revendication 5, dans lequel *Xist* est inactivé en utilisant un système CRISPR pour introduire une mutation par délétion ou inactivante dans un polynucléotide génomique de *Xist* dans la cellule de culture de l'étape (a).

8. Procédé selon la revendication 5, dans lequel l'expression ou l'activité du polynucléotide de Xist est réduite en utilisant un petit ARN interférent ou un petit ARN en épingle à cheveux dans la cellule de culture de l'étape (a).

9. Cellule non humaine comprenant une délétion dans Xist ou présentant un niveau d'expression de Xist réduit au moyen d'une édition génomique ; d'un système CRISPR qui introduit une mutation par délétion ou d'inactivité dans un polynucléotide génomique de Xist ; d'un petit ARN interférent ; d'un petit ARN en épingle à cheveux ; ou d'une interférence ARN ; et comprenant un polynucléotide hétérologue codant pour Kdm4d, qui est exprimé à un niveau accru dans la cellule non humaine.
